# EUROPÄISCHE PATENTSCHRIFT

(11) **EP 1 432 415 B1**
(45) Veröffentlichungstag und Bekanntmachung des Hinweises auf die Patenterteilung: **28.12.2005**
(21) Anmeldenummer: 02767488.6
(22) Anmeldetag: 16.09.2002
(51) Int. Cl.: A61K 31/404, A61K 31/4439, A61K 31/4245, A61K 31/506, A61K 31/4178, C07D 209/42, C07D 401/12, C07D 405/12, C07D 417/12, C07D 403/12, C07D 417/06

(54) **SUBSTITUIERTE 2,5-DIAMIDOINDOLE ALS ECE-INHIBITOREN ZUR BEHANDLUNG VON KARDIOVASKULÄREN ERKRANKUNGEN**
SUBSTITUTED 2,5-DIAMIDOINDOLES AS ECE INHIBITORS FOR THE TREATMENT OF CARDIOVASCULAR DISEASES
2,5-DIAMIDOINDOLS SUBSTITUES EN TANT QU'INHIBITEURS D'ECE POUR LE TRAITEMENT DE MALADIES CARDIO-VASCULAIRES

(30) Priorität: 27.09.2001 DE 10147672
(43) Veröffentlichungstag der Anmeldung: 30.06.2004
(73) Patentinhaber: Bayer HealthCare AG, 51368 Leverkusen (DE)
(72) Erfinder: ERGÜDEN, Jens-Kerim, 42489 Wülfrath (DE); KRAHN, Thomas, 58135 Hagen (DE); SCHRÖDER, Christian, 50129 Bergheim (DE); STASCH, Johannes-Peter, 42651 Solingen (DE); WEIGAND, Stefan, 42115 Wuppertal (DE); WILD, Hanno, 42113 Wuppertal (DE); BRANDS, Michael, CT 06516 West Haven (US); SIEGEL, Stephan, 42105 Wuppertal (DE); HEIMBACH, Dirk, 40629 Düsseldorf (DE); KELDENICH, Jörg, 42113 Wuppertal (DE)
(86) Internationale Anmeldenummer: PCT/EP2002/010349
(87) Internationale Veröffentlichungsnummer: WO 2003/028719

(56) Entgegenhaltungen:
- EP-A- 0 563 475
- WO-A-94/14434
- WO-A-97/32874
- WO-A-98/52925
- WO-A-99/33800

## Beschreibung

Die vorliegende Erfindung betrifft 2,5-Diamidoindol-Derivate, ein Verfahren zu ihrer Herstellung sowie ihre Verwendung als Arzneimittel bei der Behandlung von Erkrankungen bei Menschen und/oder Tieren.

Endothelzellen sowie eine Vielzahl weiterer Zelltypen produzieren Endothelin (ET), ein Polypeptidhormon mit 21 Aminosäureresten. Endothelin ist ein potenter Vasokonstriktor, der aus dem Prohormon "Big Endothelin" (bET, 38 Aminosäurereste) durch Spaltung der Peptidbindung zwischen Trp 21 und Val 22 gebildet wird. Die Umwandlung von Prohormon bET in die aktive Form ET erfolgt durch eine Metalloprotease, das Endothelinkonversionsenzym (ECE). Durch Inhibition von ECE wird also die Konversion von bET zum biologisch aktiven ET verhindert.

ET ist ein potenter Konstriktor arterieller and venöser Gefäße. Deshalb ist anzunehmen, dass abnorme ET Level direkt an der Pathophysiologie verschiedener Erkrankungen beteiligt sind. Erhöhte Endothelinspiegel werden bei kardiovaskulären Erkrankungen wie essentieller, pulmonaler und maligner Hypertonie, bei fortgeschrittener Atherosklerose, Herzinfarkt, Herz- und Nierenversagen beobachtet (Miyauchi T, Masaki T.; Pathophysiology of endothelin in the cardiovascular system. Annu Rev Physiol. 1999;61:391-415). Zusätzliche Hinweise ergeben sich aus der Analyse verschiedener Tiermodelle für ischämische Erkrankungen wie Angina pectoris, Herzinfarkt und Schlaganfall und für kardiale Arrythmie und renale Dysfunktion. Die Reduktion von ET-Spiegeln führt bei diesen unterschiedlichen Krankheitsbildem zu einer Verringerung pathologischer Parameter.

Deshalb ist anzunehmen, dass die Behandlung der oben beschriebenen Erkrankungen mit ECE-Inhibitoren zu einer Verbesserung führt (Otter W., Kentsch M.; Endothelin converting enzyme inhibitors, Current opinion in Cardiovascular, Pulmonary & Renal Investigational drugs 2000 2(4):316-329).

Aufgabe der vorliegenden Erfindung ist die Bereitstellung von Arzneimitteln für die Behandlung von kardiovaskulären Erkrankungen insbesondere der oben beschriebenen Erkrankungen.

Die Aufgabe der vorliegenden Erfindung wird durch Verbindungen der Formel (I) gelöst, die als ECE-Inhibitoren wirken.

Strukturell ähnliche Verbindungen sind in anderen Indikationen bzw. für andere Wirkmechanismen bekannt. So beschreibt beispielsweise WO 99/33800 Indol-Derivate als Faktor Xa-Inhibitoren, WO 94/14434 beschreibt Indol-Derivate als Endothelinrezeptor-Antagonisten und EP-A 0 655 439 beschreibt Glycoprotein IIB/IIIA-Antagonisten zur Inhibition der Blutplättchen-Aggregation.

Gegenstand der vorliegenden Erfindung sind Verbindungen der Formel (I) 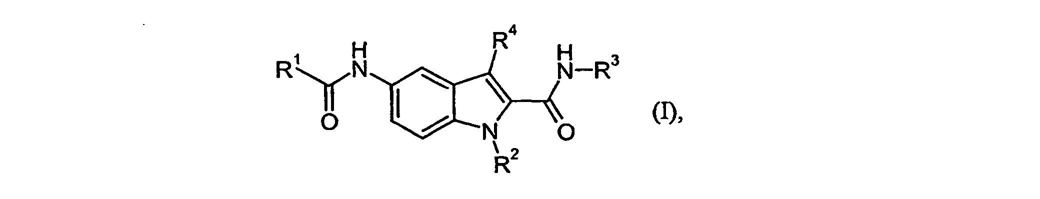 in welcher
- R¹: für (C₅-C₁₅)-Alkyl, (C₅-C₁₅)-Alkenyl oder (CH₂)ₙG steht,
worin
G für Cycloalkyl oder für einen 5- oder 6-gliedrigen Heterocyclus mit einem oder zwei Sauerstoffatomen steht,
n für 0 bis 4 steht und
Alkyl, Alkenyl und G gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Carboxyl, Alkoxycarbonyl, Amino, Alkylamino, Alkylcarbonylamino und Alkylaminocarbonyl, substituiert sind,
- R²: für (C₁-C₈)-Alkyl, (CH₂)ₘCycloalkyl, (CH₂)ₘHeterocyclyl, (CH₂)ₘAryl oder (CH₂)ₘHeteroaryl steht,
worin
m für 0 bis 4 steht und
Alkyl, Cycloalkyl, Heterocyclyl, Aryl und Heteroaryl gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Alkoxycarbonyl, Amino, Alkylamino, Alkylcarbonylamino, Alkylaminocarbonyl, Alkylaminosulfonyl und Alkylsulfonylamino, substituiert sind,
- R³: für (CH₂)ₒCycloalkyl, (CH₂)ₒHeterocyclyl, (CH₂)ₒAryl oder (CH₂)ₒHeteroaryl steht,
worin
o für 0 bis 4 steht und
Cycloalkyl, Heterocyclyl, Aryl und Heteroaryl gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Hydroxycarbonyl, Alkoxycarbonyl, Amino, Alkylamino, Alkylcarbonylamino, Alkylaminocarbonyl, Alkylaminosulfonyl und Alkylsulfonylamino, substituiert sind,
- R⁴: für Wasserstoff, (C₁-C₄)-Alkyl, (CH₂)ₚCycloalkyl, (CH₂)ₚHeterocyclyl, (CH₂)ₚAryl oder (CH₂)ₚHeteroaryl steht,
worin
p p für 0 bis 4 steht und
Alkyl, Cycloalkyl, Heterocyclyl, Aryl und Heteroaryl gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Hydroxycarbonyl, Alkoxycarbonyl, Amino, Alkylamino, Alkylcarbonylamino, Alkylaminocarbonyl Alkylaminosulfonyl und Alkylsulfonylamino, substituiert sind,
und ihre Salze, Hydrate, Hydrate der Salze und Solvate.

Die Verbindungen der Formel (I) können in Abhängigkeit vom Substitutionsmuster in stereoisomeren Formen, die sich entweder wie Bild und Spiegelbild (Enantiomere) oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemat-Formen lassen sich ebenso wie die Diastereomeren in bekannter Weise in die stereoisomer einheitlichen Bestandteile trennen. Gleichermaßen betrifft die vorliegende Erfindung auch die übrigen Tautomeren der Verbindungen der Formel (I) und deren Salze.

Salze der Verbindungen der Formel (I) können physiologisch unbedenkliche Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Trifluoressigsäure, Essigsäure, Propionsäure, Milchsäure, Weinsäure, Zitronensäure, Fumarsäure, Maleinsäure oder Benzoesäure.

Als Salze können auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methylpiperidin.

Als Hydrate bzw. Solvate werden erfindungsgemäß solche Formen der Verbindungen der Formel (I) bezeichnet, welche in festem oder flüssigem Zustand durch Hydratation mit Wasser oder Koordination mit Lösungsmittelmolekülen eine Molekül-Verbindung bzw. einen Komplex bilden. Beispiele für Hydrate sind Sesquihydrate, Monohydrate, Dihydrate oder Trihydrate. Gleichermaßen kommen auch die Hydrate bzw. Solvate von Salzen der erfindungsgemäßen Verbindungen in Betracht.

Außerdem umfasst die Erfindung auch Prodrugs der erfindungsgemäßen Verbindungen. Als Prodrugs werden erfindungsgemäß solche Formen der Verbindungen der Formel (I) bezeichnet, welche selbst biologisch aktiv oder inaktiv sein können, jedoch unter physiologischen Bedingungen in die entsprechende biologisch aktive Form überführt werden können (beispielsweise metabolisch oder solvolytisch).

Im Rahmen der vorliegenden Erfindung haben die Substituenten, soweit nicht anders angegeben, die folgende Bedeutung:
Alkyl steht für geradliniges oder verzweigtes Alkyl und umfasst, wenn nicht anders angegeben, C₁-C₆-Alkyl, insbesondere Methyl, Ethyl, Propyl, Isopropyl, Butyl, Isobutyl.
(C₅-C₁₅)-Alkyl, C₁-C₈)-Alkyl, (C₁-C₄)-Alkyl steht für geradliniges oder verzweigtes Alkyl mit 5 bis 15, 1 bis 8 bzw. 1 bis 4 Kohlenstoffatomen. Beispielhaft und vorzugsweise seien genannt: Neopentyl, Isoamyl.
Cycloalkyl umfasst gesättigte Kohlenwasserstoffreste mit bis zu 14 C-Atomen, nämlich monocyclisches C₃-C₁₂-Cycloalkyl, vorzugsweise C₃-C₈-Cycloalkyl, wie z.B. Cyclopropyl, Cyclobutyl, Cyclopentyl, Cyclohexyl, Cycloheptyl, Cyclooctyl, Cyclononyl, und polycyclisches Alkyl, d.h. vorzugsweise bicyclisches und tricyclisches, gegebenenfalls spirocyclisches C₇-C₁₄-Cyclolkyl, wie z.B. Bicyclo-[2.2.1]-hept-1-yl, Bicyclo[2.2.1]-hept-2-yl, Bicyclo[2.2.1]-hept-7-yl, Bicyclo[2.2.2]-oct-2-yl, Bicyclo[3.2.1]-oct-2-yl, Bicyclo[3.2.2]-non-2-yl und Adamantyl.
Aryl steht für einen aromatischen Rest mit 6 bis 10 Kohlenstoffatomen. Bevorzugte Arylreste sind Phenyl und Naphthyl.
Alkoxy steht für einen geradkettigen oder verzweigten Alkylrest insbesondere mit 1 bis 6, 1 bis 4 bzw. 1 bis 3 Kohlenstoffatomen, der über ein Sauerstoffatom gebunden ist. Bevorzugt ist ein geradkettiger oder verzweigter Alkoxyrest mit 1 bis 3 Kohlenstoffatomen. Beispielhaft und vorzugsweise seien genannt: Methoxy, Ethoxy, n-Propoxy, Isopropoxy, t-Butoxy, n-Pentoxy und n-Hexoxy.
Alkylthio steht für einen geradkettigen oder verzweigten Alkylrest insbesondere mit 1 bis 6, 1 bis 4 bzw. 1 bis 3 Kohlenstoffatomen, der über ein Schwefelatom gebunden ist. Bevorzugt ist ein geradkettiger oder verzweigter Alkylthiorest mit 1 bis 3 Kohlenstoffatomen. Beispielhaft und vorzugsweise seien genannt: Methylthio, Ethylthio, n-Propylthio, Isopropylthio, t-Butylthio, n-Pentylthio und n-Hexylthio.
Alkoxycarbonyl steht für einen geradkettigen oder verzweigten Alkoxyrest mit 1 bis 6 bzw. 1 bis 4 Kohlenstoffatomen, der über eine Carbonylgruppe verknüpft ist. Bevorzugt ist ein geradkettiger oder verzweigter Alkoxycarbonylrest mit 1 bis 4 Kohlenstoffatomen. Beispielhaft und vorzugsweise seien genannt: Methoxycarbonyl, Ethoxycarbonyl, n-Propoxycarbonyl, Isopropoxycarbonyl und t-Butoxycarbonyl.
Alkylamino steht für eine Amino-Gruppe, die einen geradkettigen oder verzweigten bzw. zwei gleiche oder verschiedene geradkettige oder verzweigte Alkylsubstituenten mit vorzugsweise jeweils 1 bis 6, 1 bis 4 bzw. 1 bis 2 Kohlenstoffatome aufweist. Bevorzugt sind geradkettige oder verzweigte Alkylamino-Reste mit jeweils 1 bis 4 Kohlenstoffatomen. Beispielhaft und vorzugsweise seien genannt: Methylamino, Ethylamino, n-Propylamino, Isopropylamino, t-Butylamino, n-Pentylamino, n-Hexylamino, *N*,*N*-Dimethylamino, *N*,*N*-Diethylamino, *N*-Ethyl-*N*-methylamino, *N*-Methyl-*N*-n-propylamino, *N*-Isopropyl*-N*-n-propylamino, *N*-t-Butyl-*N*-methylamino, *N-Ethyl-N*-n-pentylamino und *N*-n-Hexyl-*N*-methylamino.
Alkylcarbonylamino (Acylamino) steht im Rahmen der Erfindung für eine Amino-Gruppe mit einem geradkettigen oder verzweigten über eine Carbonylgruppe verknüpften Alkylrest, der vorzugsweise 1 bis 6, 1 bis 4 bzw. 1 bis 2 Kohlenstoffatome aufweist und über die Carbonylgruppe verknüpft ist. Bevorzugt ist ein Monoacylamino-Rest mit 1 bis 2 Kohlenstoffatomen. Beispielhaft und vorzugsweise seien genannt: Acetamido, Propionamido, n-Butyramido und Pivaloylamido.
Alkylaminocarbonyl steht für eine Amino-Gruppe, die über eine Carbonylgruppe verknüpft ist und die einen geradkettigen oder verzweigten bzw. zwei gleiche oder verschiedene geradkettige oder verzweigte Alkylsubstituenten mit vorzugsweise jeweils 1 bis 4 bzw. 1 bis 2 Kohlenstoffatomen aufweist. Beispielhaft und vorzugsweise seien genannt: Methylaminocarbonyl, Ethylaminocarbonyl, Isopropylaminocarbonyl, t-Butylaminocarbonyl, *N,N-*Dimethylaminocarbonyl, *N,N-*Diethylaminocarbonyl, *N*-Ethyl-*N*-methyl-aminocarbonyl und *N*-t-Butyl-*N*-methylaminocarbonyl.
Heteroaryl steht für einen 5- bis 10-gliedrigen aromatischen Heterocyclus mit bis zu 3 Heteroatomen aus der Reihe S, O und/oder N. Beispielhaft und vorzugsweise seien genannt: Pyridyl, Pyrimidyl, Thienyl, Furyl, Pyrrolyl, Thiazolyl, N-Triazolyl, Oxazolyl oder Imidazolyl. Bevorzugt sind Pyridyl, Furyl, Thiazolyl und N-Triazolyl.
Heterocyclyl steht für einen gegebenenfalls über ein Stickstoffatom gebundenen 3-bis 8- gliedrigen gesättigten oder teilweise ungesättigten Heterocyclus, der bis zu 3 Heteroatome aus der Reihe S, O und N enthalten kann. Beispielhaft und vorzugsweise seien genannt: Morpholinyl, Piperidinyl, Piperazinyl, Methylpiperazinyl, Thiomorpholinyl, Pyrrolidinyl, sowie 3-, 7- und 8-gliedrige Heterocyclen, wie beispielsweise Aziridine (z.B. 1-Azacyclopropan-1-yl), Azetidine (z.B. 1-Azacyclobutan-1-yl) und Azepine (z.B. 1-Azepan-1-yl) ein. Die ungesättigten Vertreter können 1 bis 2 Doppelbindungen im Ring enthalten.
Halogen steht für Fluor, Chlor, Brom oder Jod, wobei Fluor und Chlor bevorzugt sind, wenn nichts anderes angegeben ist.
Alkylaminosulfonyl steht für eine Amino-Gruppe, die über eine Sulfonylgruppe verknüpft ist und die einen geradkettigen oder verzweigten bzw. zwei gleiche oder verschiedene geradkettige oder verzweigte Alkylsubstituenten mit vorzugsweise 1 bis 4 bzw. 1 bis 2 Kohlenstoffatomen aufweist. Beispielhaft und vorzugsweise seien genannt: Methylaminosulfonyl, Ethylaminosulfonyl, Isopropylaminosulfonyl, t-Butylaminosulfonyl, *N,N*-Dimethylaminosulfonyl, *N,N-*Diethylaminosulfonyl, *N-*Ethyl*-N*-methyl-anunosulfonyl und *N*-t-Butyl-*N*-methylaminosulfonyl.
Alkylsulfonylamino steht für eine Sulfonyl-Gruppe, die über eine Amino-Gruppe verknüpft ist und die einen geradkettigen oder verzweigten Alkylsubstituenten mit vorzugsweise 1 bis 4 bzw. 1 bis 2 Kohlenstoffatomen aufweist. Beispielhaft und vorzugsweise seien genannt: Methylsulfonylamino, Ethylsulfonylamino, Isopropylsulfonylamino, t-Butylsulfonylamino.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Bevorzugt sind Verbindungen der Formel (I), in welcher
- R¹: für (C₅-C₁₅)-Alkyl oder (CH₂)ₙCycloalkyl steht,
worin
n für 0 bis 4 steht und
Alkyl und Cycloalkyl gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Carboxyl, Alkoxycarbonyl, Alkylcarbonylamino und Alkylaminocarbonyl, substituiert sind,
- R²: für (C₁-C₈)-Alkyl, (CH₂)ₘCycloalkyl, (CH₂)ₘHeterocyclyl, (CH₂)ₘAryl oder (CH₂)ₘHeteroaryl steht,
worin
m für 0 bis 4 steht und
Alkyl, Cycloalkyl, Heterocyclyl, Aryl und Heteroaryl gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Alkoxycarbonyl, Amino, Alkylamino, Alkylcarbonylamino, Alkylaminocarbonyl, Alkylaminosulfonyl und Alkylsulfonylamino, substituiert sind,
- R³: für (CH₂)ₒCycloalkyl, (CH₂)ₒHeterocyclyl, (CH₂)ₒAryl oder (CH₂)ₒHeteroaryl steht,
worin
o für 0 bis 4 steht und
Cycloalkyl, Heterocyclyl, Aryl und Heteroaryl gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Hydroxycarbonyl, Alkoxycarbonyl, Amino, Alkylamino, Alkylcarbonylamino, Alkylaminocarbonyl Alkylaminosulfonyl und Alkylsulfonylamino, substituiert sind,
- R⁴: für Wasserstoff, (C₂-C₄)-Alkyl, (CH₂)ₚCycloalkyl, (CH₂)ₚHeterocyclyl, (CH₂)ₚAryl oder (CH₂)ₚHeteroaryl steht,
worin
p für 0 bis 4 steht und
Alkyl, Cycloalkyl, Heterocyclyl, Aryl und Heteroaryl gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Hydroxycarbonyl, Alkoxycarbonyl, Amino, Alkylamino, Alkylcarbonylamino, Alkylaminocarbonyl Alkylaminosulfonyl und Alkylsulfonylamino, substituiert sind,
und ihre Salze, Hydrate, Hydrate der Salze und Solvate.

Besonders bevorzugt sind Verbindungen der Formel (I), in welcher
- R¹: für Neopentyl, (Bicyclo[2.2.1]heptyl)methyl, Cyclohexylmethyl, Cyclobutylmethyl, Cyclopentylmethyl, 2,2-Dimethyl-1-butyl, 2-Ethyl-2-metyl-1-butyl, (1-Methylcyclopentyl)methyl, 1-Methylcyclohexyl, 4-Hydroxy-2,2-dimethyl-1-butyl oder 2,2-Dimethyl-1-but-3-enyl steht,
- R²: für (C₁-C₄)-Alkyl, das durch Hydroxy oder Fluor substituiert sein kann oder für Benzyl, das gegebenenfalls durch 1 oder 2 Substituenten, unabhängig voneinander ausgewählt aus der Gruppe von Fluor, Chlor, Brom, Methyl und Trifluormethyl substituiert ist, steht,
- R³: für Phenyl, Pyridyl oder Pyrimidyl steht, die ihrerseits gegebenenfalls durch einen Substituenten ausgewählt aus der Gruppe von Fluor, Chlor, Trifluormethyl, Methyl, Ethyl, Methoxy, Ethoxy, n-Propoxy, Isopropoxy, Amino, Hydroxy, Hydroxycarbonyl, (C₁-C₃)-Alkylcarbonylamino und Mono-(C₁-C₄)--Alkylaminocarbonyl substituiert sind,
- R⁴: für Wasserstoff steht
und ihre Salze, Hydrate, Hydrate der Salze und Solvate.

Bevorzugt sind Verbindungen der Formel (I), in welcher
- R¹: für Neopentyl steht.

Ebenfalls bevorzugt sind Verbindungen der Formel (I), in welcher
- R²: für Benzyl steht, das bis zu zweifach, unabhängig voneinander, durch Alkyl oder Halogen, bevorzugt Fluor, substituiert sein kann.

Ebenfalls bevorzugt sind Verbindungen der Formel (I), in welcher
- R³: für Phenyl steht, das bis zu zweifach, unabhängig voneinander, durch Alkyl oder Alkoxy substituiert sein kann.

Ebenfalls bevorzugt sind Verbindungen der Formel (I), in welcher
- R³: für Phenyl, Pyridyl oder Pyrimidyl steht,
die ihrerseits gegebenenfalls durch einen Substituenten ausgewählt aus der Gruppe von Fluor, Chlor, Trifluormethyl, Methyl, Ethyl, Methoxy, Ethoxy, n-Propoxy, Isopropoxy, Amino, Hydroxy, Hydroxycarbonyl, (C₁-C₃)-Alkylcarbonylamino und Mono-(C₁-C₄)-Alkylaminocarbonyl substituiert sind.

Ebenfalls bevorzugt sind Verbindungen der Formel (I), in welcher
- R⁴: für Wasserstoff steht.

Ebenfalls bevorzugt sind Verbindungen der Formel (I),
in welcher
- R¹: für (C₅-C₁₀)-Alkyl oder (CH₂)ₙ(C₄-C₇)-Cycloalkyl, bevorzugt (CH₂)ₙCyclobutyl, (CH₂)ₙCyclopentyl, (CH₂)ₙCyclohexyl oder (CH₂)ₙBicyclo[2.2.1]-heptyl, steht,
worin
n für 1 bis 3 steht und
Alkyl und Cycloalkyl, gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Carboxyl, Alkoxycarbonyl, Alkylcarbonylamino und Alkylaminocarbonyl, substituiert sind,
- R²: für (C₁-C₄)-Alkyl, (CH₂)ₘCycloalk-yl oder (CH₂)ₘAryl steht,
worin
m für 0 bis 4 steht und
Alkyl, Cycloalkyl, und Aryl gegebenenfalls durch 1 bis 3 Substituenten, unabhängig voneinander ausgewählt aus der Gruppe von Halogen, Trifluormethyl, Cyano, Nitro, Alkyl, Alkoxy, Amino, Alkylamino, Alkylcarbonylamino und Alkylaminocarbonyl, substituiert sind,
- R³: für (CH₂)ₒAryl oder (CH₂)ₒHeteroaryl steht,
worin
o für 0 bis 3 steht und
Aryl und Heteroaryl gegebenenfalls durch 1 bis 3 Substituenten, unabhängig voneinander ausgewählt aus der Gruppe von Halogen, Trifluormethyl, Cyano, Nitro, Alkyl, Alkoxy, Amino, Alkylamino, Alkylcarbonylamino und Alkylaminocarbonyl, substituiert sind,
- R⁴: für Wasserstoff, (C₁-C₄)-Alkyl oder (CH₂)ₚAryl steht,
worin
p für 1 bis 4 steht und Alkyl, und Aryl gegebenenfalls durch 1 bis 3 Substituenten, unabhängig voneinander ausgewählt aus der Gruppe von Halogen, Trifluormethyl, Cyano, Nitro, Alkyl, Alkoxy, Alkylcarbonylamino und Alkylaminocarbonyl, substituiert sind,
und ihre Salze, Hydrate, Hydrate der Salze und Solvate.

Besonders bevorzugt sind Verbindungen der Formel (I),
in welcher
- R¹: für Neopentyl, Bicyclo[2.2.1]heptyl, Cyclohexylmethyl, Cyclobutylmethyl, Cyclopentylmethyl, 2,2-Dimethyl-4-butyl, 2,2-Dimethyl-1-butyl oder 2-Ethyl-2-metyl-1-butyl steht, die ihrerseits gegebenenfalls durch 1 bis 2 Substituenten, unabhängig voneinander ausgewählt aus der Gruppe von Halogen, Cyano, Alkyl, Alkoxy, Alkylcarbonylamino und Alkylaminocarbonyl, substituiert sind,
- R²: für (C₁-C₄)-Alkyl oder (CH₂)ₘPhenyl steht,
worin
m für 0 bis 4 steht und
Alkyl und Phenyl gegebenenfalls durch 1 bis 2 Substituenten, unabhängig voneinander ausgewählt aus der Gruppe von Halogen, Trifluormethyl, Cyano, Alkyl, Alkoxy, Alkylcarbonylamino und Alkylaminocarbonyl, substituiert sind,
- R³: für (CH₂)ₒPhenyl, (CH₂)ₒPyridyl, (CH₂)ₒThienyl oder (CH₂)ₒPyrimidyl steht,
worin
o für 0 bis 3 steht und
Phenyl, Pyridyl, Thienyl und Pyrimidyl ihrerseits gegebenenfalls durch 1 bis 3 Substituenten, unabhängig voneinander ausgewählt aus der Gruppe von Halogen, Trifluormethyl, Cyano, Nitro, Alkyl, Alkoxy, Alkylcarbonylamino und Alkylaminocarbonyl, substituiert sind,
- R⁴: für Wasserstoff oder (C₁-C₄)-Alkyl steht,
worin Alkyl gegebenenfalls durch 1 bis 3 Substituenten, unabhängig voneinander ausgewählt aus der Gruppe von Halogen und Trifluormethyl, substituiert ist,
und ihre Salze, Hydrate, Hydrate der Salze und Solvate.

Ebenfalls besonders bevorzugt sind Kombinationen von zwei oder mehreren der oben genannten Vorzugsbereiche.

Gegenstand der vorliegenden Erfindung ist auch ein Verfahren zur Herstellung der Verbindungen der Formel (I), dass dadurch gekennzeichnet ist, dass man entweder

### [A] Verbindungen der Formel (II)

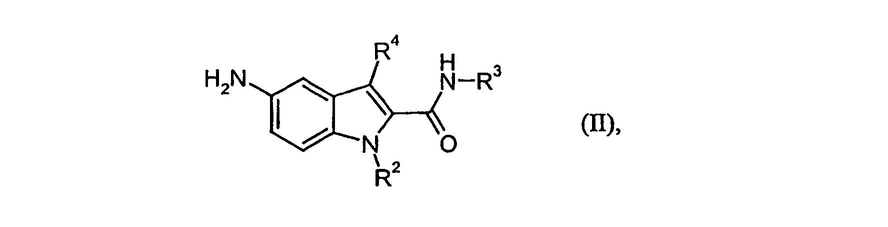

in welcher
R², R³ und R⁴ die oben angegebene Bedeutung aufweisen,
mit Verbindungen der Formel (III) 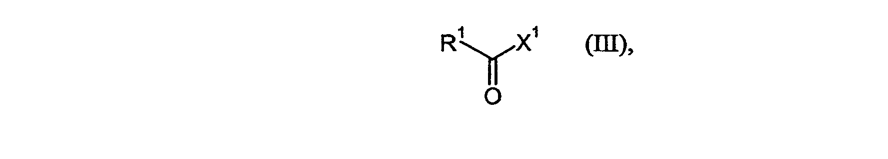 in welcher
- R¹: die oben angegebene Bedeutung aufweist und
- X¹: für Halogen, bevorzugt Brom oder Chlor, oder Hydroxy steht,
oder

### [B] Verbindungen der Formel (XI)

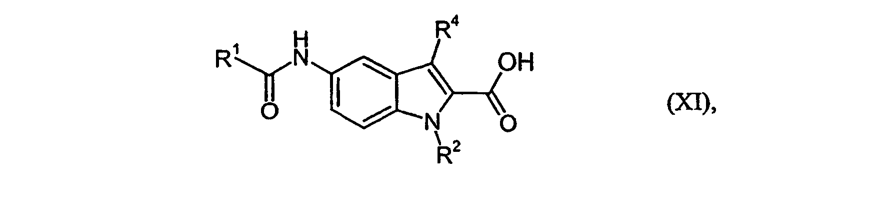

in welcher
R¹, R² und R⁴ die oben angegebene Bedeutung aufweisen,
mit Verbindungen der Formel (VI) 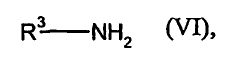 in welcher
- R³: die oben angegebene Bedeutung aufweist,
zu Verbindungen der Formel (I) umsetzt.

Im Verfahrensschritt A für den Fall, dass X¹ für Halogen steht, erfolgt die Umsetzung in inerten Lösungsmitteln, gegebenenfalls in Gegenwart einer Base, bevorzugt in einem Temperaturbereich von 0°C bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 1,2-Dimethoxyethan, 2-Butanon, Dimethylsulfoxid, Acetonitril, Pyridin oder Hexamethylphosphorsäuretriamid, bevorzugt sind Dioxan oder Methylenchlorid.

Basen sind beispielsweise Alkalihydroxide wie Natrium- oder Kaliumhydroxid, oder Alkalicarbonate wie Cäsiumcarbonat, Natrium- oder Kaliumcarbonat, oder Amide wie Lithiumdiisopropylamid, oder andere Basen wie DBU, Triethylamin oder Diisopropyjethylamin, bevorzugt Diisopropylethylamin oder Triethylamin.

Im Verfahrensschritt A für den Fall, dass X¹ für Hydroxy steht, sowie im Verfahrensschritt B erfolgt die Umsetzung von Verbindung (II) mit Verbindung (III) bzw. von Verbindung (XI) mit Verbindung (VI) zu Verbindungen der Formel (I) in inerten Lösungsmitteln, in Gegenwart von üblichen Kondensationsmitteln, gegebenenfalls in Gegenwart einer Base, bevorzugt in einem Temperaturbereich von Raumtemperatur bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 1,2-Dimethoxyethan, Dimethylsulfoxid, Acetonitril oder Pyridin, bevorzugt sind Tetrahydrofuran, Dimethylformamid, 1,2-Dichlorethan oder Methylenchlorid.

Übliche Kondensationsmittel sind beispielsweise Carbodiimide wie z.B. N,N'-Diethyl-, N,N,'-Dipropyl-, N,N-Diisopropyl-, N,N'-Dicyclohexylcarbodiimid, N-(3-Dimethylaminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphoniumhexafluorophosphat, oder O-(Benzotriazol-1-yl)-N,N,N,N-tetra-methyluronium-hexafluorophosphat (HBTU), 2-(2-Oxo-1-(2H)-pyridyl)-1,1,3,3-tetramethyluroniumtetrafluoro-borat (TPTU) oder O-(7-Azabenzotriazol-1-yl)-N,N,N,N-tetramethyl-uroniumhexafluorophosphat (HATU), oder 1-Hydroxybenztriazol (HOBt), oder Benzotriazol-1-yloxytris(dimethylamino)-phosphoniumhexafluoro-phosphat (BOP), oder Mischungen aus diesen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Bevorzugt ist die Kombination von N-(3-Dimethylaminoisopropyl)-N'-ethylcarbodiimid-Hydrochlorid (EDC), 1-Hydroxybenztriazol (HOBt) und Triethylamin; O-(7-Azabenzotriazol-1-yl)-N,N,N',N'-tetramethyl-uroniumhexafluorophosphat (HATU) und Triethylamin oder N-(3-Dimethylaminoisopropyl)-N'-ethylcarbodiimid-Hydrochlorid (EDC) und 4- Dimethylaminopyridin in Dimethylformamid oder Carbonyldiimidazol in 1,2-Dichlorethan.

Zur Herstellung der Verbindungen der Formel (II) aus [A] setzt man Verbindungen der Formel (IV) 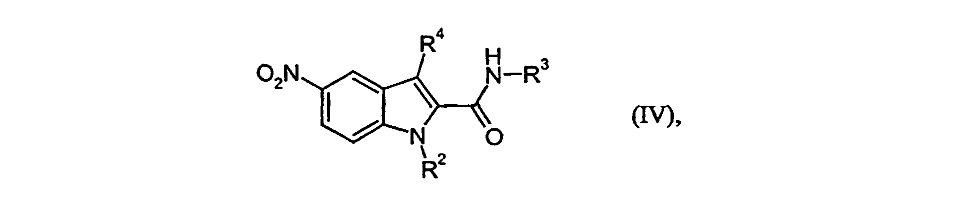 in welcher
R², R³ und R⁴ die oben angegebene Bedeutung aufweisen,
mit Reduktionsmitteln in inerten Lösungsmitteln um.

Verbindungen der Formel (IV) können auf zwei unterschiedlichen Wegen hergestellt werden.

**[A1]** Zum einen setzt man Verbindungen der Formel (V) 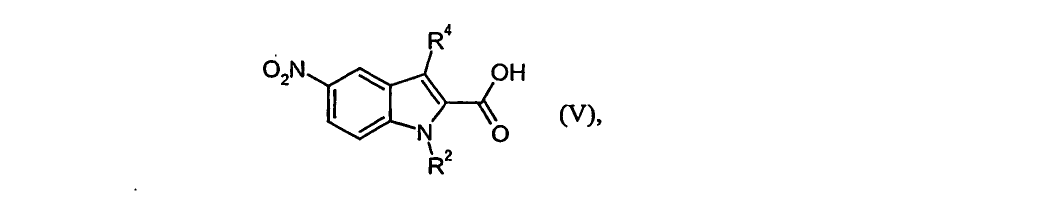 in welcher
- R² und R⁴: die oben angegebene Bedeutung aufweisen,
entweder mit Verbindungen der Formel (VI) 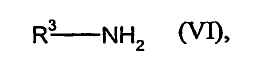 in welcher
- R³: die oben angegebene Bedeutung aufweist,
unter den für die Umsetzung von Verbindungen der Formel (II) mit Verbindungen der Formel (III) zu Verbindungen der Formel (I) beschriebenen Reaktionsbedingungen für den Fall, dass X¹ für Hydroxy steht, um.

Oder es werden Verbindungen der Formel (V) zunächst mit Thionylchlorid und anschließend mit Verbindungen der Formel (VI) in inerten Lösungsmitteln, gegebenenfalls in Gegenwart einer Base, umgesetzt.

Zur Herstellung der Verbindungen der Formel (V), setzt man Verbindungen der Formel (VII), 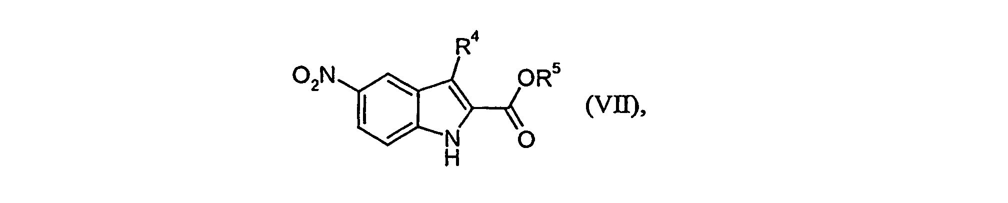 in welcher
- R⁴: die oben angegebene Bedeutung aufweist und
- R⁵: für Alkyl, bevorzugt Methyl oder Ethyl, steht,
mit Verbindungen der Formel (VIII), 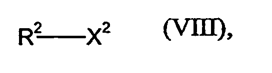 in welcher
- R²: die oben angegebene Bedeutung aufweist und
- X²: für Halogen, bevorzugt Brom oder Chlor, steht,
in Gegenwart einer Base, in inerten Lösungsmitteln, in einem ein- oder zweistufigen Verfahren um. Beim zweistufigen Verfahren wird im ersten Schritt am Indolstickstoffatom alkyliert und in einem zweiten Schritt, nach einem Wechsel der Base, der Ester zur Säure verseift.

**[A2]** Zum anderen können Verbindungen der Formel (IV) hergestellt werden, indem man Verbindungen der Formel (IX) 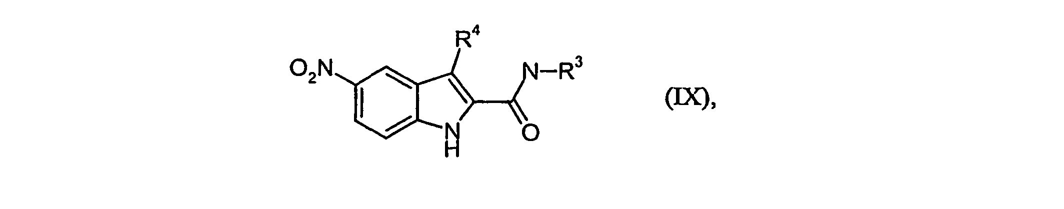 in welcher
- R³ und R⁴: die oben angegebene Bedeutung aufweisen,
mit Verbindungen der Formel (VIII), 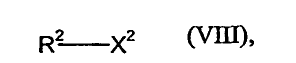 in welcher
- R² und X²: die oben angegebenen Bedeutungen aufweisen,
in Gegenwart einer Base, in inerten Lösungsmitteln umsetzt.

Zur Herstellung von Verbindungen der Formel (IX) setzt man Verbindungen der Formel (X) 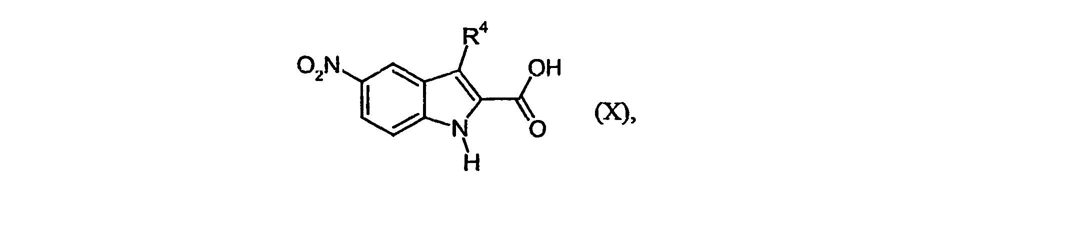 in welcher
- R⁴: die oben angegebene Bedeutung aufweist,
entweder mit Verbindungen der Formel (VI) 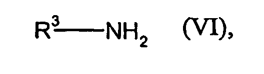 in welcher
- R³: die oben angegebene Bedeutung aufweist,
unter den für die Umsetzung von Verbindungen der Formel (II) mit Verbindungen der Formel (III) zu Verbindungen der Formel (I) beschriebenen Reaktionsbedingungen für den Fall, dass X¹ für Hydroxy steht, um.

Oder es werden Verbindungen der Formel (X) zunächst mit Thionylchlorid und anschließend mit Verbindungen der Formel (VI) in inerten Lösungsmitteln, gegebenenfalls in Gegenwart einer Base, umgesetzt.

Zur Herstellung der Verbindungen der Formel (X) wird die Esterfunktion von Verbindungen der Formel (VII) 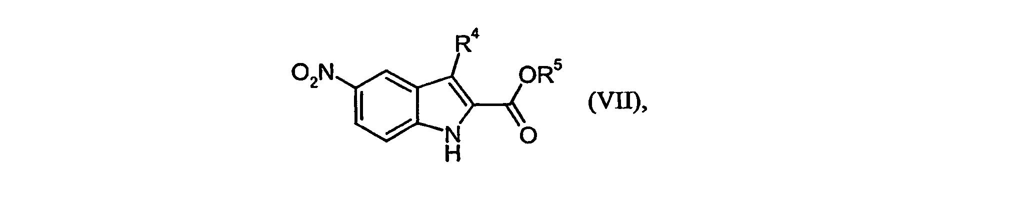 in welcher
- R⁴ und R⁵: die oben angegebene Bedeutung aufweisen,
gespalten.

Zur Herstellung der Verbindungen der Formel (XI) aus [B] wird die Esterfunktion von Verbindungen der Formel (XII) 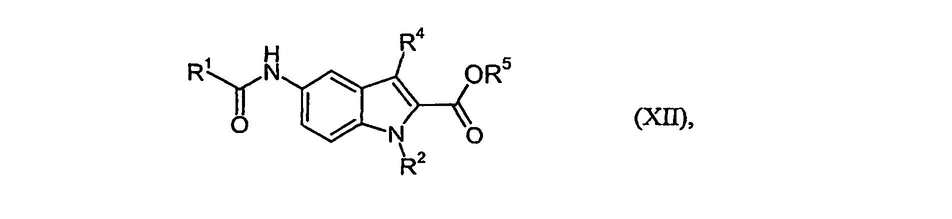 in welcher
- R¹, R² , R⁴ und R⁵: die oben angegebene Bedeutung aufweisen,
gespalten.

Verbindungen der Formel (XII) können auf zwei unterschiedlichen Wegen hergestellt werden.

**[B1]** Zum einen reduziert man die Nitrogruppe in Verbindungen der Formel (XIII) 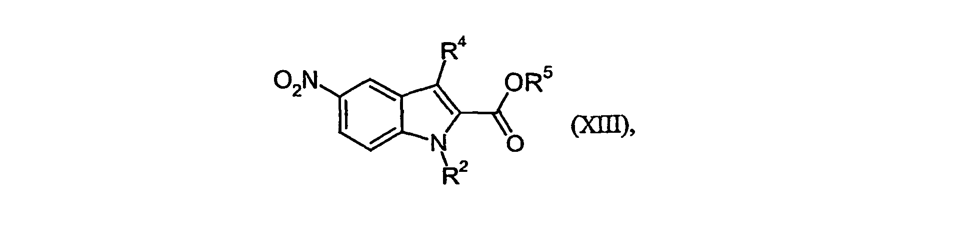 in welcher
R² , R⁴ und R⁵ die oben angegebene Bedeutung aufweisen,
und setzt anschließend mit Verbindungen der Formel (III) 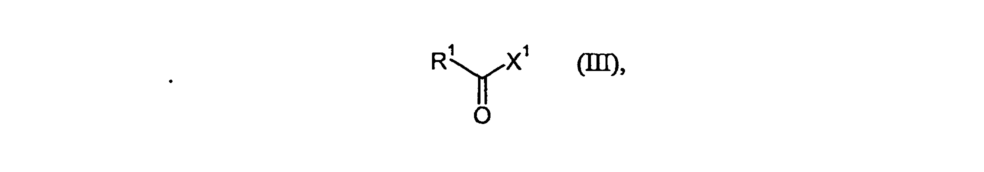 in welcher
- R¹ und X¹: die oben angegebenen Bedeutungen aufweisen,
um.

Zur Herstellung der Verbindungen der Formel (XIII), setzt man Verbindungen der Formel (VII), 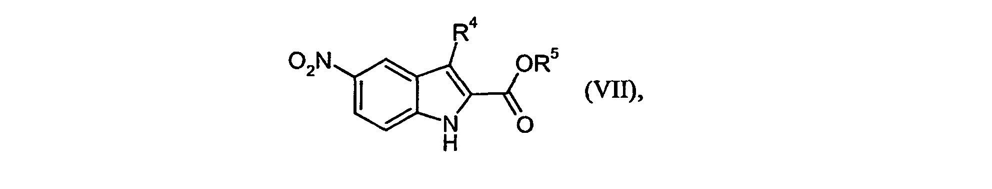 in welcher
- R⁴ und R⁵: die oben angegebenen Bedeutungen aufweisen
mit Verbindungen der Formel (VIII), 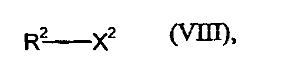 in welcher
- R² und X²: die oben angegebenen Bedeutungen aufweisen,
in Gegenwart einer Base in inerten Lösungsmitteln um.

[**B2**] Zum anderen können Verbindungen der Formel (XII) hergestellt werden, indem man Verbindungen der Formel (XIV) 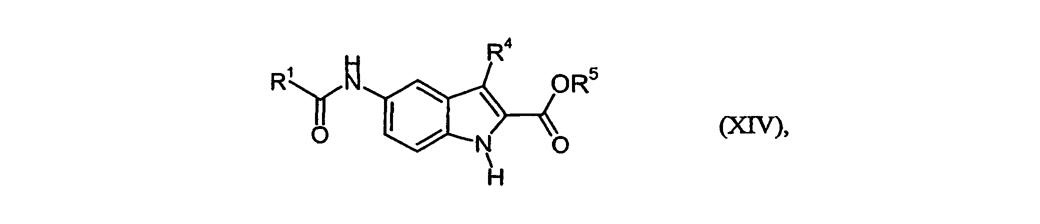 in welcher
- R¹ , R⁴ und R⁵: die oben angegebene Bedeutung aufweisen,
mit Verbindungen der Formel (VIII), 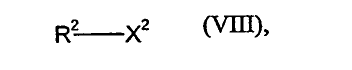 in welcher
- R² und X²: die oben angegebenen Bedeutungen aufweisen,
in Gegenwart einer Base in inerten Lösungsmitteln umsetzt.

Zur Herstellung von Verbindungen der Formel (XIV) reduziert man die Nitrogruppe in Verbindungen der Fonnel (VII) 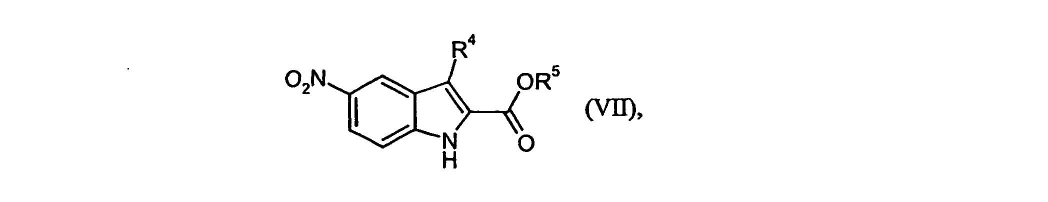 in welcher
R⁴ und R⁵ die oben angegebene Bedeutung aufweisen,
und setzt anschließend mit Verbindungen der Formel (III) 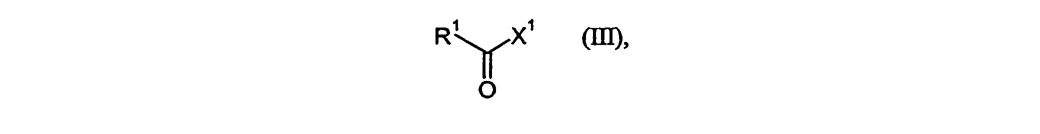 in welcher
- R¹ und X¹: die oben angegebenen Bedeutungen aufweisen,
um.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, Glykoldimethylether oder Diethylenglykoldimethylether, Alkohole wie Methanol, Ethanol, Propanol, Isopropanol oder Butanol, Kohlenwasserstoffe wie Benzol, Xylol, Toluol, Hexan, Cyclohexan oder Erdölfraktionen, oder andere Lösungsmittel wie Nitromethan, Ethylacetat, Aceton, Dimethylformamid, Dimethylacetamid, 1,2-Dimethoxyethan, Dimethylsulfoxid, Acetonitril oder Pyridin.

Als Basen eignen sich die üblichen anorganischen oder organischen Basen. Hierzu gehören bevorzugt Alkali- und Erdalkalihydroxide wie beispielsweise Lithium, Natrium- oder Kaliumhydroxid oder Alkali- und Erdalkalicarbonate wie Cäsiumcarbonat, Natrium- oder Kaliumcarbonat oder Natrium- oder Kaliummethanolat oder Natrium- oder Kaliumethanolat oder Kalium-tert.-butylat oder Amide wie Natriumamid, Lithium-bis-(trimethylsilyl)amid oder Lithiumdiisopropylamid, oder metallorganische Verbindungen wie Butyllithium oder Phenyllithium, oder Amine wie Triethylamin, Diisopropylethylamin, Diisopropylamin, N-Methylmorpholin, 4-Dimethylaminopyridin oder Pyridin, oder andere Basen Natriumhydrid oder DBU. Gegebenenfalls werde zusätzlich zu den Basen Additive wie Kronenether (z.B. 18-Krone-6) oder anorganische Salze wie z.B. Natriumiodid oder Kupfer(I)bromid verwendet.

Reduktionsmittel sind beispielsweise Zinndichlorid, Titantrichlorid oder Palladium auf Aktivkohle und Wasserstoff, wobei Palladium auf Aktivkohle gegebenenfalls unter Zusatz von Ammoniumacetat und/oder Essigsäure verwendet wird.

Der Reaktionsschritt (IV) -> (II) sowie die erste Stufe (Reduktion) in den Reaktionsschritten (XIII) + (III) -> (XII) bzw. (VII) + (III) -> (XIV) erfolgt vorzugsweise mit Zinndichlorid in Ethanol, Methanol oder Dimethylformamid oder mit Palladium auf Kohle in Gegenwart von Ammoniumformiat in Essigsäureethylester/Ethanol bevorzugt in einem Temperaturbereich von Raumtemperatur bis zum Rückfluss der Lösungsmittel bei Normaldruck bis 3 bar.

Die Reaktionsschritte (V) + (VI) -> (IV) und (X) + (VI) -> (IX) erfolgen in der ersten Stufe vorzugsweise mit einem Überschuss an Thionylchlorid als Lösungsmittel, bevorzugt in einem Temperaturbereich von 50°C bis zum Rückfluss der Reaktionspartner bei Normaldruck. In der zweiten Stufe wird vorzugsweise in Methylenchlorid mit Triethylamin als Base, bevorzugt in einem Temperaturbereich von 0°C bis 40°C bei Normaldruck umgesetzt.

Der Reaktionsschritt (VII) + (VIII) -> (V) erfolgt beim einstufigen Verfahren vorzugsweise in Dimethylsulfoxid mit Kalium- oder Natriumhydroxid als Base, bevorzugt in einem Temperaturbereich von 0°C bis 40°C bei Normaldruck.

Bei der ersten Stufe des zweistufigen Verfahrens und bei den Reaktionsschritten (XI) + (VIII) -> (IV) ; (VII) + (VIII) -> (XIII) ; (XIV) + (VIII) -> (XII) wird die Alkylierung vorzugsweise in Dimethylsulfoxid mit Natriumhydrid als Base oder in THF mit Kalium-tert-butylat als Base und unter Zusatz von Kronenether, bevorzugt in einem Temperaturbereich von Raumtemperatur bis 50°C bei Normaldruck durchgeführt. Für den Fall, dass R² in Verbindungen (VIII) für einen aromatischen Rest steht, erfolgt die Umsetzung (VII) + (VIII) -> (XIII) in Gegenwart von Kaliumcarbonat als Base unter dem Zusatz von Kupfer(I)bromid.

Die Verseifung in der zweiten Stufe des Reaktionsschritts (VII) + (VIII) -> (V) wird vorzugsweise in Dimethylsulfoxid mit Kalium- oder Natriumhydroxid als Base, bevorzugt in einem Temperaturbereich von 0°C bis 40°C bei Normaldruck durchgeführt.

Die Reaktionsschritte (VII) -> (X) sowie (XII) -> (XI) erfolgen vorzugsweise in Methanol und THF mit wässriger Lithiumhydroxidlösung als Base, bevorzugt in einem Temperaturbereich von RT bis 90°C bei Normaldruck.

Die zweite Stufe (Acylierung) in den Reaktionsschritten (XIII) + (III) -> (XII) bzw. (VII) + (III) -> (XIV) erfolgt vorzugsweise in Dichlormethan oder THF als Lösungsmittel in Gegenwart von Triethylamin als Base in einem Temperaturbereich von 0°C bis 40°C bei Normaldruck.

Die Verbindungen der Formel (III), (VI) und (VIII) sind dem Fachmann an sich bekannt oder lassen sich nach üblichen, literaturbekannten Verfahren herstellen.

Die Verbindungen der Formel (VII) sind dem Fachmann an sich bekannt oder lassen sich nach üblichen, literaturbekannten Verfahren herstellen (vgl.: A. Guy, J.-P. Guette, *Synthesis* **1980,** 222-223.

Die oben beschriebene Verfahren können durch folgende Formelschemata beispielhaft erläutert werden:

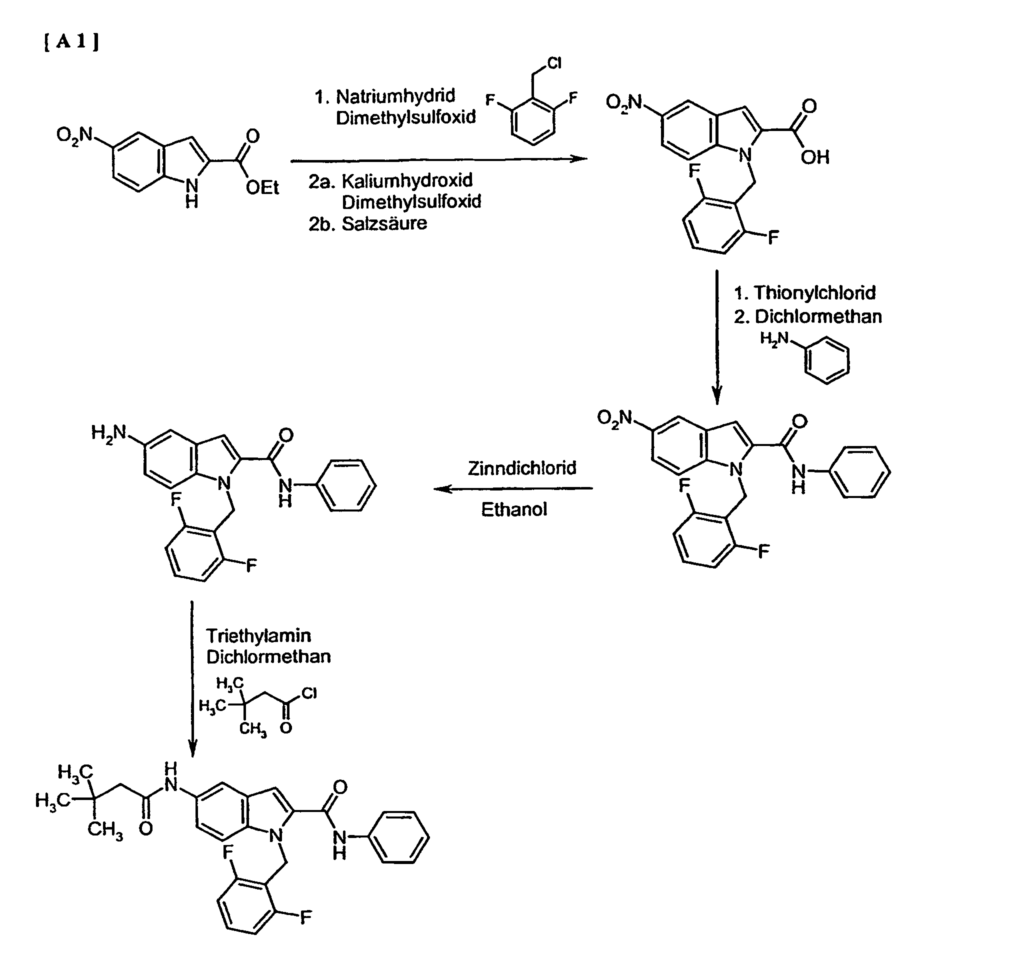

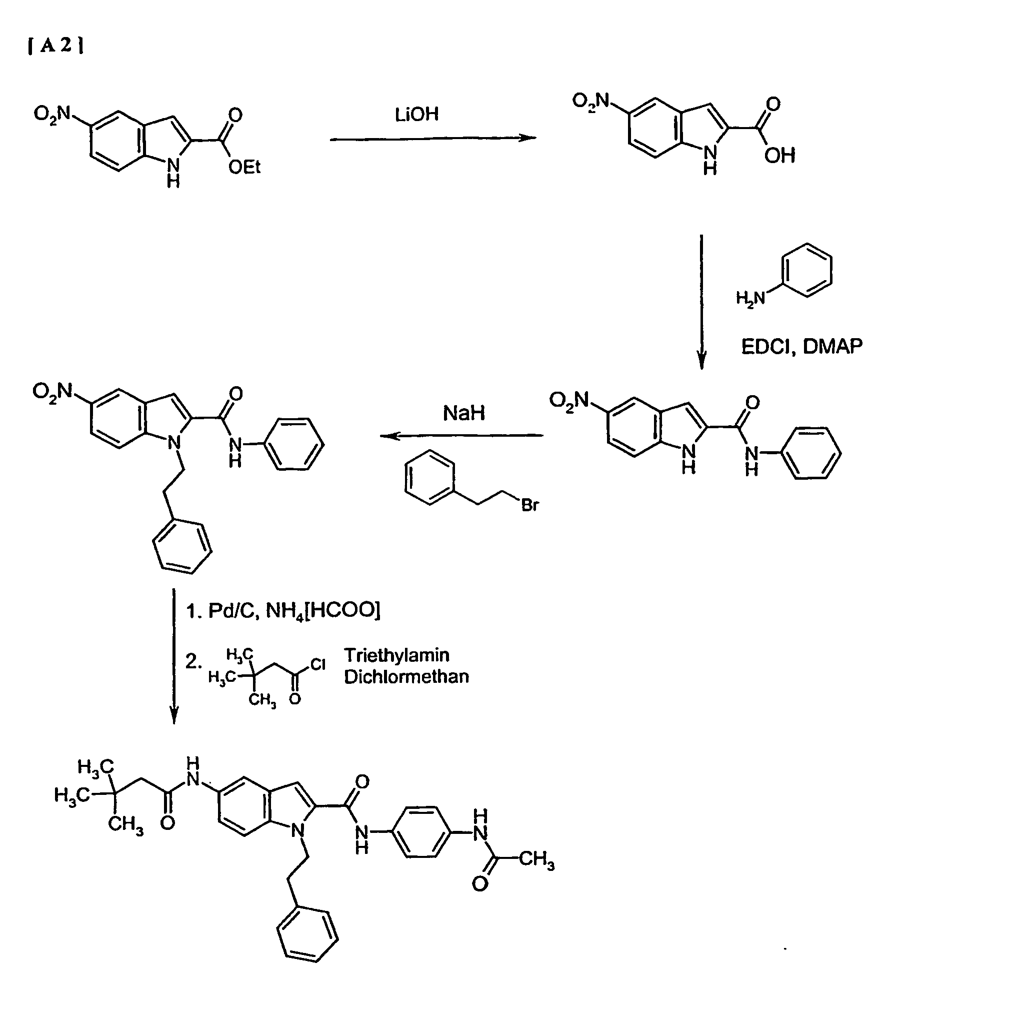

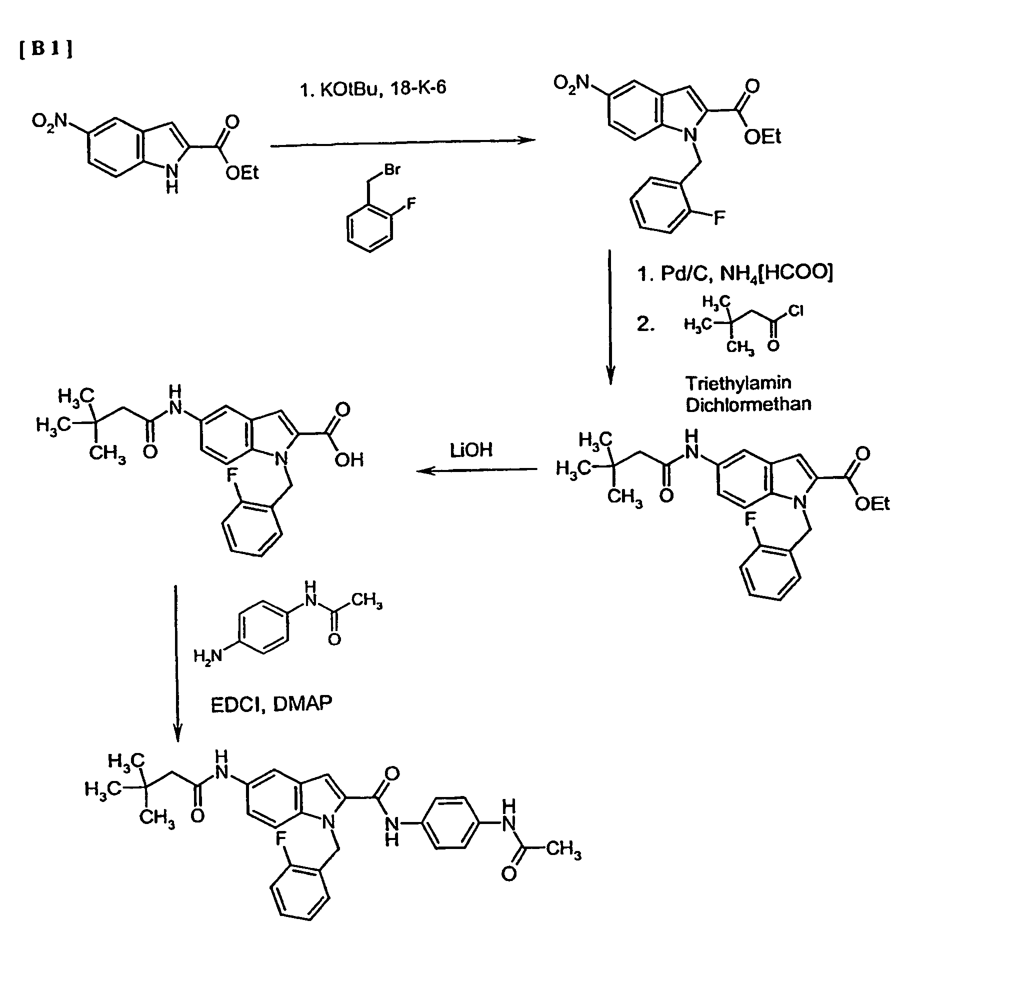

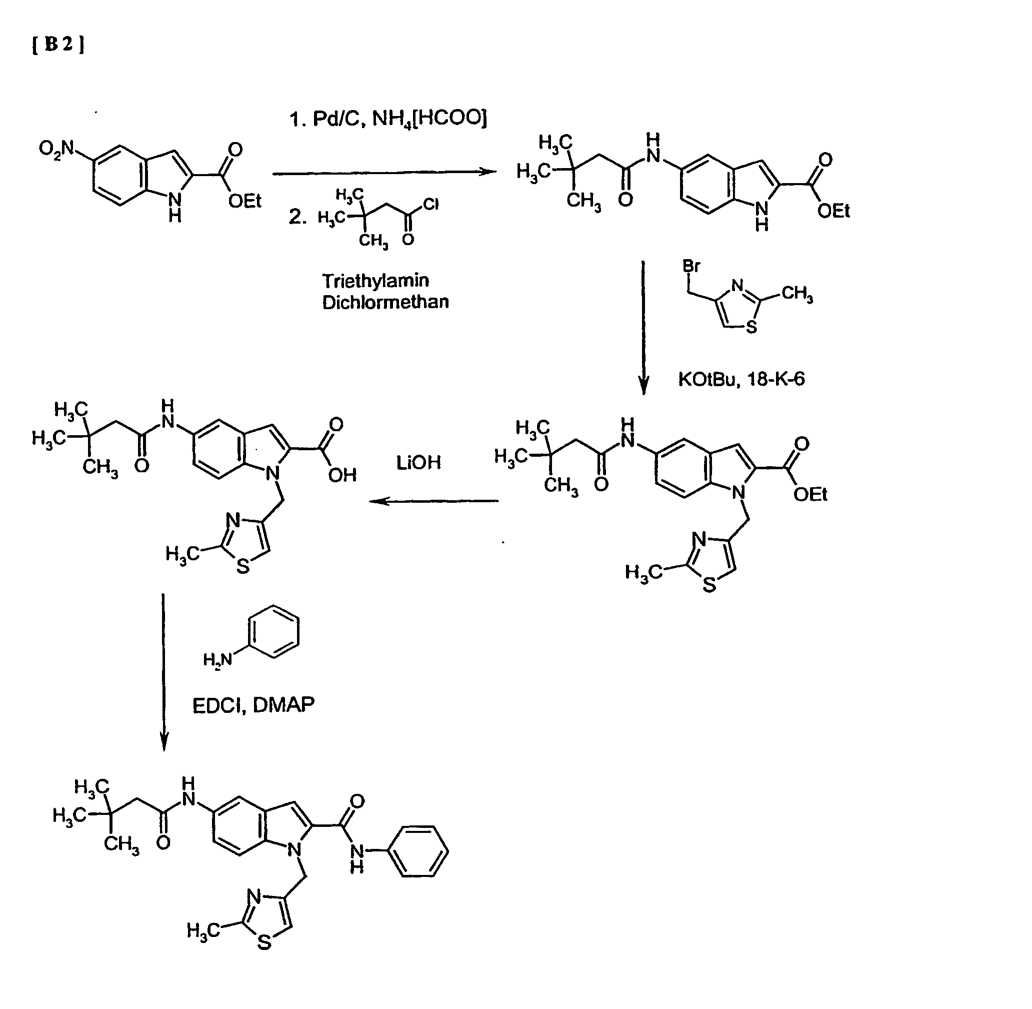

Überraschenderweise zeigen die Verbindungen der Formel (I) ein nicht vorhersehbares, wertvolles pharmakologisches Wirkspektrum und sind daher insbesondere zur Prophylaxe und/oder Behandlung von Erkrankungen bei Menschen und Tieren geeignet.

Die pharmazeutische Wirksamkeit der Verbindungen der Formel (I) lässt sich durch ihre Wirkung als ECE-Inhibitoren erklären.

Die Verbindungen der Formel (I) können aufgrund ihrer pharmakologischen Eigenschaften allein oder in Kombination mit einem oder mehreren anderen Wirkstoffen eingesetzt werden zur Prophylaxe und/oder Behandlung von Erkrankungen in der Human- und Tiermedizin, insbesondere von kardiovaskulären Erkrankungen.

Die Verbindungen der Formel (I) sind geeignet für die Prophylaxe und/oder Behandlung von essentieller, pulmonaler und maligner Hypertonie, von fortgeschrittener Atherosklerose, Herzinfarkt, Herzinsuffizienz, Herz- und Nierenversagen, von ischämischen Erkrankungen wie Angina pectoris, Herzinfarkt und Schlaganfall und von kardialer Arrythmie und renaler Dysfunktion.

Die vorliegende Erfindung betrifft auch die Verwendung der Verbindungen der Formel (I) zur Herstellung von Arzneimitteln zur Prophylaxe und/oder Behandlung der zuvor genannten Krankheitsbilder.

Die vorliegende Erfindung betrifft weiterhin ein Verfahren zur Prophylaxe und/oder Behandlung der zuvor genannten Krankheitsbilder mit den Verbindungen der Formel (I).

Weiterer Gegenstand der vorliegenden Erfindung sind Arzneimittel, die mindestens eine Verbindung der Formel (I), vorzugsweise zusammen mit einem oder mehreren pharmakologisch unbedenklichen Hilfs- oder Trägerstoffen enthalten, sowie deren Verwendung zu den zuvor genannten Zwecken.

Der Wirkstoff kann systemisch und/oder lokal wirken. Zu diesem Zweck kann er auf geeignete Weise appliziert werden, wie z.B. oral, parenteral, pulmonal, nasal, sublingual, lingual, buccal, rectal, transdermal, conjunctival, otisch, als Stents oder als Implantat.

Für diese Applikationswege kann der Wirkstoff in geeigneten Applikationsformen verabreicht werden, wobei die orale Applikation bevorzugt ist.

Für die orale Applikation eignen sich bekannte, den Wirkstoff schnell und/oder modifiziert abgebende Applikationsformen, wie z.B. Tabletten (nichtüberzogene sowie überzogene Tabletten, z.B. mit magensaftresistenten Überzüge versehene Tabletten oder Filmtabletten), Kapseln, Dragees, Granulate, Pellets, Pulver, Emulsionen, Suspensionen und Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen/-lösungen, Sprays; lingual, sublingual oder buccal zu applizierende Tabletten oder Kapseln, Suppositorien, Ohren- und Augen-präparationen, Vaginalkapseln, wässrige Suspensionen (Lotionen, Schüttelmixturen), lipophile Suspensionen, Salben, Cremes, Milch, Pasten, Streupuder oder Implantate.

Die Wirkstoffe können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Im allgemeinen hat es sich als vorteilhaft erwiesen, Mengen von etwa 0,001 bis 50 mg/kg, vorzugsweise etwa 1 bis 50 mg/kg Körpergewicht, bei oraler Anwendung etwa 0,01 bis 25 mg/kg, vorzugsweise etwa 0,5 bis 5 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die vorliegende Erfindung wird an den folgenden, nicht einschränkenden bevorzugten Beispielen veranschaulicht, die die Erfindung jedoch keinesfalls beschränken.

Die Prozentangaben der nachfolgenden Beispiele beziehen sich, sofern nicht anders angegeben, jeweils auf das Gewicht; Teile sind Gewichtsteile.

### A Beiwertung der physiologischen Wirksamkeit

Zur Untersuchung der *in vitro*-Wirkung der erfindungsgemäßen Verbindungen können folgende biologische Assays verwendet werden:

### Funktioneller in vitro-Assay

Die ECE-Aktivität für die Identifizierung der hier beschriebenen Substanzen stammt aus der endothelialen Zellline EA.hy926. Die ECE inhibitorische Wirkung der Verbindungen in dieser Erfindung wird wie folgt beschrieben durchgeführt:
EA.hy296-Zellen werden für 12 - 48 h in einer 384-er Zellkulturschale in 80 µl Zellkulturmedium (DMEM ergänzt mit 10 % FCS, 2mM Glutamine, 10 mM HEPES, 1 mM Natriumpyruvate and 1x HAT (Gibco 21060-017)) in einer Feuchtatmosphäre (100 % Luftfeuchtigkeit) angereichert mit 7 % v/v CO₂ bei 37°C kultiviert. Nach Erreichen der Konfluenz und unmittelbar vor dem eigentlichen Messbeginn wird der Zellkulturüberstand abpipettiert und durch 40 oder 80 µl desselben Mediums ersetzt, dem 1-100 nM bET zugesetzt sind. Nach 30 - 120 Minuten unter sonst identischen Zellkulturbedingungen wird der Überstand abpipettiert. Zelluläre Bestandteile werden durch Zentrifugation in einer handelsüblichen Tischzentrifuge entfernt (10000 RPB; 2 Minuten). Der entstehende, klare Überstand wird entweder direkt wie unten beschrieben verwendet oder durch Schockfrieren in Trockeneis und anschließender Lagerung bei -20°C aufbewahrt. Direkt abgenommener Überstand oder aufgetauter, gelagerter Überstand werden in einem Enzymimmunoassay (EIA) gemessen.

Um die inhibitorische Aktivität von ECE-Inhibitoren zu bestimmen, werden EA.hy296-Zellen mit der Testsubstanz in einer Konzentration zwischen 0,001-5 µM unter den oben beschriebenen Bedingungen inkubiert. Um einen möglichen Einfluss durch die neutrale Endopeptidase (NEP24.11) zu minimieren, werden 100 µM Thiorphan während der bET Inkubation der EA.hy926-Zellen eingesetzt.

Der Anteil des durch ECE-Spaltung entstehenden ET-1 wird wie folgt gemessen: in Abhängigkeit von der Menge an umgesetztem bET werden die Proben vor der Verwendung im EIA 2 - 100-fach verdünnt. Die entsprechende Verdünnung des Zellüberstandes wird in 100µl Portionen für 14-18 Stunden in den Probenröhrchen des EIA-Kits, Biomedica B1-20052, inkubiert.

Die experimentelle Daten sind in der folgenden Tabelle zusammengestellt.

| **Beispiel-Nr.** | **IC**_{**50**} **(µM)** |
|---|---|
| 7 | 1 |
| 57 | 1,5 |
| 58 | 1,6 |
| 60 | 0,7 |
| 62 | 0,7 |

### Big hET-1 Pressor Response an narkotisierten Ratten

Männliche Wistar-Ratten mit einem Körpergewicht von 300-350 g werden mit 100 mg/kg i.p. Thiopental anästhesiert. Nach einer Tracheotomie wird in die Femoralarterie ein Katheter zur Blutdruck- und Herzfrequenzmessung und in die Femoralvene ein Katheter zur Substanzgabe eingeführt. Die Tiere werden mit Raumluft beatmet und ihre Körpertemperatur wird kontrolliert. Eine Ganglienblockade wird durch eine intravenöse Applikation von 5 mg/kg Pentolinium in einem Volumen von 1 ml/kg eingeleitet. Nach 2 Minuten wird die Prüfsubstanz in einer Lösung von Transcutol/Cremophor EL/PBS 0.9 % (10/10/80 = g/g/g) in einem Volumen von 1 ml/kg intravenös verabreicht. Die big hET-1 Gabe erfolgt mit 9 µg/kg als intravenöser Bolus in einem Volumen von 1ml/kg 1 Minute nach der Substanzgabe. Die hämodynamischen Parameter werden über 30 Minuten verfolgt.

### B. Beispiele

### Abkürzungen:

- aq.: wässrig
- ca.: circa
- CDCl₃: Chloroform
- CH: Cyclohexan
- DCI: direkte chemische Ionisation (bei MS)
- DCM: Dichlormethan
- DMAP: 4- Dimethylaminopyridin
- DMF: Dimethylformamid
- DMSO: Dimethylsulfoxid
- d. Th.: der Theorie
- EDC: N-(3-Dimethylaminoisopropyl)-N'-ethylcarbodiimid-Hydrochlorid
- EE: Ethylacetat (Essigsäureethylester)
- EI: Elektronenstoß-Ionisation (bei MS)
- eq: Äquivalent(e)
- ESI: Elek-trospray-Ionisation (bei MS)
- Fp.: Schmelzpunkt
- ges.: gesättigt
- h: Stunde
- HATU: O-(7-Azabenzotriazol-1-yl)-N,N,N',N'-tetramethyl-uroniumhexafluorophosphat
- HPLC: Hochdruck-, Hochleistungsflüssigchromatographie
- LC-MS: Flüssigchromatographie-gekoppelte Massenspektroskopie
- Lit.: Literatur(stelle)
- Lsg.: Lösung
- MG: Molekulargewicht
- ml: Milliliter
- MS: Massenspektroskopie
- NMR: Kernresonanzspektroskopie
- o: ortho
- p: para
- p.A.: pro analysi
- präp.: präparativ
- RF: Rückfluss
- RP: reverse phase (bei HPLC)
- RT: Raumtemperatur
- R,: Retentionszeit (bei HPLC)
- THF: Tetrahydrofuran
- verd.: verdünnt
- vgl.: vergleiche
- Vol.: Volumen
- wässr.: wässrig
- Zers.: Zersetzung

### LC/MS und HPLC-Methoden:

### MHZ2Q = Methode 4

| | | |
|---|---|---|
| Gerätetyp MS | Micromass Quattro LCZ | |
| | Ionisierung | ESI positiv /negativ |
| Gerätetyp HPLC | HP 1100 | |
| | UV-Detektor DAD: | 208-400 nm |
| | Ofentemp. | 40°C |
| Säule | Symmetry C 18 | |
| | 50 mm x 2.1 mm 3 .5 µm | |

| | | | | |
|---|---|---|---|---|
| Gradient | Zeit (min) | A:% | B:% | Fluss (ml/min) |
| | 0.00 | 10.0 | 90.0 | 0.50 |
| | 4.00 | 90.0 | 10.0 | 0.50 |
| | 6.00 | 90.0 | 10.0 | 0.50 |
| | 6.10 | 10.0 | 90.0 | 1.00 |
| | 7.50 | 10.0 | 90.0 | 0.50 |
| A | Acetonitrile + 0.1% Ameisensäure | | | |
| B | Wasser + 0.1 % Ameisensäure | | | |

### Methode 1 (LCMS) = Methode MHZ2P01

Instrument: Micromass Platform LCZ, HP1100; Säule: Symmetry C18, 50 mm x 2.1 mm, 3.5 µm; Eluent A: Wasser + 0.05 % Ameisensäure, Eluent B: Acetonitril + 0.05 % Ameisensäure; Gradient: 0.0min 90 % A → 4.0 min 10 % A → 6.0 min 10 % A; Ofen: 40°C; Fluss: 0.5 ml/min; UV-Detektion: 208-400 nm.

### Methode 2 (LCMS) = Methode SMKL-ZQ-2

Instrument: Waters Alliance 2790 LC; Säule: Symmetry C18, 50 mm x 2.1, 3.5 µm; Eluent A: Wasser + 0.1 % Ameisensäure, Eluent B: Acetonitril + 0.1 % Ameisensäure; Gradient: 0.0 min 5 % B → 5.0 min 10 % B → 6.0 min 10 % B; Temperatur: 50°C; Fluss: 1.0 ml/min; UV-Detektion: 210 nm.

### Methode 3 (LCMS) = Methode SMKL_03042001-sauer-210

Instrument: Finnigan MAT 900S, TSP: P4000,AS3000,UV3000HR; Säule: Symmetry C 18, 150 mm x 2.1 mm, 5.0 µm; Eluent C: Wasser, Eluent B: Wasser + 0.3g 35 %ige HCl, Eluent A: Acetonitril; Gradient: 0.0 min 2 % A → 2.5 min 95 % A → 5 min 95 % A; Ofen: 70°C; Fluss: 1.2 ml/min; UV-Detektion: 210 nm.

### Methode 4 (LCMS) = Methode MHZ2Q

### Methode 5 (LCMS) = Methode SMKL-ZQ-5-CS

Gerätetyp MS: Micromass ZQ; Gerätetyp HPLC: Waters Alliance 2790; Säule: Symmetry C 18, 50 mm x 2.1 mm, 3.5 µm; Eluent B: Acetonitril + 0.05 % Ameisensäure, Eluent A: Wasser + 0.05 % Ameisensäure; Gradient: 0.0 min 10 % B → 3.5 min 90 % B → 5.5 min 90 % B; Ofen: 50°C; Fluss: 0.8 ml/min; UV-Detektion: 210 nm.

### Methode 6 (HPLC) = Methode SYA-HPPSK2

Instrument: HP 1100 mit DAD-Detektion; Säule: Kromasil RP-18, 60 mm x 2 mm, 3.5 µm; Eluent: A = 5 ml HClO₄/l H₂O, B = ACN; Gradient: 0 min 2 % B, 0.5 min 2 % B, 4.5 min 90 % B, 6.5 min 90 % B; Fluss: 0.75 ml/min; Temp.:30°C; Detektion UV 210 nm.

### Methode 7 (HPLC) = Methode SMKL-N1-1-Low Vol ACN-HCL-210.met

Instrument: 1 Säule: Symmetry C1 8 2.1 x 150 mm; Eluent: A = ACN, B= 0.6 g 30 %ige HCl/Wasser; Gradient: 0 min 10 % A Fluss 0.60 ml/min, 4 min 90 % A Fluss 0.60 ml/min, 9 min 90 % A Fluss 0.80 ml/min; Temp.:50°C; Detektion UV 210 nm.

### Ausgangsverbindungen

### Beispiel I

### 5-Nitro-1-propyl-1H-indol-2-carbonsäureethylester

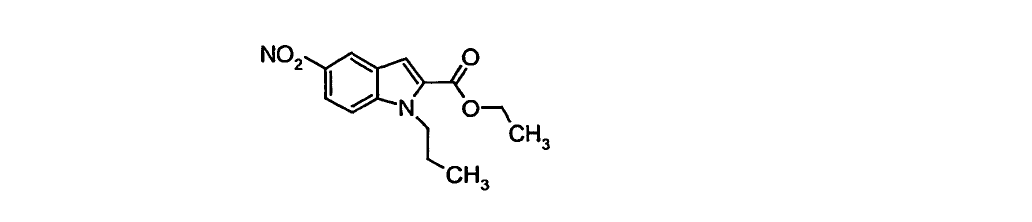

937 mg (4.00 mmol) 5-Nitro-1H-indol-2-carbonsäureethylester (A. Guy, J.-P. Guetté, *Synthesis* **1980**, 222-223) werden unter Argon in 12 ml Dimethylsulfoxid vorgelegt. 4.40 mmol Natriumhydrid (176 mg 60 %ige Dispersion in Paraffin) werden portionsweise zugegeben und der Ansatz 30 min bei 50°C gerührt. Nach Abkühlen auf RT werden 170 mg (4.40 mmol) Propyliodid zugegeben und der Ansatz für 3 h bei RT nachgerührt. Das Reaktionsgemisch wird auf 30 ml Wasser gegeben und mit Essigsäureethylester (6 x 30 mL) ausgeschüttelt. Die vereinigten organischen Phasen werden mit 50 ml ges. Natriumchlorid-Lsg. gewaschen, über Natriumsulfat getrocknet und am Rotationsverdampfer vom Lösemittel befreit. Das erhaltene, braune Rohprodukt wird säulenchromatographisch gereinigt (Kieselgel 60, Laufmittelgradient Cyclohexan → Cyclohexan-Essigsäureethylester 3:1), wobei das Produkt als zweite Fraktion erhalten wird.
Ausbeute: 958 mg (3.48 mmol, 77 % d. Th.).
MS (DCI]: m/z = 294 (M+NH₄)⁺.
¹H-NMR (500 MHz, DMSO-d₆): δ = 8.76 (d, 1H), 8.17 (dd, 1H), 7.89 (d, 1H), 7.58 (s, 1H), 4.60 (dd, 2H), 4.36 (q, 2H), 1.74 (sextett, 2H), 1.35 (t, 3H), 0.84 (t, 3H).

### Beispiel II

### 1-(2-Fluorbenzyl)-5-nitro-1H-indol-2-carbonsäureethylester

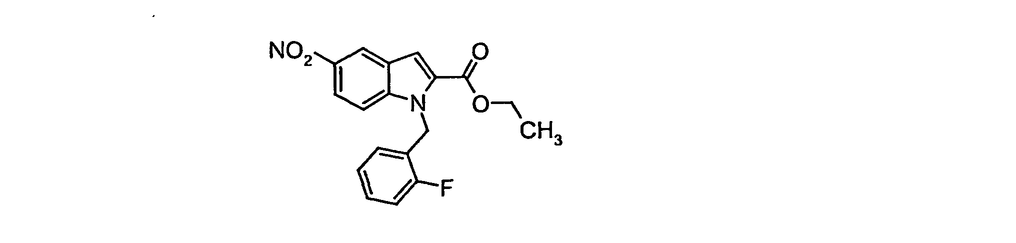

Die Darstellung erfolgt wie für Beispiel 1 beschrieben aus 940 mg (4.00 mmol) 5-Nitro-1H-indol-2-carbonsäureethylester und 780 mg (4.15 mmol) 2-Fluorbenzylbromid, Reaktionszeit 6 h.
Ausbeute: 980 mg (72 % d. Th.).
MS (DCI): m/z = 360 (M+NH₄)⁺.
¹H-NMR (300 MHz, DMSO-d₆): δ = 8.80 (d, 1H), 8.17 (dd, 1H), 7.82 (d, 1H), 7.68 (d, 1H), 7.35-7.18 (m, 2H), 7.03 (dt, 1H), 6.56 (dt, 1H), 5.98 (s, 2H), 4.29 (q, 2H), 1.27 (t, 3H).

### Beispiel III

### 5-Nitro-1-propyl-1H-indol-2-carbonsäure

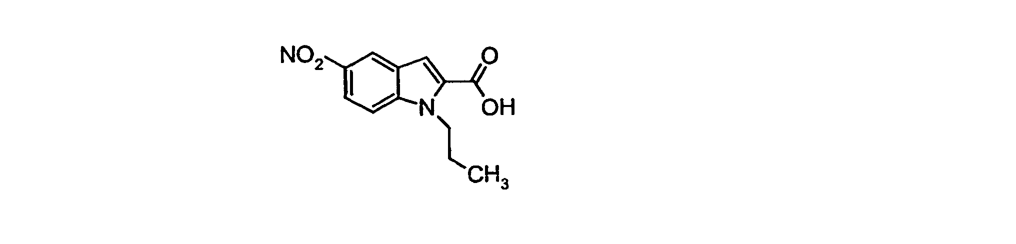

236 mg (3.68 mmol, 85 %ig) Kaliumhydroxid (Pulver) werden in 10 ml Dimethylsulfoxid vorgelegt, 961 mg (3.48 mmol) der Verbindung aus Beispiel I zugegeben und der Ansatz eine halbe Stunde bei RT gerührt. Die Reaktionsmischung wird auf ca. 100 ml Wasser gegeben und die Lösung unter Kühlung portionsweise mit 10 %iger Salzsäure versetzt, bis sich kein Niederschlag mehr bildet. Der ausgefallene Feststoff wird abgesaugt und über Nacht im Exsikkator unter Vakuum getrocknet.
Ausbeute: 812 mg (94 % d. Th.)
Fp.: 197°C
¹H-NMR (300 MHz, DMSO-d₆): δ = 13.33 (br. s, 1H), 8.73 (d, 1H), 8.15 (dd, 1H), 7.85 (d, 1H), 7.53 (s, 1H), 4.61 (t, 2H), 1.74 (sextett, 2H), 0.83 (t, 3H).

### Beispiel IV

### 1-(2,6-Difluorbenzyl)-5-nitro-1H-indol-2-carbonsäure

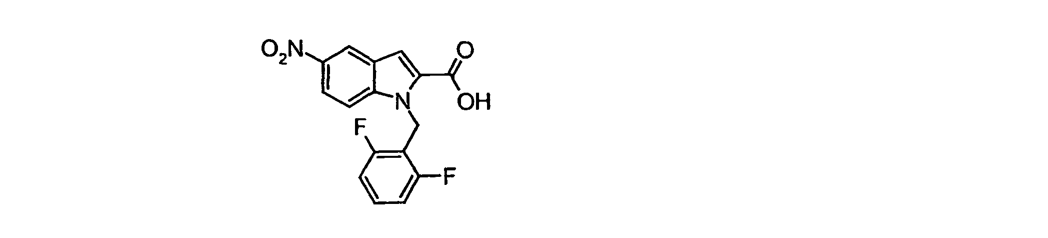

Unter Argonatmosphäre werden 5.49 g (83.2 mmol, 85 %ig) Kaliumhydroxid (Pulver) in 110 ml Dimethylsulfoxid vorgelegt, bei RT 6.43 g (27.5 mmol) 5-Nitro-1H-indol-2-carbonsäureethyleser (A. Guy, J.-P. Guetté, *Synthesis* **1980**, 222-223) zugegeben und der Ansatz für 30 min gerührt. Unter Eiskühlung wird anschließend bei 5-10°C Innentemperatur 2,6-Difluorbenzylchlorid (10.0 g, 61.5 mmol) innerhalb von 15 min zugetropft und der Ansatz 16 h bei RT nachgerührt. Zur Aufarbeitung wird auf 500 ml Wasser gegeben, mit verd. Salzsäure sauer gestellt, der ausgefallene Feststoff abgesaugt, und über Kieselgel 60 (Laufmittelgradient Dichlormethan → Dichlormethan-Methanol 3:1) chromatographisch vorgereinigt. Das erhaltene Produkt wird aus Ethanol umkristallisiert. Man erhält 4.33 g (47 % d. Th.) eines hellgelben, kristallinen Feststoffes.
MS (ESIpos): m/z = 333 (M+H)⁺.
¹H-NMR (300 MHz, DMSO-d₆): δ = 13.41 (br. s, 1H), 8.72 (d, 1H), 8.17 (dd, 1H), 7.71 (d, 1H), 7.54 (s, 1H), 7.37 (m, 1H), 7.05 (t und m, 2H), 6.07 (s, 2H).

### Beispiel V

### 1-(2-Fluorbenzyl)-5-nitro-1H-indol-2-carbonsäure

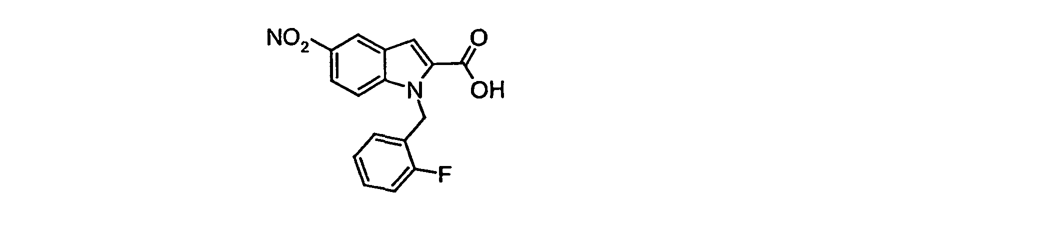

Darstellung analog Beispiel III aus 877 mg (2.56 mmol) der Verbindung aus Beispiel II. Ausbeute: 732 mg (91 % d. Th.)
Fp.: 223°C
MS (DCI): m/z = 332 (M+NH₄)⁺.
¹H-NMR (500 MHz, DMSO-d₆): δ = 13.49 (br. s, 1H), 8.79 (d, 1H), 8.15 (dd, 1H), 7.78 (d, 1H), 7.61 (s, 1H), 7.29 (m, 1H), 7.23 (m, 1H), 7.03 (t, 1H), 6.52 (t, 1H), 6.02 (s, 2H).

### Beispiel VI

### 1-(2,6-Difluorbenzyl)-N-(3-methylphenyl)-5-nitro-1H-indol-2-carbonsäureamid

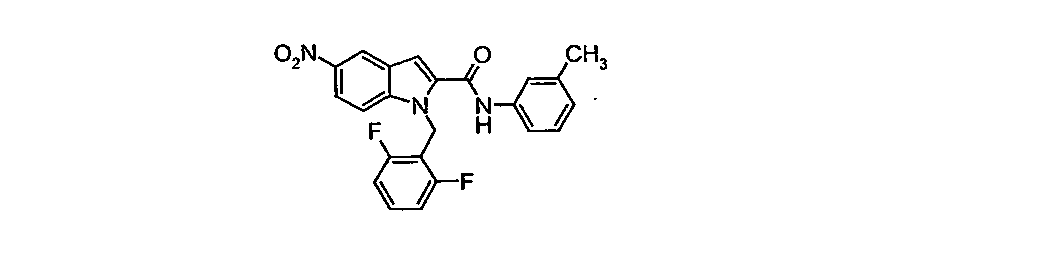

Die Verbindung aus Beispiel IV (1.40 g, 4.21 mmol) wird portionsweise in 10 ml Thionylchlorid eingetragen und der Ansatz nach beendeter Zugabe in der Siedehitze gerührt. Nach 60 min wird eingeengt, der Rückstand jeweils 3 x mit ca. 50 ml Toluol versetzt und erneut eingeengt. Das erhaltene Indolcarbonsäurechlorid wird in 50 ml Dichlormethan aufgenommen und bei 0°C mit 2.94 ml (21.1 mmol) Triethylamin und dann mit 587 mg (5.48 mmol) 3-Methylanilin versetzt. Der Ansatz wird 16 h bei Raumtemperatur gerührt. Die Reaktionslösung wird auf 200 ml Wasser gegeben, das organische Lösemittel aus dem Gemisch am Rotationsverdampfer entfernt, und der ausgefallene Feststoff abgesaugt und getrocknet. Man erhält 1.48 g (76 % d. Th.) Produkt.
MS (DCI): m/z = 439 (M+NH₄)⁺.
¹H-NMR (300 MHz, DMSO-d₆): δ = 10.53 (s, 1H), 8.74 (d, 1H), 8.17 (dd, 1H), 7.75 (d, 1H), 7.60-7.47 (m, 3H), 7.36 (m, 1H), 7.24 (t, 1H), 7.05 (t, 2H); 6.95 (d, 1H), 6.05 (s, 2H), 2.32 (s, 3H).

### Beispiel VII

### 1-(2-Fluorbenzyl)-5-nitro-N-phenyl-1H-indol-2-carbonsäureamid

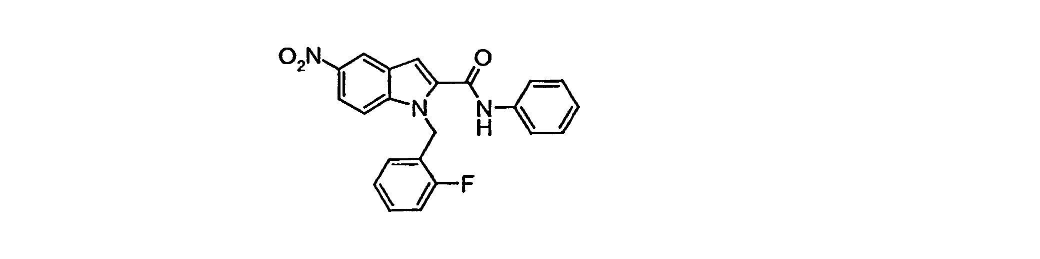

Umsetzung von 500 mg (1.59 mmol) der Verbindung aus Beispiel V und 163 mg (1.75 mmol) Anilin wie für Beispiel VI beschrieben. Die Reaktionszeit beträgt ca. 30 min. Zur Aufarbeitung wird die Reaktionsmischung auf 100 ml Wasser gegeben, mit Dichlormethan (4 x 50 ml) ausgeschüttelt, die vereinigten organischen Phasen über Natriumsulfat getrocknet und eingeengt. Ausbeute: 610 mg (98 % d. Th.). Eine Probe des erhaltenen Produktes wird zur Charakterisierung aus Ethanol umkristallisiert, die Hauptmenge direkt weiterverwendet.
MS (DCI): m/z = 407 (M+NH₄)⁺.
¹H-NMR (400 MHz, DMSO-d₆): δ = 10.62 (s, 1H), 8.81 (d, 1H), 8.16 (dd, 1H), 7.81 (d, 1H), 7.72 (d, 2H), 7.65 (s, 1H), 7.36 (t, 2H), 7.32-7.16 (m, 2H), 7.12 (t, 1H), 7.04 (dt, 1H), 6.75 (dt, 1H), 6.00 (s, 2H).

### Beispiel VIII

### 1-(2,6-Difluorbenzyl)-5-nitro-N-phenyl-1H-indol-2-carbonsäureamid

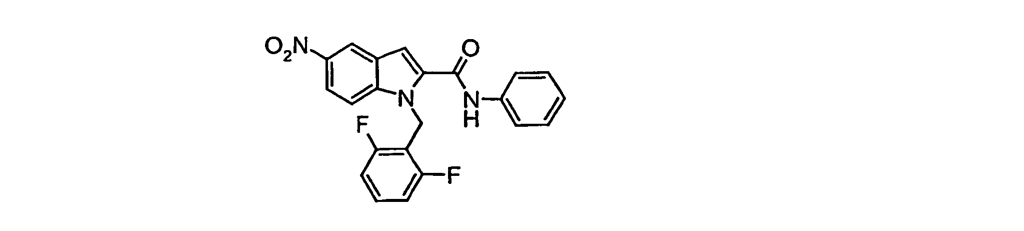

Darstellung aus den entsprechenden Edukten wie für Beispiel VI beschrieben.
MS (ES1pos): m/z = 408 (M+H)⁺.

### Beispiel IX

### 1-(2-Fluorbenzyl)-5-nitro-N-(3-pyridinyl)-1H-indol-2-carbonsäureamid

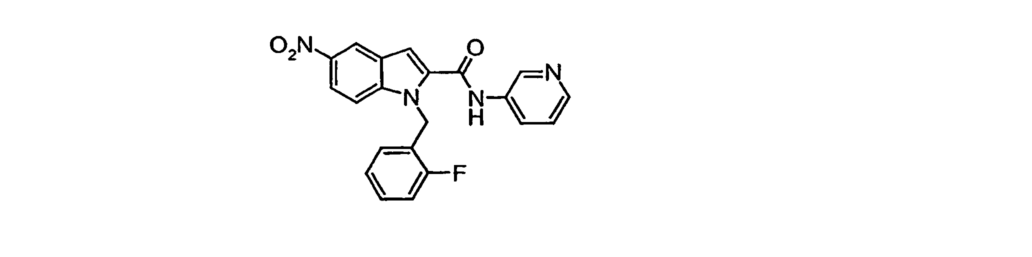

Darstellung aus den entsprechenden Edukten wie für Beispiel VII beschrieben. Das nach Aufarbeitung erhaltene Produkt wird in Diethylether suspendiert, abgesaugt und getrocknet.
Fp.: 234°C (Zers.)
MS (ESIpos): m/z = 391 (M+H)⁺.

### Beispiel X

### 1-(2)-Fluorbenzyl)-N-(4-methoxyphenyl)-5-nitro-1H-indol-2-carbonsäureamid

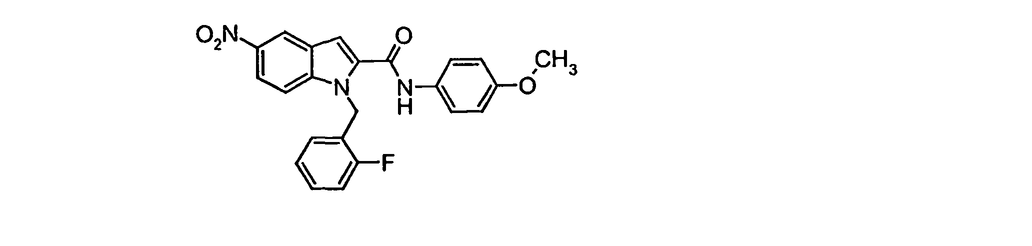

Darstellung aus den entsprechenden Edukten wie für Beispiel VII beschrieben. Das nach Aufarbeitung erhaltene Produkt wird in Diethylether suspendiert, abgesaugt und getrocknet.
Fp.: 233°C
MS (ESIpos): m/z = 420 (M+H)⁺.

### Beispiel XI

### 1-(2-Fluorbenzyl)-N-(3-methoxyphenyl)-5-nitro-1H-indol-2-carbonsäureamid

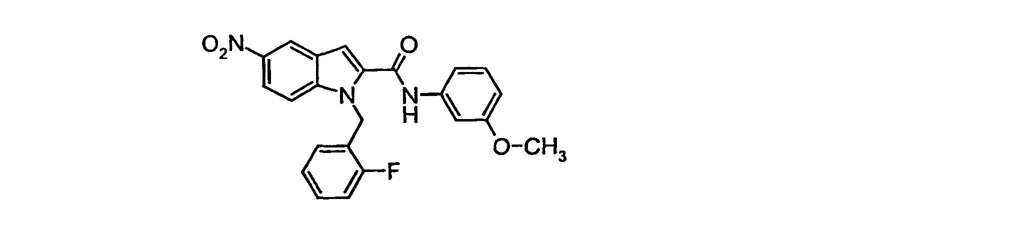

Darstellung aus den entsprechenden Edukten wie für Beispiel VII beschrieben. Das aus der Reaktionslösung ausgefallene Produkt wird in Diethylether suspendiert, abgesaugt und getrocknet.
Fp.: 203°C
MS (ESIpos): m/z = 420 (M+H)⁺.

### Beispiel XII

### 1-(2-Fluorbenzyl)-N-(3-methylphenyl)-5-nitro-1H-indol-2-carbonsäureamid

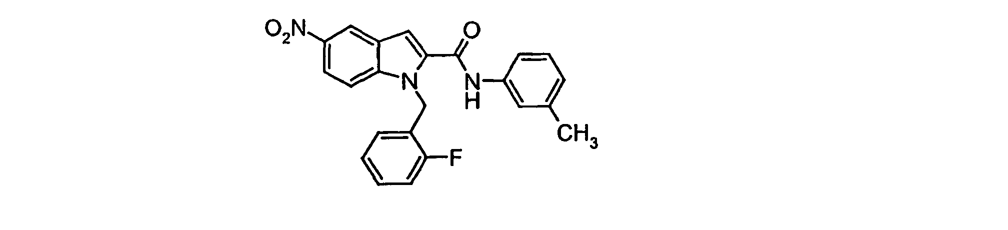

Darstellung aus den entsprechenden Edukten wie für Beispiel VII beschrieben. Das aus der Reaktionslösung ausgefallene Produkt wird in Diethylether suspendiert, abgesaugt und getrocknet.
Fp.: 211°C
MS (ESIpos): m/z = 404 (M+H)⁺.

### Beispiel XIII

### 5-Nitro-N-phenyl-1-propyl-1H-indol-2-carbonsäureamid

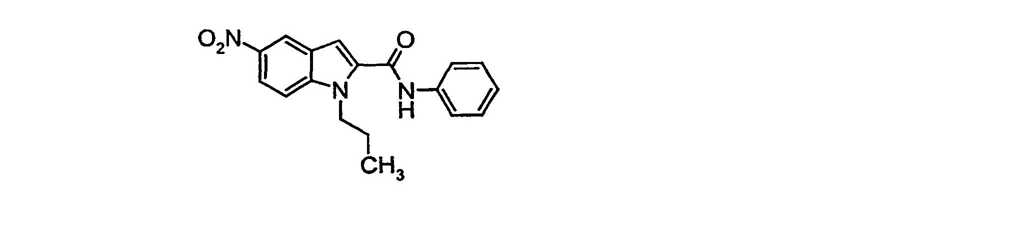

Darstellung aus den entsprechenden Edukten wie für Beispiel VII beschrieben. Das nach Aufarbeitung erhaltene Produkt wird ohne weitere Reinigung weiter umgesetzt.
Fp.: 201-205°C
MS (ESIpos): m/z = 324 (M+H)⁺.

### Beispiel XIV

### 5-Amino-1-(2,6-difluorbenzyl)-N-(3-methylphenyl)-1H-indol-2-carbonsäureamid

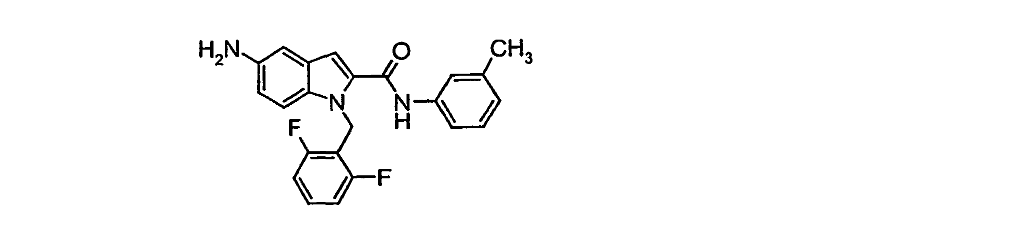

1.38 g (3.28 mmol) der Verbindung aus Beispiel VI werden in 100 ml Ethanol vorgelegt. Anschließend werden 3.70 g (16.4 mmol) Zinn(II)chlorid-Dihydrat zugegeben und der Ansatz 16 h in der Siedehitze gerührt. Die Reaktionslösung wird auf ca. 200 ml Wasser gegeben, mit verd. Natronlauge basisch gestellt und mit Essigsäureethylester (5 x 50 ml) ausgeschüttelt. Die vereinigten org. Phasen werden mit 50 ml ges. Natriumchlorid-Lösung gewaschen, über Natriumsulfat getrocknet und eingeengt. Von dem erhaltenen hellbraunen Produkt (1.19 g, 85 % d. Th.) wird eine Probe zur Charakterisierung über präp. HPLC (GROM-SIL 120 OSD4 HE, 10 µm, Laufmittelgradient Acetonitril-Wasser 30:70 → 95:5) feingereinigt, die Hauptmenge wird direkt weiterverwendet.
MS (ESIpos): m/z = 392 (M+H)⁺.
¹H-NMR (200 MHz, DMSO-d₆): δ = 10.20 (s, 1H), 7.64 (s, 1H), 7.56 (d, 1H), 7.43-7.13 (m, 3H), 7.12-6.85 (m, 4H), 6.73 (s, 1H), 6.65 (d, 1H), 5.89 (s, 2H), 4.70 (s, 2H), 2.31 (s, 3H).

Die in der folgenden Tabelle aufgeführten Verbindungen werden in Analogie zu Beispiel XIV hergestellt.

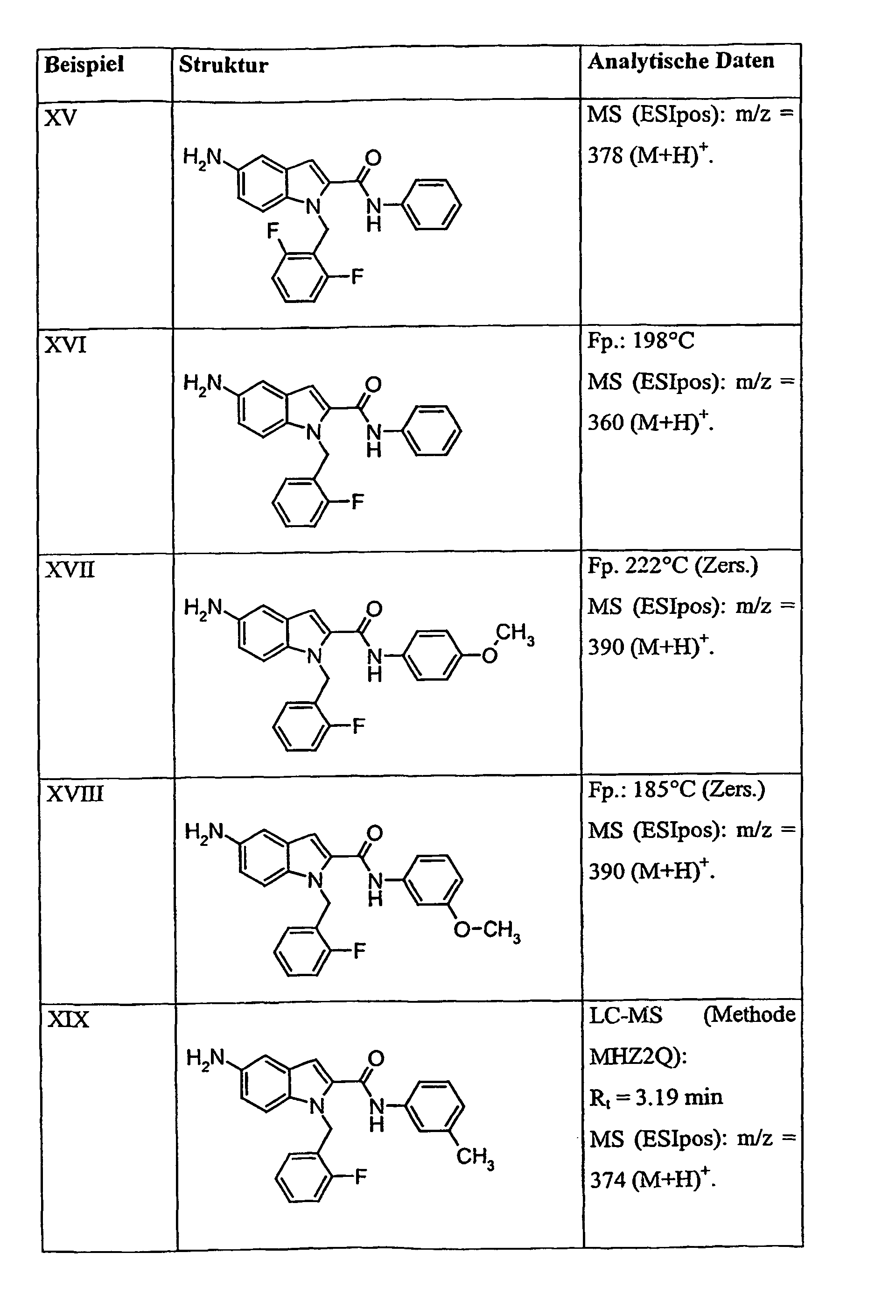

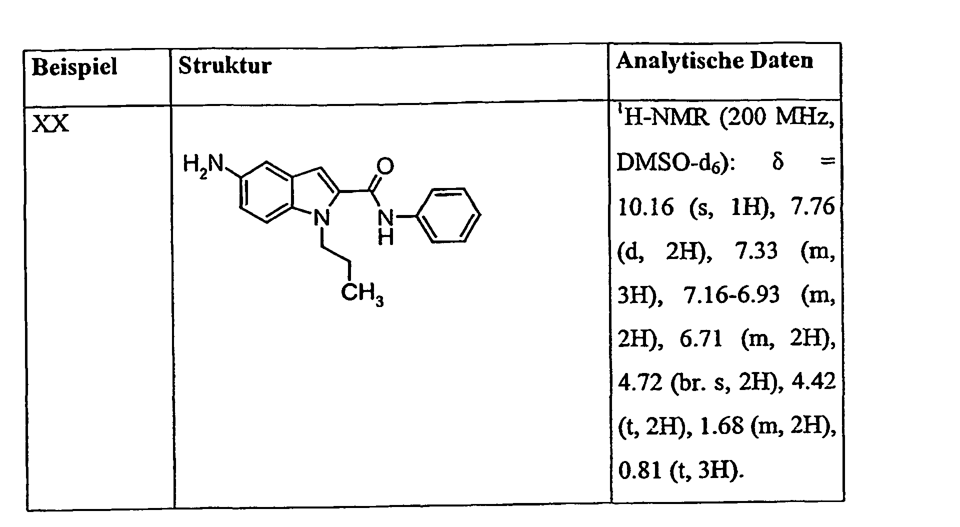

### Beispiel XXI

### 1-(2,4-Difluorbenzyl)-5-nitro-1H-indol-2-carbonsäureethylester

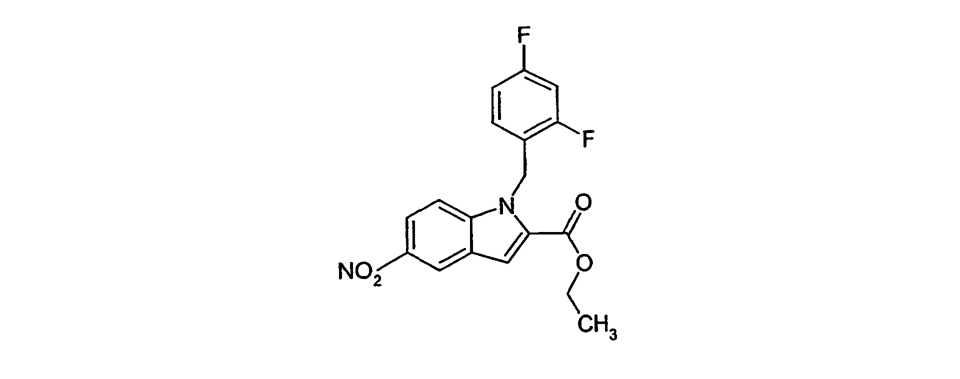

Unter Argon wird 214 mg (0.81 mmol) 1,4,7,10,13,16-Hexaoxacyclooctadecan (18-Krone-6) in 43 ml THF vorgelegt und mit 9.73 ml (9.73 mmol) 1 molarer Kalium-tert.-butylatlösung in THF und 2000 mg (8.11 mmol) 5-Nitro-1H-indol-2-carbonsäureethylester versetzt. Es wird 15 Minuten bei RT nachgerührt und auf 0°C abgekühlt. Dazu wird eine Lösung aus 1713 mg (8.11 mmol) 2,4-Difluorbenzylbromid in 13 ml THF langsam zugetropft. Das Eisbad wird entfernt und 1 Stunde bei RT gerührt. Zur Aufarbeitung wird mit Wasser verdünnt und das THF im Vakuum abrotiert. Der wässrige Rückstand wird mit Ethylacetat extrahiert und die organische Phase wird mit ges. Natriumchloridlösung gewaschen, mit Natriumsulfat getrocknet, filtriert und im Vakuum getrocknet. Der Rückstand wird säulenchromatographisch (Laufmittel: Cyclohexan:Ethylacetat 5:1) gereinigt.
Ausbeute: 888 mg (29 % d. Th.)
LC/MS (Methode 3): Rₜ = 3.07 min
MS (EI): m/z = 361 (M+H)⁺
¹H-NMR (200 MHz, DMSO-d₆): δ = 1.27 (t, 3H), 4.29 (q, 2H), 5.94 (s, 2H), 6.57-6.73 (m, 1H), 6.87-7.01 (m, 1H), 7.21-7.37 (m, 1H), 7.68 (s, 1H), 7.85 (d, 1H), 8.19 (dd, 1H), 8.81 (d, 1H).

### Beispiel XXII

### 1-(2,4-Difluorbenzyl)-5-nitro-1H-indol-2-carbonsäure

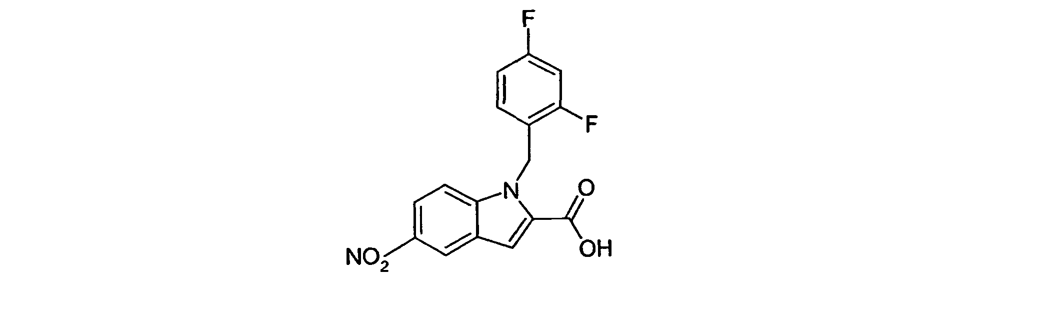

Es wird 880 mg (2.44 mmol) 1-(2,4-Difluorbenzyl)-5-nitro-1H-indol-2-carbonsäureethylester aus Beispiel XXI in 11 ml THF und 11 ml Methanol vorgelegt. Dazu gibt man 194 mg 2.44 ml (4.88 mmol) 2 molare Lithiumhydroxydlösung und erhitzt die Mischung für 30 Minuten auf 90°C. Es wird wieder abgekühlt und mit wässriger Salzsäure und Ethylacetat verdünnt. Die organische Phase wird mit ges. Natriumchloridlösung gewaschen, mit Natriumsulfat getrocknet, filtriert und im Vakuum getrocknet.
Ausbeute: 831 mg (100 % d. Th.)
LC/MS (Methode 4): Rₜ = 4.26 min
MS (EI): m/z = 331 (M-H)⁺
¹H-NMR (400 MHz, DMSO-d₆): δ = 5.97 (s, 2H), 6.64 (dd, 1H), 6.90-6.98 (m, 1H), 7.24-7.32 (m, 1H), 7.59 (s, 1H), 7.78 (d, 1H), 8.15 (dd, 1H), 8.77 (dd, 1H), 13.47 (br. s, 1H).

Die Herstellung der folgenden Verbindung erfolgt analog zu der in Beispiel VI beschriebenen Weise:

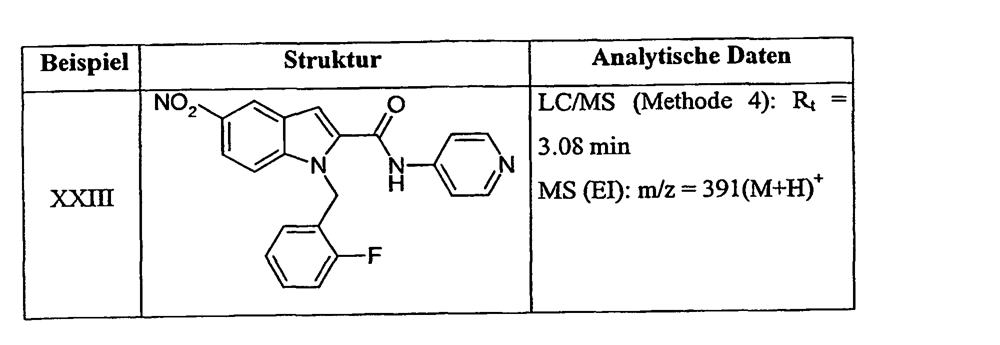

### Beispiel XXIV

### 1-(2,4-Difluorbenzyl)-N-(4-fluorphenyl)-5-nitro-1H-indol-2-carbonsäureamid

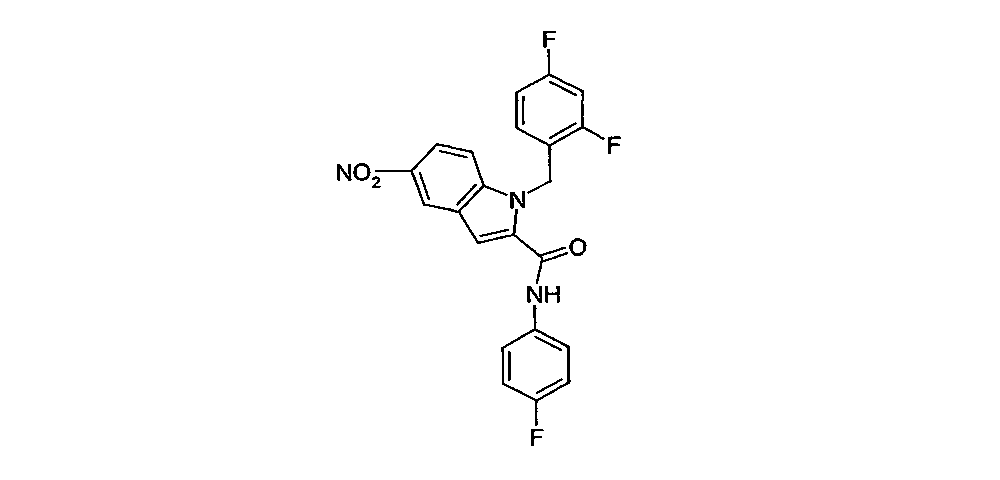

Man legt 389 mg (1.17 mmol) 1-(2,4-Difluorbenzyl)-5-nitro-1H-indol-2-carbonsäure aus Beispiel XXII, 336 mg (1.76 mmol) N'-(3-Dimethylaminopropyl)-N-ethylcarbodiimid x HCl und 71.5 mg (0.59 mmol) 4-Dimethylaminopyridin in 30 ml Dichlormethan:DMF 10:1 Gemisch vor. Dazu wird 156 mg 0.13 ml (1.40 mmol) 4-Fluoranilin zugegeben und 4 Stunden bei RT nachgerührt. Das Gemisch wird zur Aufarbeitung mit wässriger Salzsäure und Ethylacetat verdünnt und extrahiert. Die organische Phase wird mit ges. Natriumchloridlösung gewaschen, mit Natriumsulfat getrocknet, filtriert und im Vakuum getrocknet.
Ausbeute: 485 mg (62 % d. Th.)
LC/MS (Methode 1): Rₜ = 5.00 min
MS (EI): m/z = 424 (M-H)⁺
¹H-NMR (400 MHz, DMSO-d₆): δ = 5.94 (s, 2H), 6.82-6.90 (m, 1H), 6.92-6.99 (m, 1H), 7.12-7.34 (m, 4H), 7.64 (s, 1H), 7.71-7.77 (m, 2H), 7.82 (d, 1H), 8.16 (dd, 1H), 8.80 (d, 1H).

Die Herstellung der folgenden Verbindungen erfolgt analog zu der in Beispiel XXIV beschriebenen Weise:

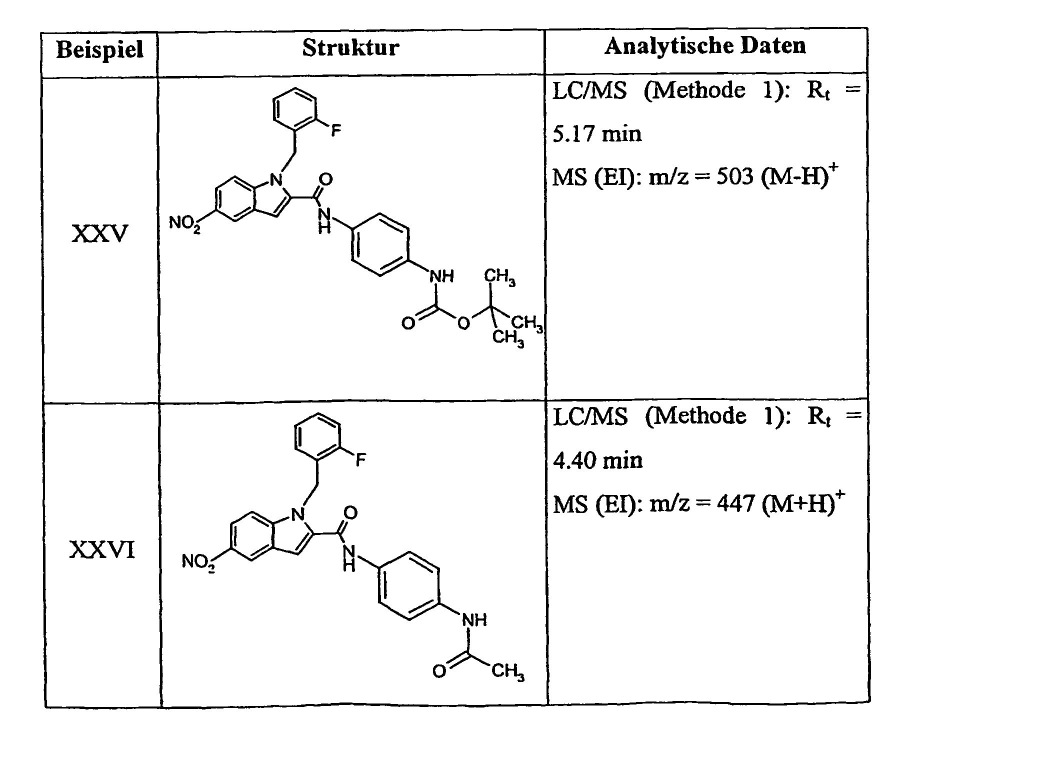

Die Herstellung der folgenden Verbindung erfolgt analog zu der in Beispiel XIV beschriebenen Weise:

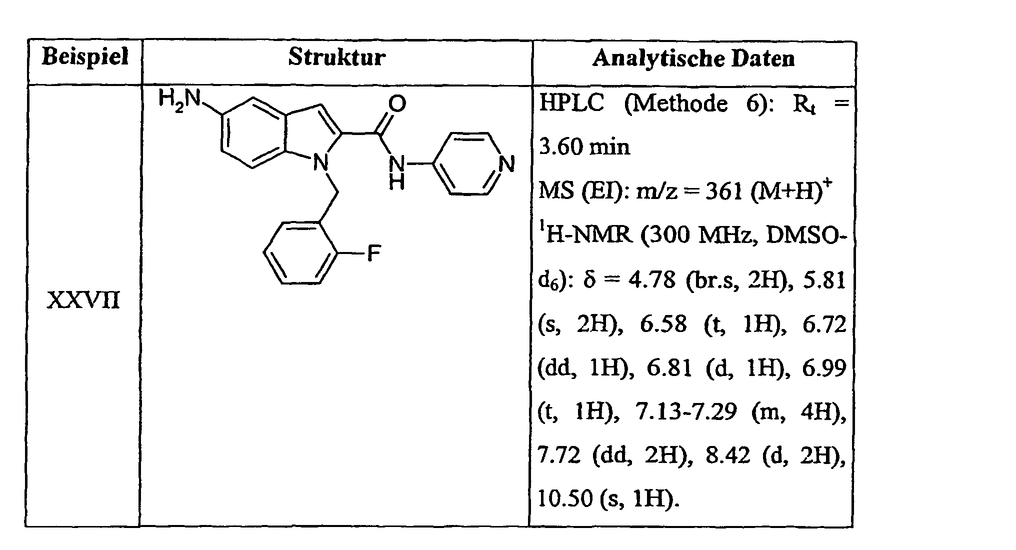

### Beispiel XXVIII

### 5-Amino-1-(2,4-difluorbenzyl)-N-(4-fluorphenyl)-1H-indol-2-carbonsäureamid

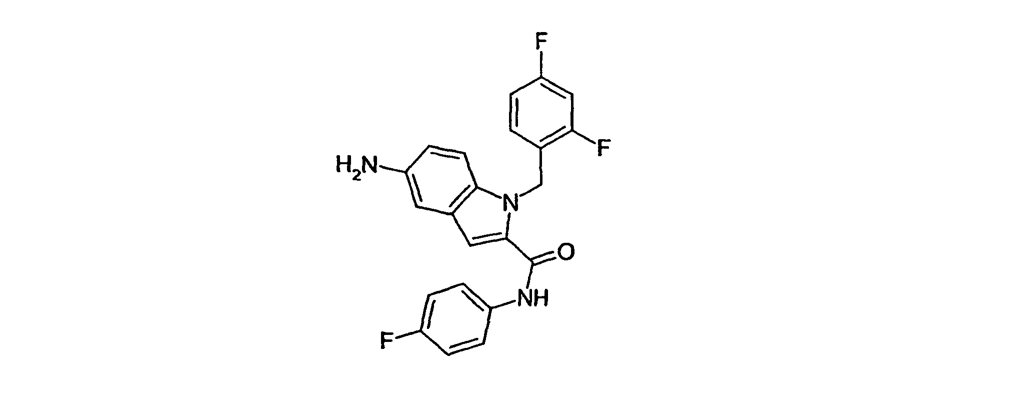

Man legt 485 mg (1.14 mmol) 1-(2,4-Difluorbenzyl)-N-4-fluorphenyl)-5-nitro-1H--indol-2-carbonsäureamid aus Beispiel XXIV in Ethylacetat und Ethanol vor. Dazu wird 287 mg (4.56 mmol) Ammoniumformiat und 49 mg 10 %ige Palladium auf Aktivkohle gegeben. Es wird zum Rückfluss gekocht und bei 50°C entsteht eine Gasentwicklung. Zur Vervollständigung der Reaktion werden die gleichen Mengen an Ammoniumformiat und Palladium zugegeben. Nach weiteren 3 Stunden bei Rückfluss wird abgekühlt und über Kieselgur abfiltriert und mit 500 ml Ethanol nachgewaschen. Das Lösungsmittel wird im Vakuum abgezogen und der Rückstand getrocknet.
Ausbeute: 546 mg (100 % d. Th.)
LC/MS (Methode 1): Rₜ = 3.40 min
MS (EI): m/z = 396 (M+H)⁺
¹H-NMR (200 MHz, DMSO-d₆): δ = 5.76 (s, 2H), 6.57-6.74 (m, 2H), 6.78 (s, 1H), 6.84-6.99 (m, 1H), 7.03-7.35 (m, 5H), 7.66-7.82 (m, 2H), 10.29 (s, 1H) NH2 nicht zu sehen.

Die Herstellung der folgenden Verbindungen erfolgt analog zu der in Beispiel XXVIII beschriebenen Weise:

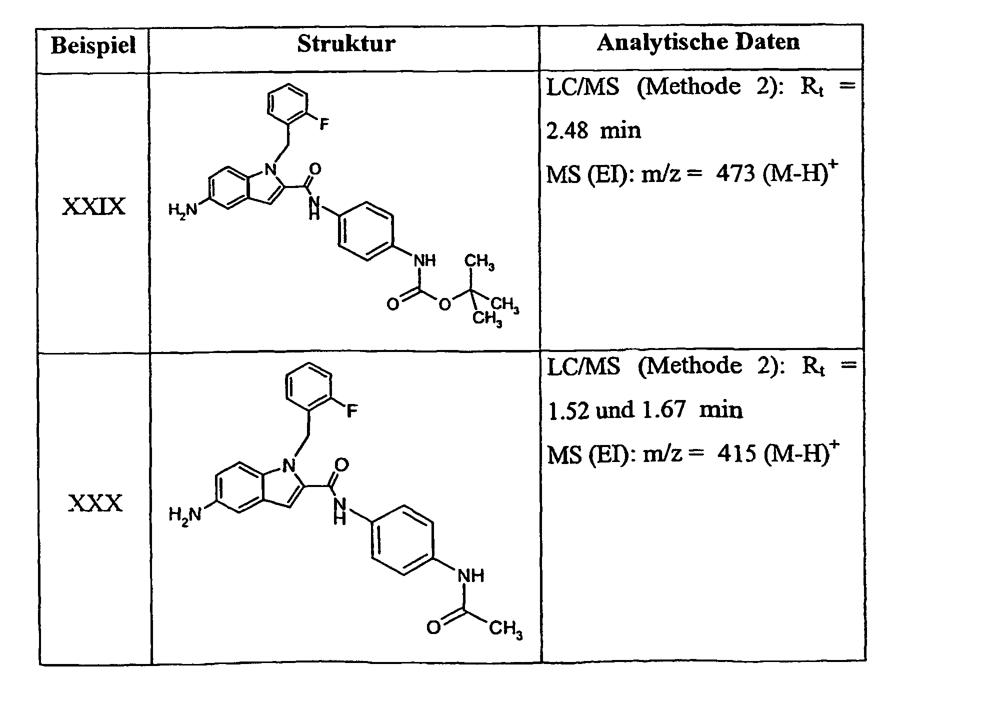

### Beispiel XXXI

### tert-Butyl 4-[({1-(2-fluorbenzyl)-5-[(tetrahydro-2-furanylacetyl)amino]-1H-indol--2-yl}carbonyl)amino]phenylcarbamat

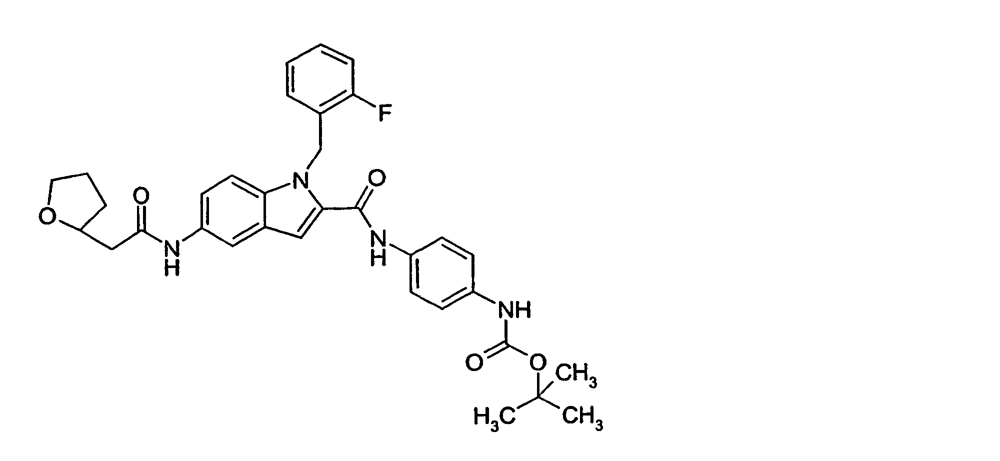

Man gibt 49 mg (0.38 mmol) Tetrahydro-2-furanylessigsäure, 19.3 mg (0.16 mmol) 4- Dimethylaminopyridin und 91 mg (0.47 mmol) N'-(3-Dimethylaminopropyl)-N-ethylcarbodiimid x HCl in 3 ml DMF. Dazu gibt man 150 mg (0.32 mmol) tert-Butyl-4-({[5-amino-1-(2-fluorbenzyl)-1H-indol-2-yl]carbonyl} amino)phenylcarbamat aus Beispiel XXIX. Es wird für 5 Stunden bei RT gerührt. Zur Aufarbeitung wird mit Dichlormethan und wässriger Salzsäure verdünnt und extrahiert. Die organische Phase wird mit ges. Natriumhydrogencarbonatlösung gewaschen, mit Natriumsulfat getrocknet, filtriert und im Vakuum einrotiert. Der Rückstand wird über eine präparative HPLC gereinigt.
Ausbeute: 115 mg (62 % d. Th.)
LC/MS (Methode 2): Rₜ = 3.57 min
MS (EI): m/z = 587 (M+H)⁺

Die Herstellung der folgenden Verbindungen erfolgt analog zu der in Beispiel XXXI beschriebenen Weise:

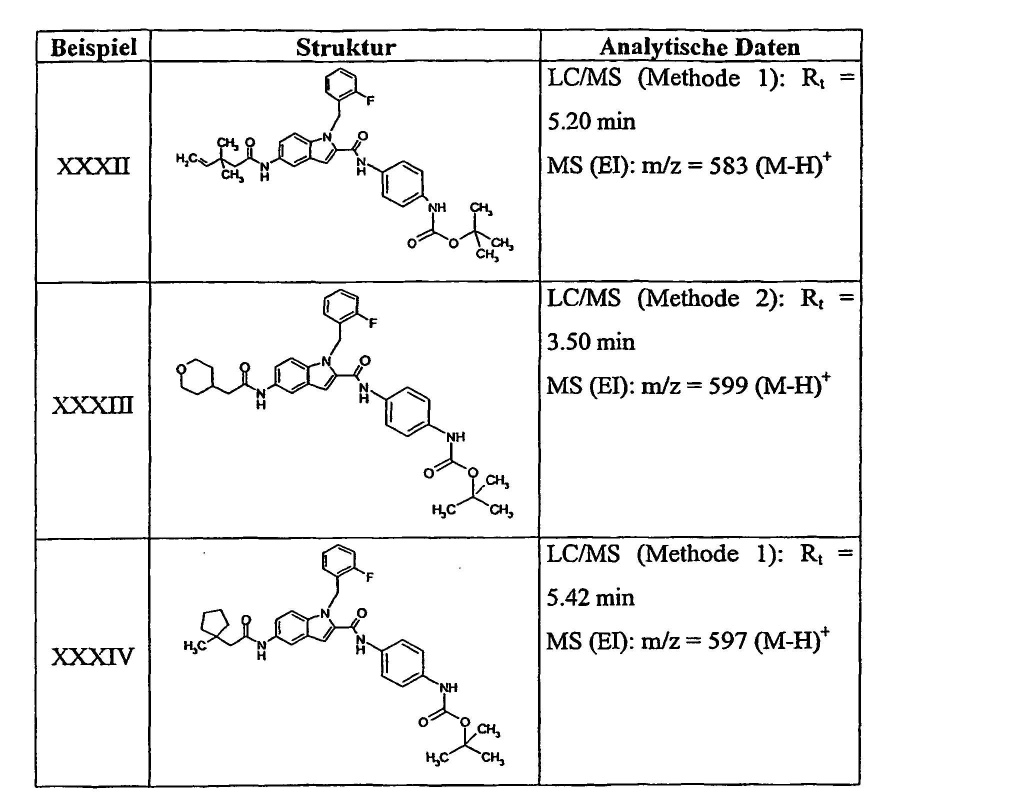

### Beispiel XXXV

### N-(4-Aminophenyl)-1-(2-fluorbenzyl)-5-[(tetrahydro-2H-pyran-4-ylacetyl)amino]-1-H-indol-2-carboxamid-Hydrochlorid

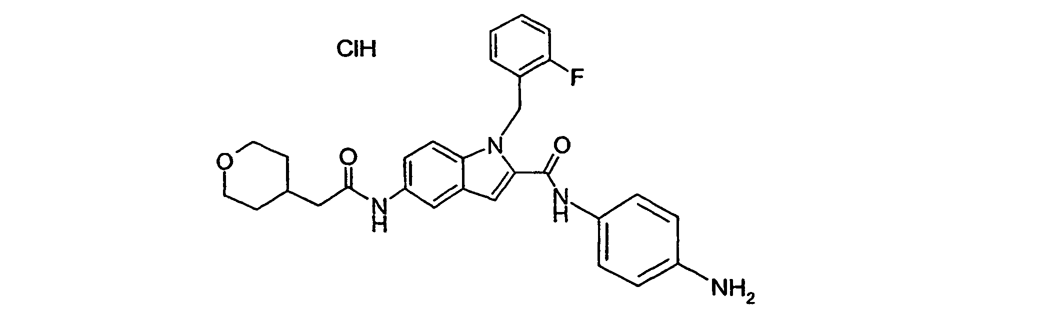

Es werden 121 mg (0.20 mmol) tert-Butyl-4-[({1-(2-fluorbenzyl)-5-[(tetrahydro-2H-pyran-4-ylacetyl)amino]-1H-indol-2-yl}carbonyl)amino]phenylcarbamat aus Beispiel XXXIII. 1.40 ml Dioxan und 1.40 ml konz. Salzsäure zusammengegeben und eine Stunde bei RT gerührt. Es wird zur Trockene einrotiert.
Ausbeute: 126 mg (64 % d. Th.)
LC/MS (Methode 2): Rₜ = 2.22 min
MS (EI): m/z = 501 (M+H-HCl)⁺

### Beispiel XXXVI

### 5-Amino-1-(2-fluorbenzyl)-1H-indol-2-carbonsäureethylester

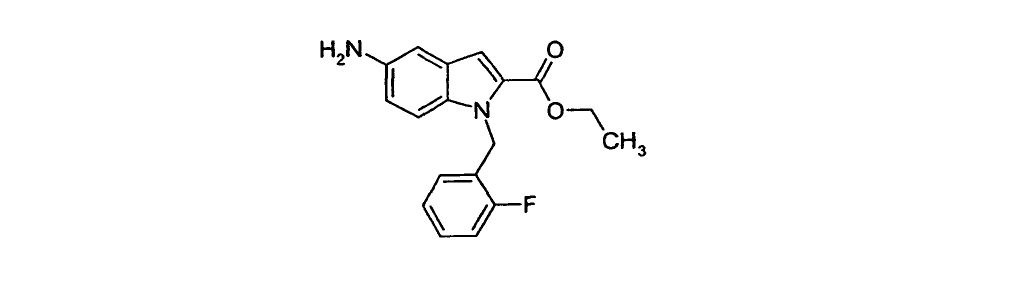

Man legt 27.84 g (81.33 mmol) 1-(2-Fluorbenzyl)-5-nitro-1H-indol-2-carbonsäureethylester aus Beispiel II in 750 ml Ethylacetat und 750 ml Ethanol vor. Dazu wird 20.51 g (325.31 mmol) Ammoniumformiat und 2.78 g Palladium auf Aktivkohle gegeben. Es wird zum Rückfluss gekocht, nach einer Stunde abgekühlt und über Kieselgur abfiltriert. Es wird mit Ethylacetat nachgewaschen. Das Lösungsmittel wird im Vakuum abgezogen und der Rückstand getrocknet.
Ausbeute: 23.2 g (86 % d. Th.)
HPLC (Methode 6): Rₜ = 4.15 min
MS (ESIpos): m/z = 313 (M+H)⁺

### Beispiel XXXVII

### 5-[(3,3-Dimethylbutanoyl)amino]-1-(2-fluorbenzyl)-1H-indol-2-carbonsäureethylester

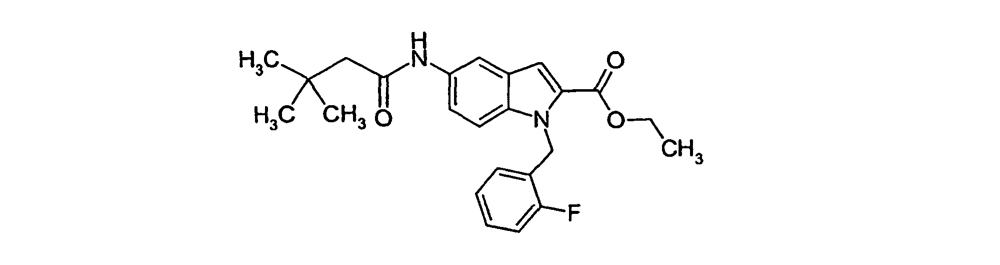

Man gibt 23.2 g (74.28 mmol) 5-Amino-1-(2-fluorbenzyl)-1H-indol-2-carbonsäureethylester aus Beispiel XXXVI mit 15.03 g 20.71 ml (148.56 mmol) Triethylamin in 300 ml Dichlormethan. Es wird auf 0°C abgekühlt und mit einer Lösung aus 11 g 11.35 ml (81.71 mmol) 3,3-Dimethylbuttersäurechlorid in 300 ml Dichlormethan versetzt. Es wird über Nacht bei RT nachgerührt und zur Aufarbeitung auf Wasser gegeben. Es wird auf pH 7 gestellt und 3 mal mit Ethylacetat extrahiert. Die vereinigte organische Phase wird mit Natriumsulfat getrocknet, filtriert und im Vakuum getrocknet.
Ausbeute: 31.7 g (100 % d. Th.)
HPLC (Methode 6): Rₜ = 5.18 min
MS (ESIpos): m/z = 411 (M+H)⁺

### Beispiel XXXVIII

### 1-(2-Fluorbenzyl)-5- {[(2-methyl-1,3-dioxolan-2-yl)acetyl]amino}-1H-indol-2-carbonsäureethylester

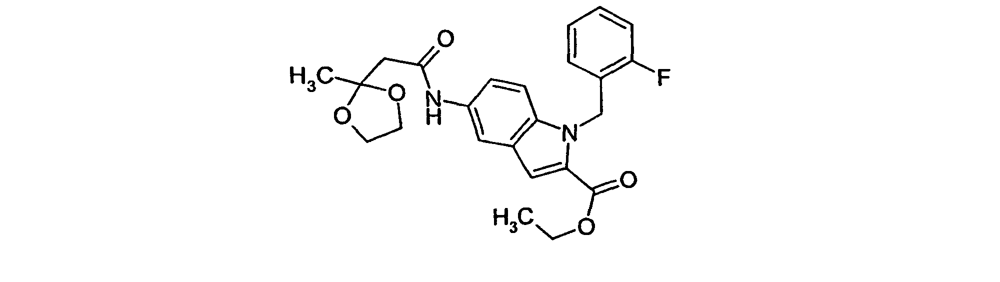

Man gibt 140 mg (0.96 mmol) (2-Methyl-1,3-dioxolan-2-yl)essigsäure in 5 ml DMF und 547 mg (1.44 mmol) O-(7-Azabenzotriazol-1-yl)-N,N,N',N'-tetramethyluroniumhexafluorophosphat mit 186 mg 0.25 ml (1.44 mmol) N,N-Diisopropylethylamin dazu. Anschließend wird 300 mg (0.96 mmol) 5-Amino-1-(2-fluorbenzyl)-1H-indol-2-carbonsäureethylester aus Beispiel XXXVI zugegeben. Die Mischung wird 3 Stunden bei RT gerührt. Zur Aufarbeitung wird das DMF abrotiert. Und der Rückstand in Dichlormethan aufgenommen und mit wässriger Salzsäure extrahiert. Die organische Phase wird mit Natriumsulfat getrocknet, filtriert und im Vakuum getrocknet.
Ausbeute: 254 mg (44 % d. Th.)
LC/MS (Methode 5): Rₜ = 2.98 min
MS (EI): m/z = 441 (M+H)⁺

### Beispiel XXXIX

### 5-[(3,3-Dimethylbutanoyl)amino]-1-(2-fluorbenzyl)-1H-indol-2-carbonsäure

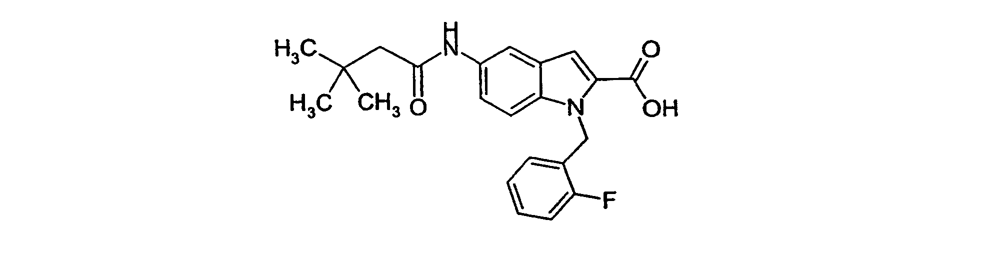

Herstellung analog wie Beispiel XXII mit 12.50 g (31.53 mmol) 5-[(3,3-Dimethylbutanoyl)amino]-1-(2-fluorbenzyl)-1H-indol-2-carbonsäureethylester aus Beispiel XXXVII und 31.5 ml (63.0 mmol) 2 M Lithiumhydroxidlösung.
Ausbeute: 9.93 g (81 % d. Th.)
HPLC (Methode 6): Rₜ = 4.57 min
MS (ESIpos): m/z= 383 (M+H)⁺

### Beispiel XL

### 1-(2-)-Fluorbenzyl)-5-{[(2-methyl-1,3-dioxolan-2-yl)acetyl]amino}-1H-indol-2-carbonsäure

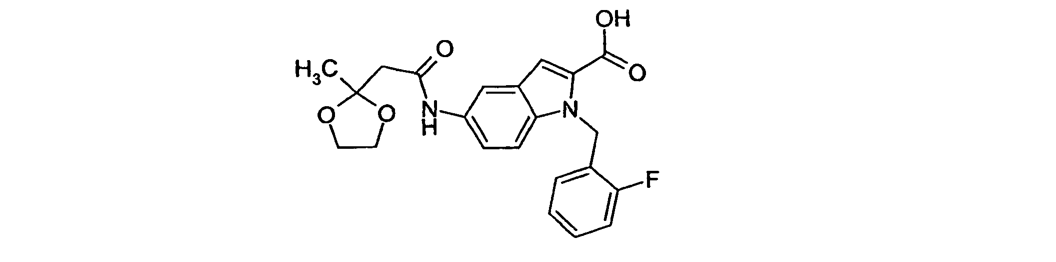

Herstellung analog wie Beispiel XXII mit 234 mg (0.63 mmol) Ethyl-1-(2-fluorbenzyl)-5-{[(2-methyl-1,3-dioxolan-2-yl)acetyl]amino}-1H-indol-2-carbonsäureethylester aus Beispiel XXXVIII und 0.53 ml (1.06 mmol) Lithiumhydroxidlösung.
Ausbeute: 198 mg (36 % d. Th.)
LC/MS (Methode 1): Rₜ = 4.00 min
MS (EI): m/z = 413 (M+H)⁺

### Beispiel XLI

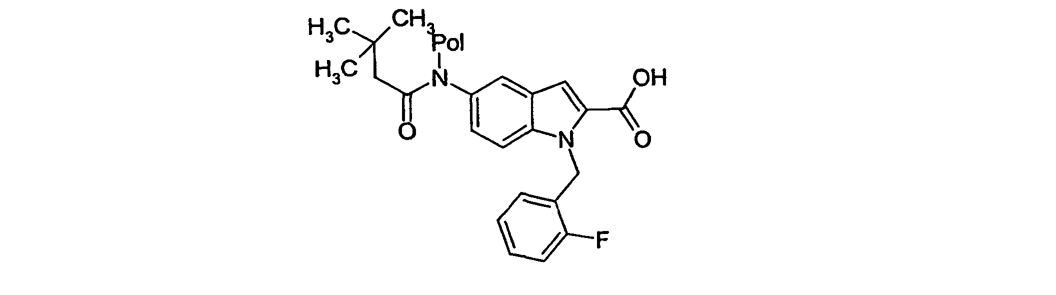

Es wird 100 mg (0.08 mmol) NovaCHO Harz in Toluol/Trimethylorthoformiat vorgelegt und mit 130 mg (0.42 mmol) 5-Amino-1-(2-fluorbenzyl)-1H-indol-2-carbonsäureethylester aus Beispiel XXXVI versetzt. Es wird 20 Stunden geschüttelt, anschließend abfiltriert und mit DMF gewaschen. Das resultierende Harz wird in DMF vorlegt und mit 86 mg (0.33 mmol) Tetra-n-butylammoniumborhydrid versetzt. Es wird 20 Stunden geschüttelt, anschließend abfiltriert und mit Methanol, Dichlormethan/Essigsäure 10/1, Methanol, Dichlormethan/Diethylether 10/1, Methanol und Dichlormethan gewaschen.

1000 mg (0.85 mmol) des beschriebenen Harzes wird mit 30 ml Dichlormethan, mit 1.29 g (1.77 ml, 12.75 mmol) Triethylamin und 1.14 g (1.19 ml, 8.50 mmol) Dimethylbuttersäurechlorid versetzt. Dann wird die Mischung 20 Stunden geschüttelt, abgesaugt und mit DMF, Methanol und Dichlormethan nachgewaschen.

1000 mg (2.61 mmol) des so entstandenen Harzes wird mit 15 ml Dioxan und mit 7.5 ml Kaliumhydroxid/Methanol (100 mg/ml) versetzt. Dann wird die Mischung über das Wochenende geschüttelt, abgesaugt und mit DMF, 30 %iger Essigsäure, Methanol und Dichlormethan nachgewaschen.

### Beispiel XLII

### Di-(tert-butyl)-5-({[5-[(3,3-dimethylbutanoyl)amino]-1-(2-fluorbenzyl)-1H-indol-2-yl]carbonyl} amino)-2-pyridinylimidodicarbonat

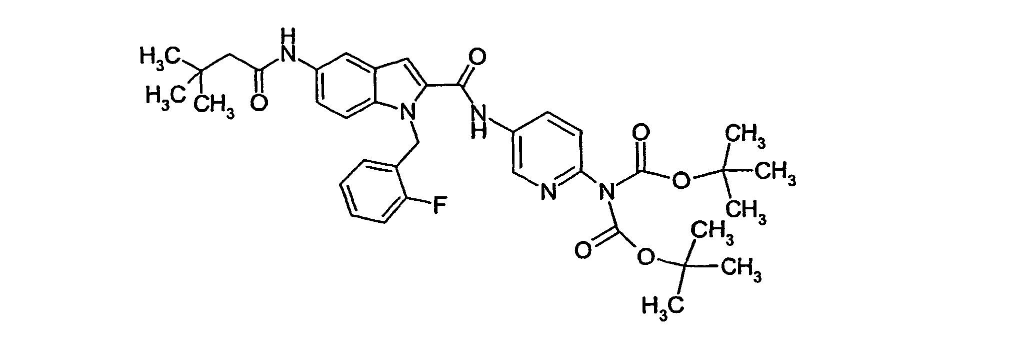

### Di-(tert-butyl)-5-nitro-2-pyridinylimidodicarbonat:

Es werden 5.0 g (35.94 mmol) 2-Amino-5-nitropyridin in 200 ml Dichlormethan gelöst und auf 0°C abgekühlt. Dazu werden dann 9.29 g 12.52 ml (71.88 mmol) N,N-Diisopropylethylamin, 19.61 g (89.86 mmol) Pyrokohlensäure-di-tert.-buytlester und 4.83 g (39.54 mmol) 4-Dimethylaminopyridin zugegeben. Das Gemisch wird über Nacht bei RT gerührt. Es wird mit Ethylacetat verdünnt, mit wässriger Ammoniumchloridlösung dreimal, mit ges. Natriumchloridlösung einmal, mit wässriger Natriumhydrogencarbonatlösung zweimal und noch einmal mit gesättigter Natriumchloridlösung gewaschen. Die organische Phase wird mit Natriumsulfat getrocknet, filtriert und im Vakuum getrocknet.
Ausbeute: 10 g (82 % d. Th.)

### Di-(tert-butyl)-5-amino-2-pyridinylimidodicarbonat:

Man löst 7.0 g (20.63 mmol) Di-(tert-butyl)-5-nitro-2-pyridinylimidodicarbonat in 150 ml Ethanol und 50 ml Dichlormethan. Es wird bei Normaldruck hydriert. Zur Aufarbeitung wird der Ansatz über einem Seitzfilter filtriert und mit THF gewaschen. Das Filtrat wird im Vakuum getrocknet.
Ausbeute: 5.70 g (89 % d. Th.)

### Titelverbindung:

Unter Argon werden 200 mg (0.52 mmol) 5-[(3,3-Dimethylbutanoyl)amino]-1-(2-fluorbenzyl)-1H-indol-2-carbonsäure aus Beispiel XXXIX und 3.91 g 4 ml (49.46 mmol) Pyridin in 2 ml DMF vorgelegt. Es wird mit 596 mg (1.57 mmol) O-(7-Azabenzotriazol-1-yl)-N,N,N',N'-tetramethyluroniumhexafluorophosphat und 323 mg (1.05 mmol) Di-(tert-butyl)-5-amino-2-pyridinylimidodicarbonat versetzt. Es wird über Nacht bei RT gerührt und das Lösungsmittel im Vakuum entfernt. Der Rückstand wird in Ethylacetat und ges. Natriumchloridlösung extrahiert. Die organische Phase wird mit Natriumsulfat getrocknet, filtriert und im Vakuum getrocknet.
Es wird über eine HPLC gereinigt.
Ausbeute: 111 mg (24 % d. Th.)
LC/MS (Methode 2): Rₜ = 4.30 min
MS (EI): m/z = 672 (M-H)⁺

Die Herstellung der folgenden Verbindung erfolgt analog zu der in Beispiel XLII (Amidkupplung) beschriebenen Weise:

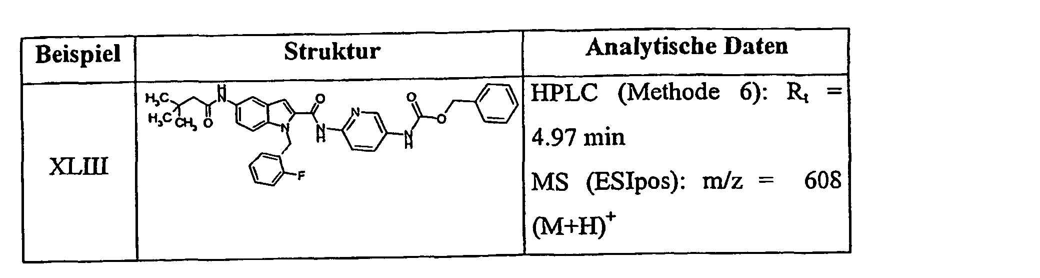

### Beispiel XLIV

### 5-[(3,3-Dimethylbutanoyl)amino]-1-(2-fluorbenzyl)-N-(5-nitro-2-pyridinyl)-1H-indol-2-carboxamid

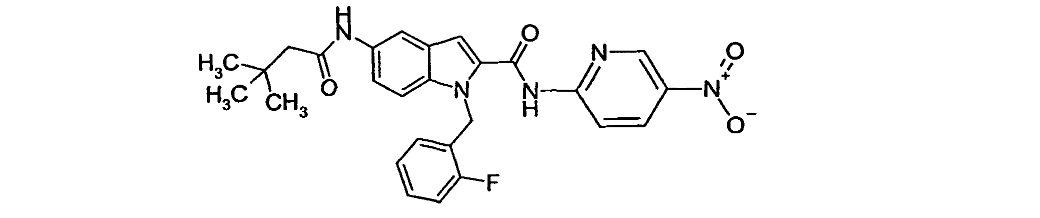

Unter Argon werden 400 mg (1.05 mmol) 5-[(3,3-Dimethylbutanoyl)amino]-1-(2-fluorbenzyl)-1H-indol-2-carbonsäure aus Beispiel XXXIX in 10 ml DMF gelöst. Es wird auf 0°C abgekühlt und mit 202 mg (81.57 mmol) N,N-Diisopropylethylamin und 278 mg (1.26 mmol) N,N-Bis-(2-methoxyethyl)-N-(trifluor-14-sulfanyl)amin versetzt. Es wird 15 Minuten bei dieser Temperatur nachgerührt und sofort weiter umgesetzt.

Die Hälfte der Lösung wird auf 0°C abgekühlt und mit 119 mg (0.86 mmol) 5-Nitro-2-pyridinamin versetzt. Nach 15 Minuten lässt man auf RT kommen und rührt für weitere 24 Stunden nach. Zur Aufarbeitung wird mit Ethylacetat verdünnt und mit wässriger Natriumhydrogencarbonatlösung 3 mal gewaschen. Die organische Phase wird einmal mit ges. Natriumchloridlösung gewaschen, mit Natriumsulfat getrocknet, filtriert und im Vakuum einrotiert. Der Rückstand wird über präparative HPLC gereinigt.
Ausbeute: 55 mg (25 % d. Th.)
HPLC (Methode 1): Rₜ = 5.20 min
MS (ESIpos): m/z = 504 (M+H)⁺

### Beispiel XLV

### tert-Butyl-4-({[5-[(3,3-dimethylbutanoyl)amino]-1-(2-fluorbenzyl)-1H-indol-2-yl]carbonyl}amino)phenylcarbamat

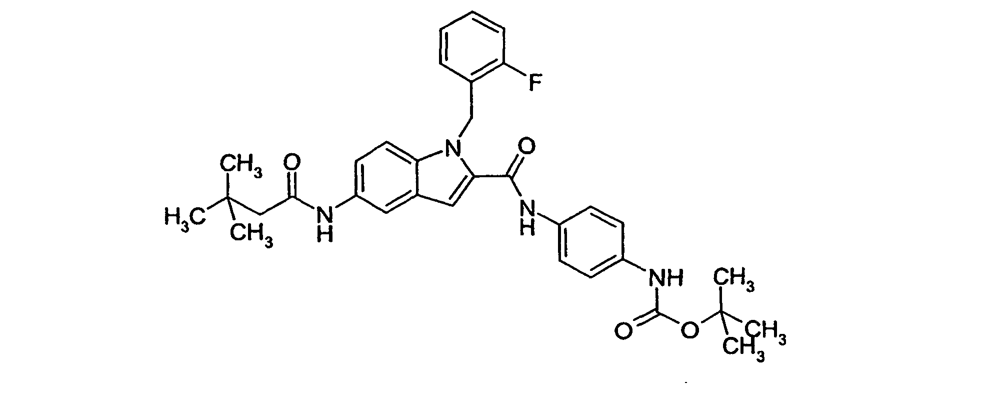

Man legt 50 mg (0.13 mmol) 5-[(3,3-Dimethylbutanoyl)amino]-1-(2-fluorbenzyl)-1H-indol-2-carbonsäure aus Beispiel XXXIX, 37.6 mg (0.20 mmol) N'-(3-Dimethylaminopropyl)-N-ethylcarbodiimid x HCl und 8 mg (0.07 mmol) 4-Dimethylaminopyridin in DMF vor. Dazu wird 32.7 mg (0.16 mmol) tert-Butyl-4-aminophenylcarbamat zugegeben und über Nacht bei RT nachgerührt. Das Gemisch wird zur Aufarbeitung mit wässriger Salzsäure und Dichlormethan verdünnt und extrahiert. Die organische Phase wird mit ges. Natriumchloridlösung gewaschen, mit Natriumsulfat getrocknet, filtriert und im Vakuum getrocknet.
Ausbeute: 104 mg (82 % d. Th.)
LC/MS (Methodc 4): Rₜ = 5.20 min
MS (EI): m/z = 571 (M-H)⁺

Die Herstellung der folgenden Verbindung erfolgt analog zu der in Beispiel XLV beschriebenen Weise:

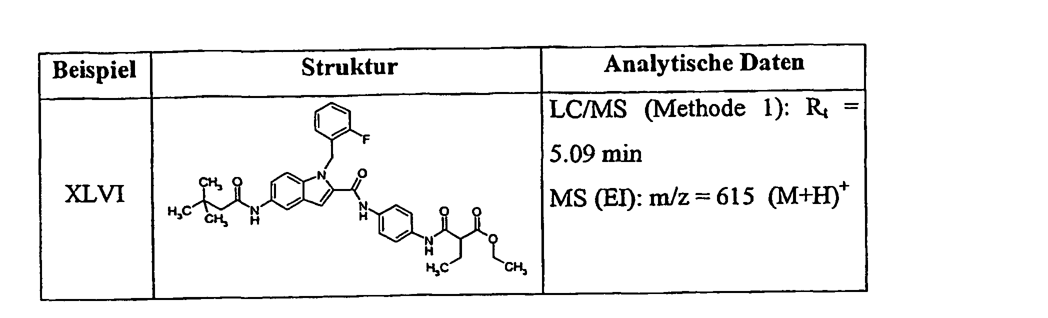

### Beispiel XLVII

### 5-Nitro- 1H-indol-2-carbonsäure

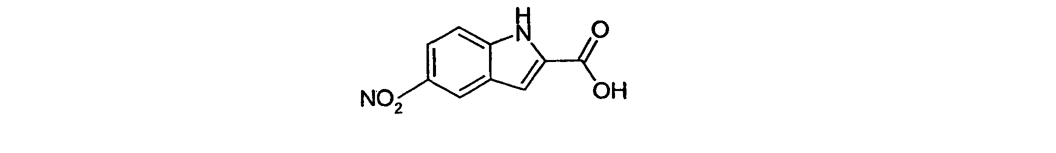

Man löst 16.5 g (66.93 mmol) 5-Nitroindol-2-carbonsäureethylester in je 200 ml Methanol und THF und gibt dazu 67 ml (133.85 mmol) Lithiumhydroxidlösung dazu. Es wird für eine halbe Stunde bei 90°C erhitzt. Nach dem Abkühlen wird das Gemisch zur Aufarbeitung mit wässriger Salzsäure und Ethylacetat verdünnt und extrahiert. Die organische Phase wird mit ges. Natriumchloridlösung gewaschen, mit Natriumsulfat getrocknet, filtriert und im Vakuum getrocknet.
Ausbeute: 15 g (100 % d. Th.)
LC/MS (Methode 4): Rₜ = 3.18 min
MS (EI): m/z = 205 (M-H)⁺

### Beispiel XLVIII

### 5-Nitro-N-phenyl-1H-indol-2-carbonsäureamid

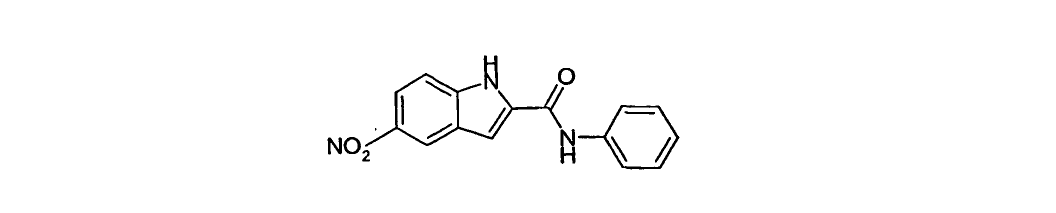

Man legt 5.44 g (26.39 mmol) 5-Nitro-1H-indol-2-carbonsäure aus Beispiel XL VII, 7.59 g (39.58 mmol) N'-(3-Dimethylaminopropyl)-N-ethylcarbodiimid x HCl und 1.61 g (13.19 mmol) 4-Dimethylaminopyridin in 400 ml Dichlormethan:DMF 10:1 Gemisch vor. Dazu wird 2.95 g 2.89 ml (31.67 mmol) Anilin zugegeben und über Nacht bei RT nachgerührt. Das Gemisch wird zur Aufarbeitung mit wässriger Salzsäure und Dichlormethan verdünnt und extrahiert. Die organische Phase wird mit ges. Natriumchloridlösung gewaschen, mit Natriumsulfat getrocknet, filtriert und im Vakuum getrocknet.
Ausbeute: 5.83 g (75 % d. Th.)
LC/MS (Methode 4): Rₜ = 4.01 min
MS (EI): m/z = 280 (M-H)⁺

### Beispiel XLIX

### 5-Nitro-N-phenyl-1-(2-phenylethyl)-1H-indol-2-carbonsäureamid

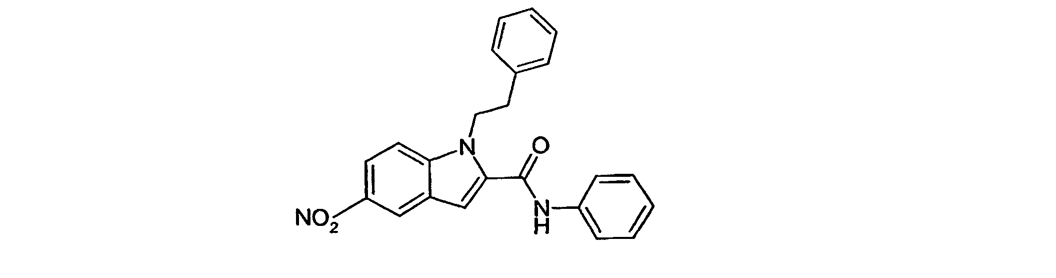

200 mg (0.71 mmol) 5-Nitro-N-phenyl-1H-indol-2-carbonsäureamid aus Beispiel XLVIII werden unter Argon in 5 ml Dimethylformamid vorgelegt. 85.3 mg (2.13 mmol Natriumhydrid (60 %ige Dispersion in Paraffin) werden portionsweise zugegeben und der Ansatz 30 min bei RT gerührt. Anschließend wird 657 mg (3.56 mmol) (2-Bromethyl)benzol zugegeben und der Ansatz für 5 h bei 100°C nachgerührt. Zur Beendigung der Reaktion erfolgt die weitere Zugabe von 3 eq. Natriumhydrid und 5 eq. Bromid und man lässt bei 100°C für 7 Stunden rühren. Das Reaktionsgemisch wird auf wässrige Salzsäure gegeben und mit Essigsäureethylester ausgeschüttelt. Die organische Phase wird über Natriumsulfat getrocknet und am Rotationsverdampfer vom Lösemittel befreit. Das erhaltene Rohprodukt wird säulenchromatographisch gereinigt (Kieselgel 60, Laufmittel: Cyclohexan-Essigsäureethylester 6:1).
Ausbeute: 56 mg (20 % d. Th.)
LC/MS (Methode 1): Rₜ = 5.05 min
MS (EI): m/z = 384 (M-H)⁺

### Beispiel L

### [2-(Anilincarbonyl)-5-nitro-1H-indol-1-yl]essigsäure-tert-butylester

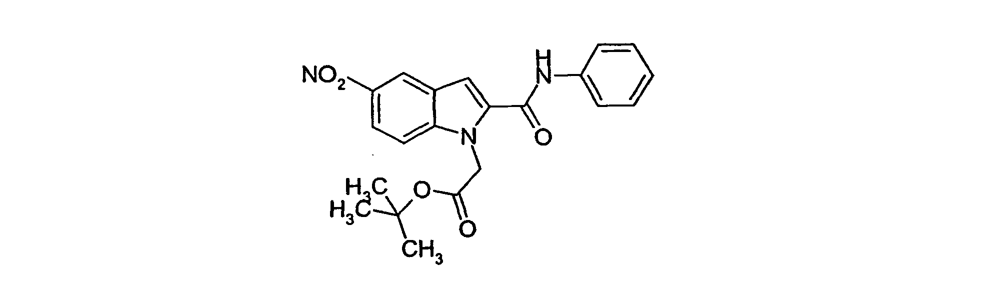

Unter Argon wird 328 mg (1.24 mmol) 1,4,7,10,13,16-Hexaoxacyclooctadecan (18-Krone-6) in 61 ml Dichlormethan vorgelegt und mit 15 ml (14.93 mmol) 1 molarer Kalium-*tert*.-butylatlösung in THF und 3.50 g (12.44 mmol) 5-Nitro-N-phenyl-1H-indol-2-carbonsäureamid aus Beispiel XLVIII versetzt. Es wird 15 Minuten bei RT nachgerührt und auf 0°C abgekühlt. Dazu wird eine Lösung aus 3.64 g (18.67 mmol) tert-Butylbromacetat in 100 ml THF langsam zugetropft. Das Eisbad wird entfernt und über Nacht bei RT gerührt. Zur Aufarbeitung wird mit Wasser verdünnt und das THF im Vakuum abrotiert. Der wässrige Rückstand wird mit Ethylacetat extrahiert und die organische Phase wird mit ges. Natriumchloridlösung gewaschen, mit Natriumsulfat getrocknet, filtriert und im Vakuum getrocknet.
Ausbeute: 4.35 g (69 % d. Th.)
LC/MS (Methode 2): Rₜ = 3.90 min
MS (EI): m/z = 418 (M+Na)⁺

### Beispiel LI

### 5-Amino-N-phenyl-1-(2-phenylethyl)-1H-indol-2-carbonsäureamid

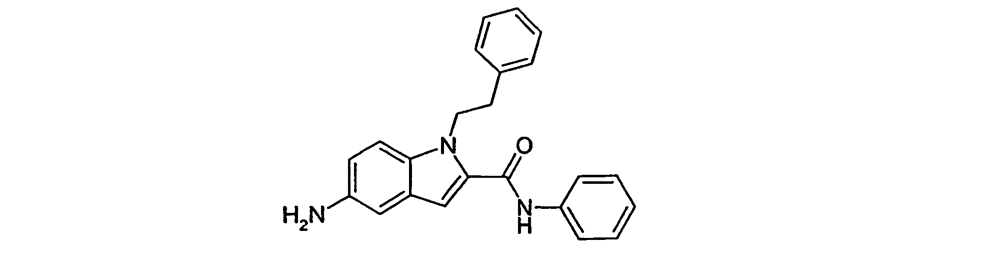

Man legt 50 mg (0.13 mmol) 5-Nitro-N-phenyl-1-(2-phenylethyl)-1H-indol-2-carbonsäureamid aus Beispiel XLIX in 7 ml Ethylacetat und 7 ml Ethanol vor. Dazu wird 49 mg (0.78 mmol) Ammoniumformiat und 14 mg Palladium auf Aktivkohle gegeben. Es wird zum Rückfluss gekocht und bei 50°C entsteht eine Gasentwicklung. Nach 4 Stunden bei Rückfluss wird abgekühlt und über Kieselgur abfiltriert und mit 500 ml Ethanol nachgewaschen. Das Lösungsmittel wird im Vakuum abgezogen und der Rückstand getrocknet. Man erhält 115 mg eines weißen Feststoffs, der noch anorganische Salze enthält und ohne Reinigung weiter umgesetzt wird.

Die Herstellung der folgenden Verbindung erfolgt analog zu der in Beispiel XLXI beschriebenen Weise:

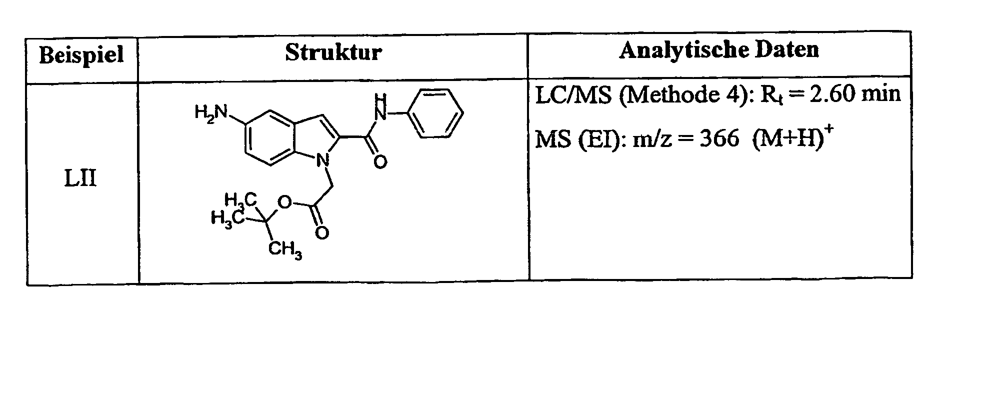

### Beispiel LIII

### {2-(Anilincarbonyl)-5-[(3,3-dimethylbutanoyl)amino]-1H-indol-1-yl} essigsäure-tert-butylester

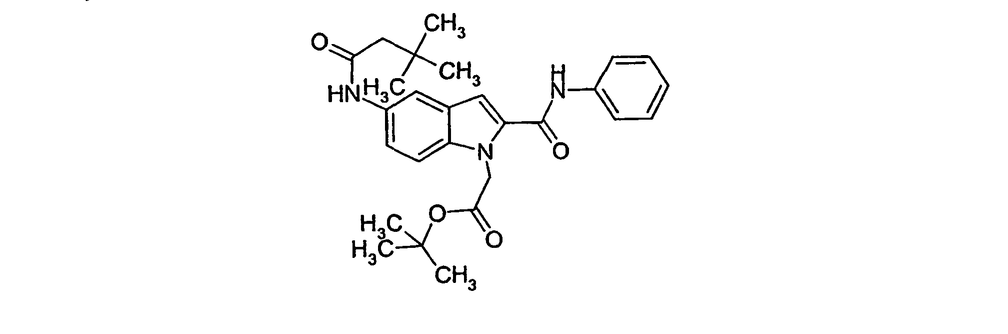

Unter Argon gibt man 50 mg (0.14 mmol) [5-Amino-2-(anilincarbonyl)-1H-indol-1-yl]essigsäure-tert-butylester aus Beispiel LII mit 15.23 mg (0.02 ml, 0.15 mmol) Triethylamin in 2 ml THF. Es wird auf 0°C abgekühlt und mit einer Lösung aus 20.26 mg (0.02 ml, 0.15 mmol) 3,3-Dimethylbuttersäurechlorid in 0.2 ml THF versetzt. Es wird 2 Stunden bei RT nachgerührt und zur Aufarbeitung in verdünnte Salzsäure und Ethylacetat gegeben und extrahiert. Die organische Phase wird mit ges. Natriumhydrogencarbonatlösung gewaschen, mit Natriumsulfat getrocknet, filtriert und im Vakuum getrocknet. Das erhaltene Rohprodukt wird säulenchromatographisch gereinigt (Kieselgel 60, Laufmittel: Cyclohexan-Essigsäureethylester 2:1).
Ausbeute: 80 mg (93 % d. Th.)
LC/MS (Methode 1): Rₜ = 4.78 min
MS (EI): m/z = 464 (M+H)⁺

### Beispiel LIV

### {2-(Anilincarbonyl)-5-[(3,3-dimethylbutanoyl)amino]-1H-indol-1-yl}essigsäure

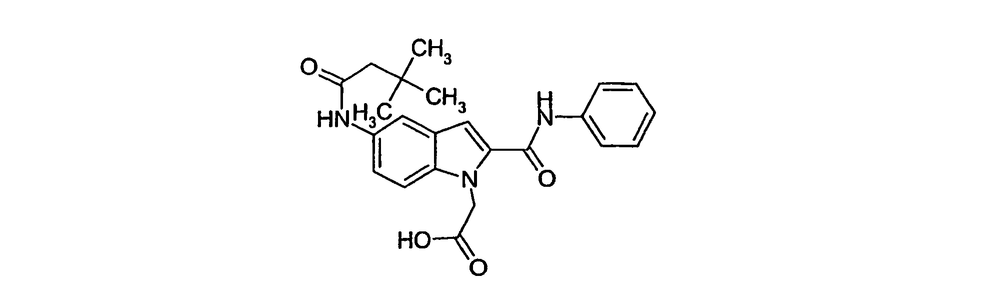

70 mg (0.15 mmol) {2-(Anilincarbonyl)-5-[(3,3-dimethylbutanoyl)amino]-1H-indol-1-yl}essigsäure-tert-butylester aus Beispiel LIII wird mit 0.50 ml Trifluoressigsäure und 1 ml Dichlormethan zusammen eine Stunde bei RT gerührt. Das Lösungsmittel wird unter vermindertem Druck entfernt und der Rückstand im Vakuum getrocknet.
Ausbeute: 86.7 mg (100 % d. Th.)
LC/MS (Methode 1): Rₜ = 4.43 min
MS (EI): m/z = 408 (M+H)⁺

### Beispiel LV

### {2-(Anilincarbonyl)-5-[(3,3-dimethylbutanoyl)amino]-1H-indol-1-yl}essigsäureethylester

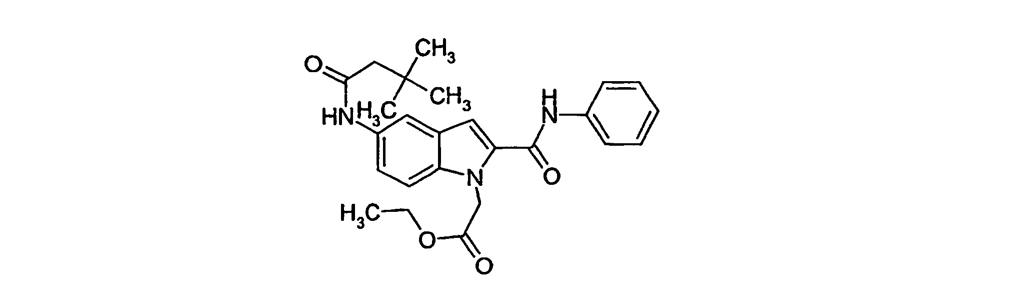

Man legt 1.50 g (3.68 mmol) {2-(Anilincarbonyl)-5-[(3,3-dimethylbutanoyl)amino]-1H-indol-1-yl}essigsäure aus Beispiel LIV, 225 mg (1.84 mmol) 4-Dimethylaminopyridin und 203.5 mg (4.42 mmol) Ethanol in Dichlormethan vor. Die Mischung wird auf 0°C abgekühlt und mit 776 mg (4.05 mmol) N'-(3-Dimethylaminopropyl)-N-ethylcarbodiimid x HCl versetzt. Es wird für 4 Stunden bei RT nachgerührt. Das Gemisch wird zur Aufarbeitung mit Wasser und Dichlormethan verdünnt und extrahiert. Die organische Phase wird mit Natriumsulfat getrocknet, filtriert und im Vakuum getrocknet. Das erhaltene Rohprodukt wird säulenchromatographisch gereinigt (Kieselgel 60, Laufmittel: Cyclohexan-Essigsäureethylester 2:1-1:1).
Ausbeute: 227 mg (14 % d. Th.)
LC/MS (Methode 4): Rₜ = 4.60 min
MS (EI): m/z = 436 (M+H)⁺

### Beispiel LVI

### 5-Nitro-1-phenyl-1H-indol-2-carbonsäureethylester

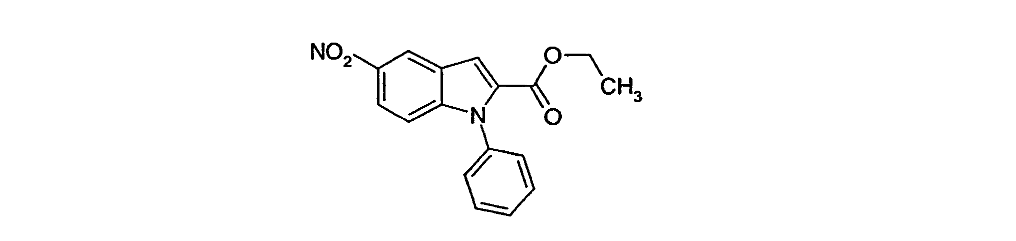

5.5 g (22.31 mmol) 5-Nitro-1H-indol-2-carbonsäureethylester, 6.16 g (44.62 mmol) wasserfreies Kaliumcarbonat, 77.53 g (52 ml, 493.8 mmol) Brombenzol und 1.6 g (11.15 mmol) Kupferbromid werden unter Rückfluss (ca. 156°C) 5 Tage gerührt. Anschließend wird das Reaktionsgemisch filtriert und der Rückstand auf der Fritte mit Toluol gewaschen. Die gesammelten Filtrate werden eingeengt, im Hochvakuum getrocknet und durch Flashchromatographie an Kieselgel gereinigt.
Ausbeute: 5.71 g (82 % d. Th.)
LC/MS (Methode 1): Rₜ = 5.14 min
MS (EI): m/z = 309 (M-H)⁺

### Beispiel LVII

### 5-Amino-1-phenyl-1H-indol-2-carbonsäureethylester

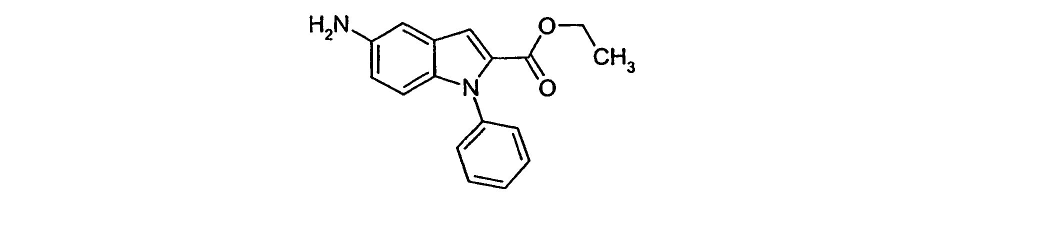

Man legt 300 mg (0.97 mmol) 5-Nitro-1-phenyl-1H-indol-2-carbonsäureethylester aus Beispiel LVI in 40 ml Ethylacetat und 40 ml Ethanol vor. Dazu wird 365 mg (5.80 mmol) Ammoniumformiat und 102 mg 10 %ige Palladium auf Aktivkohle gegeben. Es wird zum Rückfluss gekocht und bei 50°C entsteht eine Gasentwicklung. Nach 4 Stunden bei Rückfluss wird abgekühlt und über Kieselgur abfiltriert und mit 500 ml Ethanol nachgewaschen. Das Lösungsmittel wird im Vakuum abgezogen und der Rückstand getrocknet.
Ausbeute: 355 mg (93 % d. Th.)
LC/MS (Methode 5): Rₜ = 2.17 min
MS (EI): m/z = 281 (M+H)⁺

### Beispiel LVIII

### 5-[(3,3-Dimethylbutanoyl)amino]-1-phenyl-1H-indol-2-carbonsäureethylester

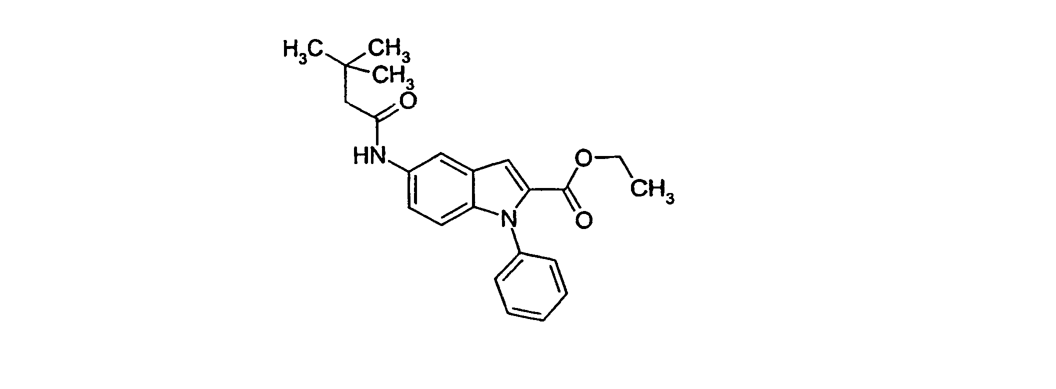

Unter Argon gibt man 355 mg (1.27 mmol) 5-Amino-1-phenyl-1H-indol-3-carbonsäureethylester aus Beispiel LVII mit 141 mg (0.19 ml, 1.39 mmol) Triethylamin in 4 ml THF. Es wird auf 0°C abgekühlt und mit einer Lösung aus 170 mg (0.18 ml, 1.27 mmol) 3,3-Dimethylbuttersäurechlorid in 2 ml THF versetzt. Es wird 2 Stunden bei RT nachgerührt. Zur vollständigen Umsetzung werden weitere 1 eq. Triethylamin und 1 eq. Säurechlorid zugegeben und 2 Stunden bei RT nachgerührt. Zur Aufarbeitung wird auf verdünnte Salzsäure und Ethylacetat gegeben und extrahiert. Die organische Phase wird mit ges. Natriumhydrogencarbonatlösung gewaschen, mit Natriumsulfat getrocknet, filtriert und im Vakuum getrocknet. Das erhaltene Rohprodukt wird säulenchromatographisch gereinigt (Kieselgel 60, Laufmittel: Cyclohexan-Essigsäureethylester 3:1-1:1).
Ausbeute: 118 mg (25 % d. Th.)
LC/MS (Methode 5): Rₜ = 3.54 min
MS (EI): m/z = 379 (M+H)⁺

### Beispiel LIX

### 5-[(3,3-Dimethylbutanoyl)amino]-1-phenyl-1H-indol-2-carbonsäure

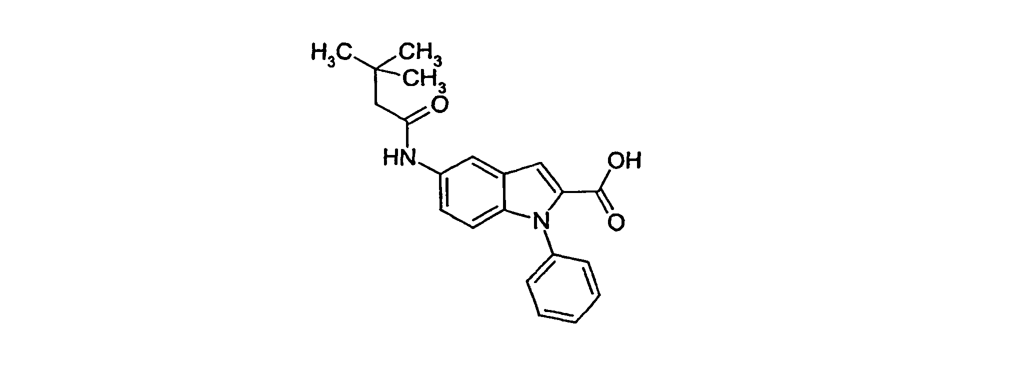

Man löst 119 mg (0.31 mmol) 5-[(3,3-Dimethylbutanoyl)amino]-1-phenyl-1H-indole-2-carbonsäureethylester aus Beispiel LVIII in je 2 ml Methanol und THF und gibt dazu 0.31 ml (0.63 mmol) 2 M Lithiumhydroxidlösung dazu. Es wird für eine Stunde bei 90°C erhitzt. Nach dem Abkühlen wird das Gemisch zur Aufarbeitung mit wässriger Salzsäure und Ethylacetat verdünnt und extrahiert. Die organische Phase wird mit Natriumsulfat getrocknet, filtriert und im Vakuum getrocknet.
Ausbeute: 151 mg (100 % d. Th.)
¹H-NMR (200 MHz, DMSO-d₆): δ =1.03 (s, 9H), 2.18 (s, 2H), 6.96 (d, 1H), 7.31-7.40 (m, 3H), 7.46-7.59 (m, 4H), 8.15 (s, 1H), 9.77 (s, 1H), 12.73 (br. s, 1H).

### Beispiel LX

### tert-Butyl-4-[({5-[(3,3-dimethylbutanoyl)amino]-1-phenyl-1H-indol-2-yl}carbonyl)-amino]phenylcarbamat

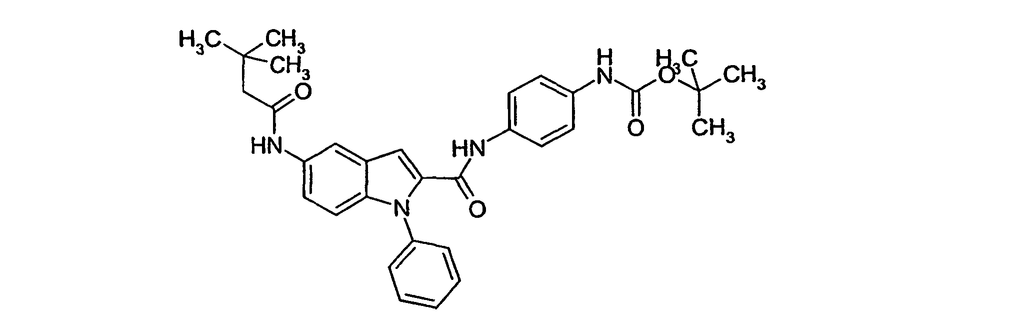

Man legt 75 mg (0.21 mmol) 5-[(3,3-Dimethylbutanoyl)amino]-1-phenyl-1H-indol-2-carbonsäure aus Beispiel LIX, 61.55 mg (0.32 mmol) N-(3-Dimethylaminopropyl)-N-ethylcarbodiimid x HCl und 13.1 mg (0.11 mmol) 4-Dimethylaminopyridin in 4 ml Dichlormethan vor. Dazu wird 44.6 mg (0.21 mmol) tert-Butyl-4-aminophenylcarbamat zugegeben und 3 Stunden bei RT nachgerührt. Das Gemisch wird zur Aufarbeitung mit wässriger Salzsäure und Dichlormethan verdünnt und extrahiert. Die organische Phase wird mit Natriumsulfat getrocknet, filtriert und im Vakuum getrocknet.
Ausbeute: 87 mg (60 % d. Th.)
LC/MS (Methode 4): Rₜ = 5.02 min
MS (EI): m/z = 539 (M-H)⁺

### Beispiel LXI

### 5-Amino-1H-indol-2-carbonsäureethylester

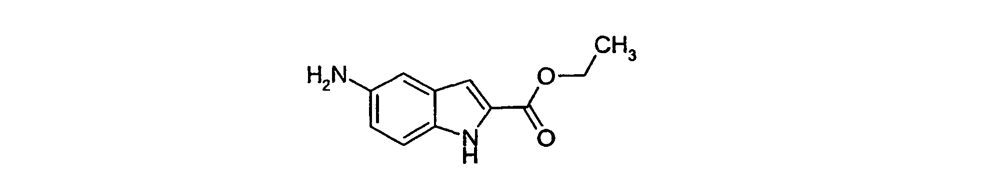

Unter Argon legt man 15 g (60.84 mmol) 5-Nitro-1H-indol-2-carbonsäureethylester in 750 ml Ethylacetat und 750 ml Ethanol vor. Dazu wird 15.82 g (15.82 mmol) Ammoniumformiat und 1.50 g 10 %ige Palladium auf Aktivkohle gegeben. Es wird 30 Minuten bei 90°C gerührt, dann wird abgekühlt und über Celite abfiltriert und mit Ethylacetat nachgewaschen. Das Lösungsmittel wird im Vakuum abgezogen und der Rückstand in Chloroform gelöst und zweimal mit Wasser gewaschen. Die organische Phase wird mit Natriumsulfat getrocknet, filtriert und im Vakuum einrotiert.
Ausbeute: 12.81 g (100 % d. Th.)
LC/MS (Methode 4): Rₜ = 0.37 min
MS (EI): m/z = 205 (M+H)⁺
¹H-NMR (200 MHz, DMSO-d₆): δ = 1.31 (t, 3H), 4.29 (q, 2H), 4.67 (s, 2H), 6.62-6.76 (m, 2H), 6.79-6.88 (m, 1H), 7.11-7.22 (m, 1H), 11.41 (br. s, 1H).

### Beispiel LXII

### 5-[(3,3-Dimethylbutanoyl)amino]-1H-indol-2-carbonsäureethylester

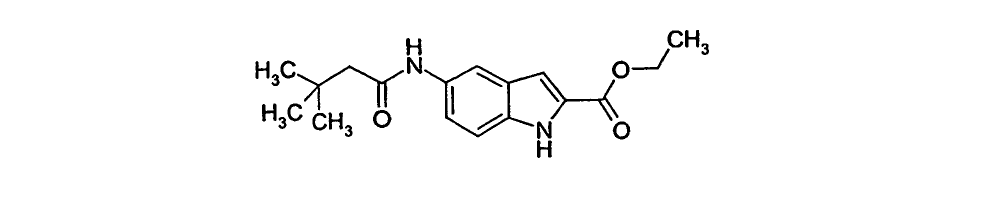

Man gibt 3.76 g (18.4 mmol) 5-Amino-1H-indol-2-carbonsäureethylester aus Beispiel LXI mit 2.05 g (2.82 ml, 20.3 mmol) Triethylamin in 40 ml THF. Es wird auf 0°C abgekühlt und mit einer Lösung aus 2.48g (2.56 ml, 18.4 mmol) 3,3-Dimethylbuttersäurechlorid in 20 ml THF versetzt. Es wird über 2 h bei RT nachgerührt und zur Aufarbeitung auf Wasser gegeben. Es wird auf pH 7 gestellt und 3 mal mit Ethylacetat extrahiert. Die vereinigte organische Phase wird mit Natriumsulfat getrocknet, filtriert und im Vakuum getrocknet.
Ausbeute: 5.49 g (98 % d. Th.)
HPLC (Methode 4): Rₜ = 4.20 min
MS (ESIpos): m/z = 303 (M+H)⁺

### Beispiel LXIII

### 5-[(3,3-Dimethylbutanoyl)amino]-1-[2-(trifluormethyl)benzyl]-1H-indol-2-carbonsäureethylester

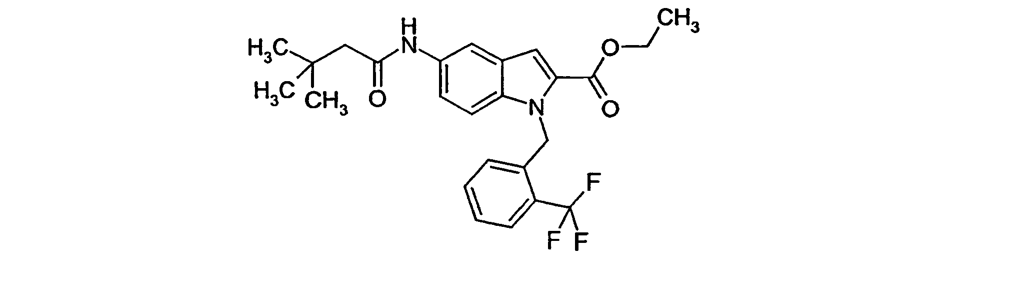

Unter Argon wird 35 mg (0.13 mmol) 1,4,7,10,13,16-Hexaoxacyclooctadecan (18-Krone-6) in 7 ml THF vorgelegt und mit 1.98 ml (1.98 mmol) 1 molarer Kalium-tert.-butylatlösung in THF und 400 mg (1.32 mmol) 5-[(3,3-Dimethylbutanoyl)-amino]-1H-indol-2-carbonsäureethylester aus Beispiel LXII versetzt. Es wird 15 Minuten bei RT nachgerührt und auf 0°C abgekühlt Dazu wird eine Lösung aus 474 mg (1.98 mmol) 2-Trifluormethylbenzylbromid in 12 ml THF langsam zugetropft. Das Eisbad wird entfernt und 1 Stunde bei RT gerührt. Zur Aufarbeitung wird mit Wasser verdünnt und das THF im Vakuum abrotiert. Der wässrige Rückstand wird mit Ethylacetat extrahiert und die organische Phase wird mit ges. Natriumchloridlösung gewaschen, mit Natriumsulfat getrocknet, filtriert und im Vakuum getrocknet. Der Rückstand wird säulenchromatographisch (Laufmittel: Cyclohexan:Ethylacetat 5:1) gereinigt.
Ausbeute: 238 mg (39 % d. Th.)
HPLC (Methode 5): Rₜ = 3.60 min
MS (ESTpos): m/z = 461 (M+H)⁺

Die Herstellung der folgenden Verbindungen erfolgt analog zu der in Beispiel LXIII beschriebenen Weise:

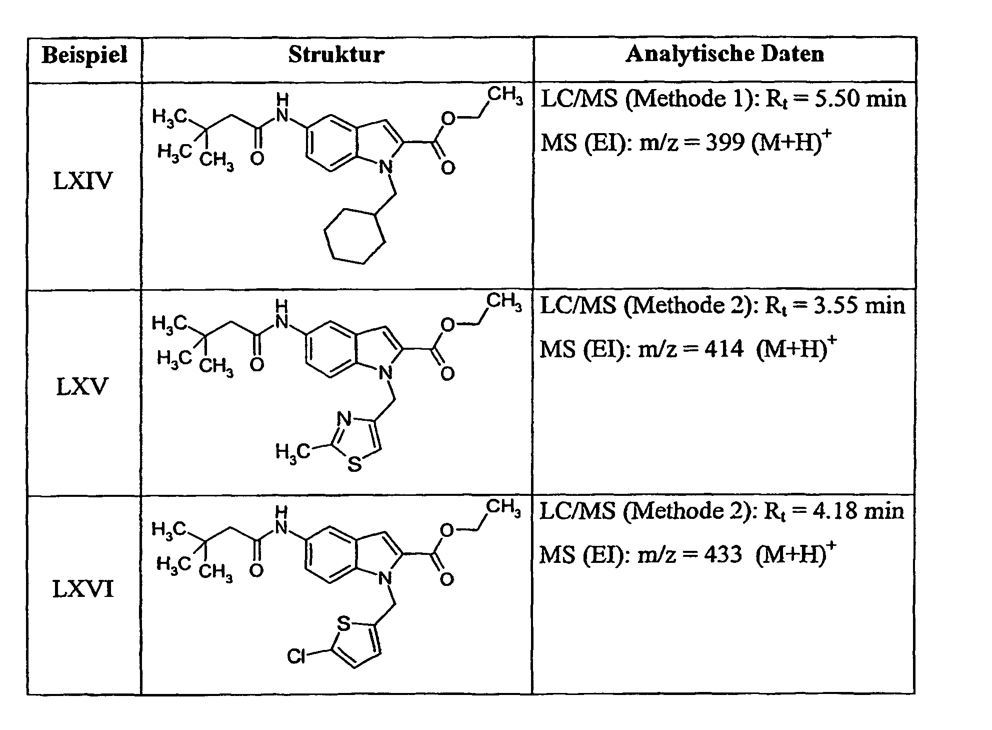

### Beispiel LXVII

### 5-[(3,3-Dimethylbutanoyl)amino]-1-[2-(trifluormethyl)benzyl]-1H-indol-2-carbonsäure

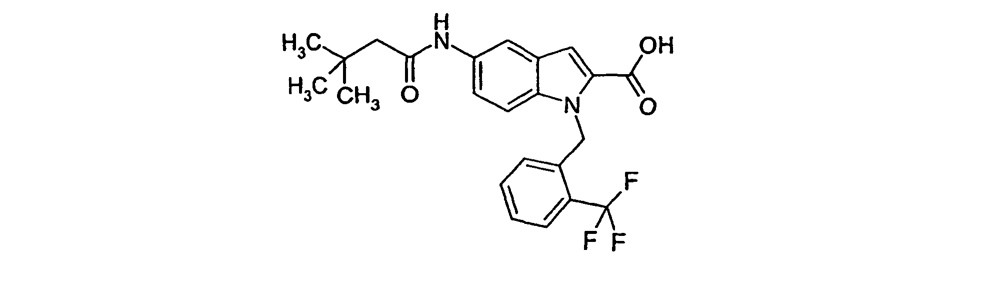

Man löst 105 mg (0.26 mmol) 5-[(3,3-Dimethylbutanoyl)amino]-1-[2-(trifluormethyl)benzyl]-lH-indol-2-carbonsäureethylester aus Beispiel LXIII in je 1 ml Methanol und THF und gibt dazu 0.26 ml (0.52 mmol) 2M Lithiumhydroxidlösung. Es wird für eine Stunde bei 90°C erhitzt. Nach dem Abkühlen wird das Gemisch zur Aufarbeitung mit wässriger Salzsäure und Ethylacetat verdünnt und extrahiert. Die organische Phase wird mit Natriumsulfat getrocknet, filtriert und im Vakuum getrocknet.
Ausbeute: 89 mg (89 % d. Th.)
HPLC (Methode 4): Rₜ = 4.76 min
MS (ESIpos): m/z = 433 (M+H)⁺

Die Herstellung der folgenden Verbindungen erfolgt analog zu der in Beispiel LXVII beschriebenen Weise:

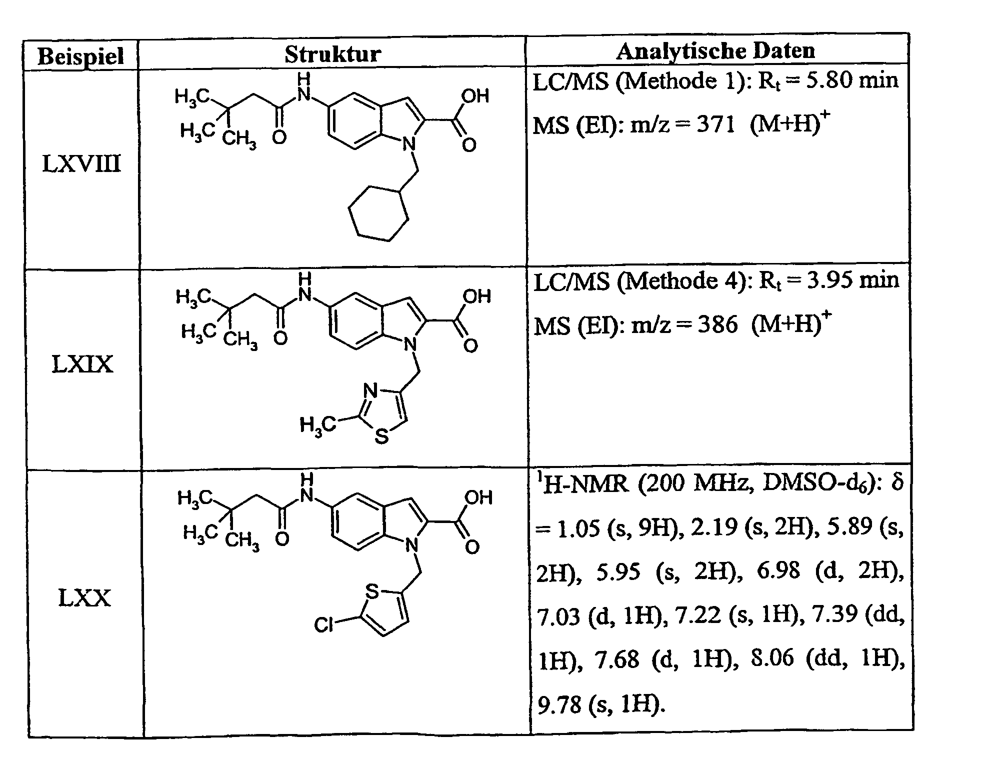

### Beispiel LXXI

### tert-Butyl-4-[({5-[(3,3-dimethylbutanoyl)amino]-1-[2-(trifluormethyl)benzyl]-1H-indol-2-yl}carbonyl)amino]phenylcarbamat

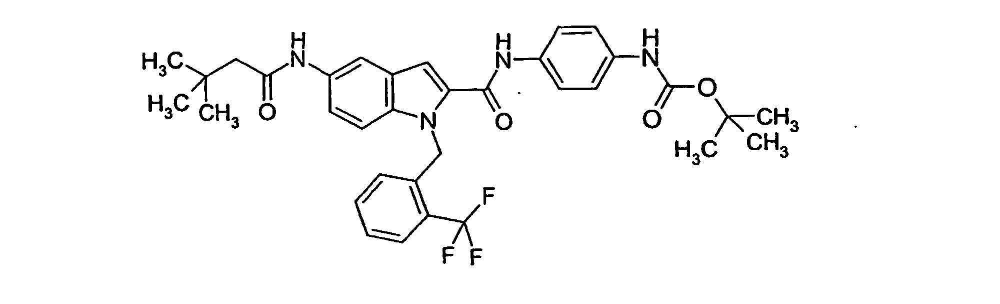

Man legt 125 mg (0.29 mmol) 5-[(3,3-Dimethylbutanoyl)amino]-1-[2-(trifluoromethyl)benzyl]-1H-indol-2-carbonsäure aus Beispiel LXVII, 83 mg (0.43 mmol) N'-(3-Dimethylaminopropyl)-N-ethylcarbodiimid x HCl und 17.7 mg (0.14 mmol) 4-Dimethylaminopyridin in 6 ml Dichlormethan vor. Dazu wird 60 mg (0.29 mmol) tert-Butyl-4-aminophenylcarbamat zugegeben und 4 Stunden bei RT nachgerührt. Das Gemisch wird zur Aufarbeitung mit wässriger Salzsäure und Ethylacetat verdünnt und extrahiert. Die organische Phase wird mit ges. Natriumchloridlösung gewaschen, mit Natriumsulfat getrocknet, filtriert und im Vakuum getrocknet.
Ausbeute: 158 mg (87 % d. Th.)
LC/MS (Methode 1): Rₜ = 5.40 min
MS (EI): m/z = 645 (M+Na)⁺

Die Herstellung der folgenden Verbindungen erfolgt analog zu der in Beispiel LXXI beschriebenen Weise:

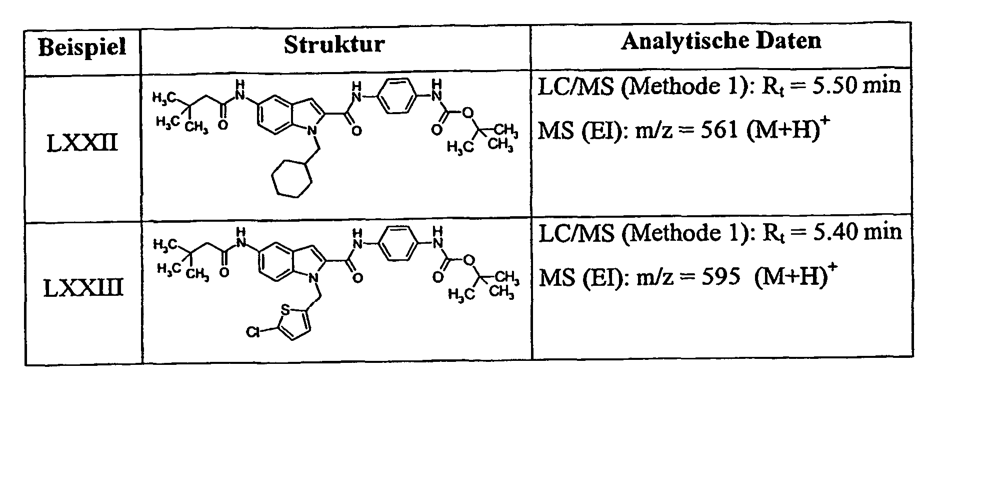

### Herstellungsbeispiele

### Beispiel 1

### 1-(2,6-Difluorbenzyl)-5-[(3,3-dimethylbutanoyl)amino]-N-(3-methylphenyl)-1H-indol-2-carbonsäureamid

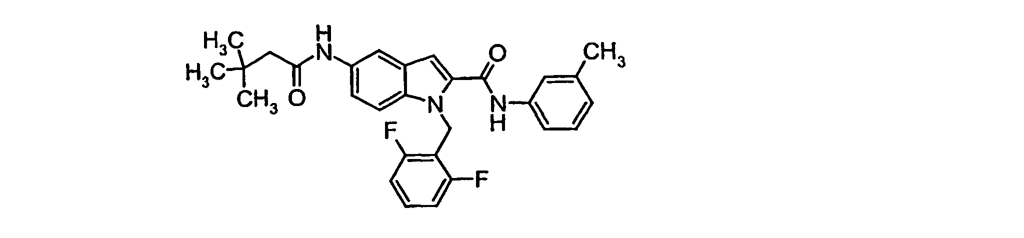

59 mg (0.150 mmol) der Verbindung aus Beispiel XIV und 0.04 ml (0.30 mmol) Triethylamin werden in 5 ml Dichlormethan vorgelegt. Bei 0°C wird eine Lösung von 26 mg 3,3-Dimethylbuttersäurechlorid (0.195 mmol) in 1 ml Dichlormethan zugetropft und der Ansatz 30 min bei RT gerührt. Die Reaktionslösung wird eingeengt und der Rückstand über Kieselgel 60 (Laufmittelgradient Cyclohexan → Cyclohexan:Essigsäureethylester 2.5:1) chromatographisch gereinigt. Das erhaltene Produkt wird in wenig Essigsäureethylester aufgenommen, unter Zugabe von n-Pentan ausgefällt, abgesaugt und getrocknet. Man erhält 35 mg (45 % d. Th.) eines hellbeigen Feststoffes.
MS (ESIpos): m/z = 490 (M+M)⁺.
¹H-NMR (300 MHz, DMSO-d₆): δ = 10.23 (s, 1H), 9.68 (s, 1H), 8.01 (d, 1H), 7.62 (s, 1H), 7.55 (d, 1H),7.45-7.29 (m, 3H), 7.23 (t, 1H), 7.19 (s, 1H), 7.03 (t, 2H), 6.92 (d, 1H), 5.96 (s, 2H), 2.32 (s, 3H), 2.18 (s, 2H), 1.03 (s, 9H).

Die nachfolgenden Beispiele werden in analoger Weise zu Beispiel 1 aus den entsprechenden Edukten hergestellt:

### Beispiel 2

### 1-(2,6-Difluorbenzyl)-5-[(3,3-dimethylbutanoyl)amino]-N-phenyl-1H-indol-2-carbonsäureamid

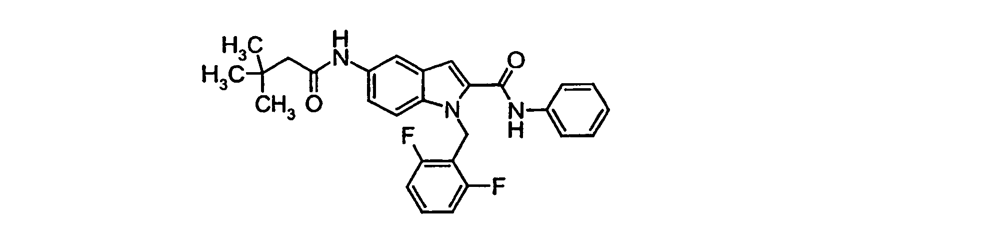

MS (ESIpos): m/z = 476 (M+H)⁺.
¹H-NMR (300 MHz, DMSO-d₆): δ = 10.35 (s, 1H), 9.68, (s, 1H), 8.01 (d, 1H), 7.77 (d, 2H), 7.35 (m, 5H), 7.20 (s, 1H), 7.05 (m, 3H), 2.18 (s, 2H), 1.03 (s, 9H).

### Beispiel 3

### 5-[(3,3-Dimethylbutanoyl)amino]-1-(2-fluorbenzyl)-N-(3-pyridinyl)-1H-indol-2-carbonsäureamid

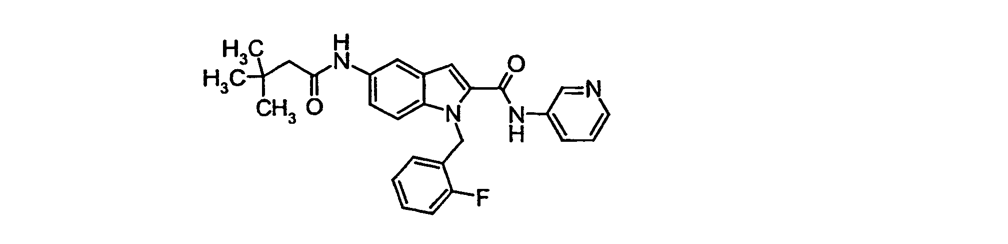

MS (ESIpos): m/z = 459 (M+H)⁺.
¹H-NMR (200 MHz, DMSO-d₆): δ = 10.57 (s, 1H), 9.77 (s, 1H), 8.88 (d, 1H), 8.30 (d, 1H), 8.20-8.09 (m, 2H), 7.53-7.12 (m, 6H), 7.01 (dt, 1H), 6.60 (t, 1H), 5.90 (s, 2H), 2.19 (s, 2H), 1.04 (s, 9H).

### Beispiel 4

### 5-[(3,3-Dimethylbutanoyl)amino]-1-(2-fluorbenzyl)-N-(3-methoxyphenyl)-1H-indol-2-carbonsäureamid

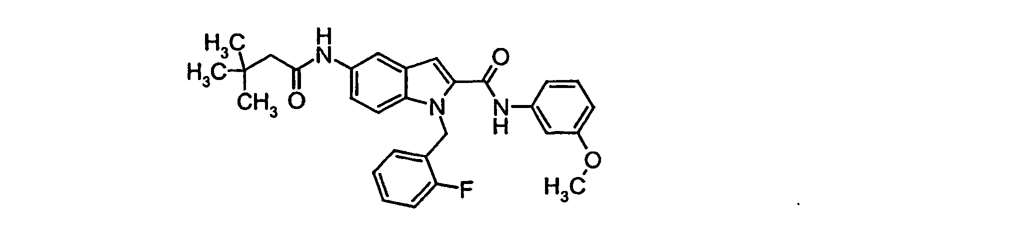

MS (ESIpos): m/z = 488 (M+H)⁺.
¹H-NMR (300 MHz, DMSO-d₆): δ = 10.30 (s, 1H), 9.71 (s, 1H), 8.08 (d, 1H), 7.48-7.14 (m, 8H), 7.00 (dt, 1H), 6.67 (ddd, 1H), 6.61 (dt, 1H), 5.89 (s, 2H), 3.74 (s, 3H), 2.19 (s, 2H), 1.04 (s, 9H).

### Beispiel 5

### 5-[(3,3-Dimethylbutanoyl)amino]-1-(2-fluorbenzyl)-N-(4-methoxyphenyl)-1H-indol-2-carbonsäureamid

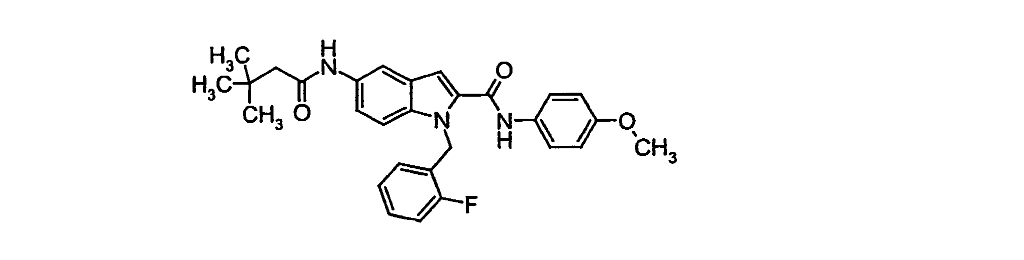

MS (ESIpos): m/z = 488 (M+H)⁺.
¹H-NMR (300 MHz, DMSO-d₆): δ = 10.21 (s, 1H), 9.70 (s, 1H), 8.07 (d, 1H), 7.61 (m, 2H), 7.43 (d, 1H), 7.33 (m, 2H), 7.21 (m, 2H), 7.00 (dt, 1H), 6.90 (m, 2H), 6.60 (dt, 1H), 5.89 (s, 2H), 3.74 (s, 3H), 2.19 (s, 2H), 1.04 (s, 9H).

### Beispiel 6

### 5-[(3,3-Dimethylbutanoyl)amino]-1-(2-fluorbenzyl)-N-(3-methylphenyl)-1H-indol-2-carbonsäureamid

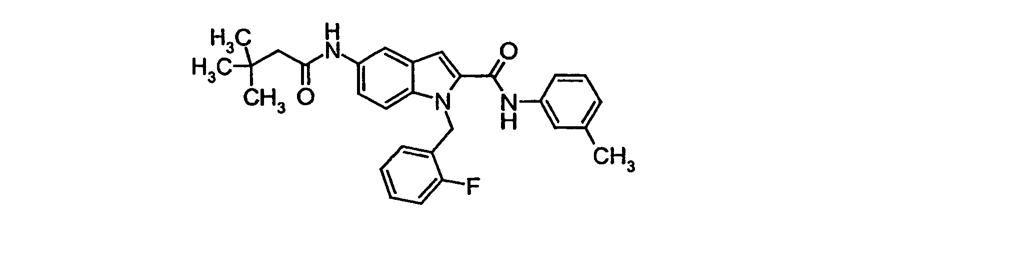

MS (ESIpos): m/z = 472 (M+H)⁺.
¹H-NMR (300 MHz, DMSO-d₆): δ = 10.24 (s, 1H), 9.70 (s, 1H), 8.08 (d, 1H), 7.58 (m, 1H), 7.50 (d, 1H), 7.44 (d, 1H), 7.38-7.14 (m, 5H), 7.00 (dt, 1H), 6.91 (d, 1H), 6.60 (dt, 1H), 5.89 (s, 2H), 2.30 (s, 3H), 2.19 (s, 2H), 1.04 (s, 9H).

### Beispiel 7

### 5-[(3,3-Dimethylbutanoyl)amino]-1-(2-fluorbenzyl)-N-phenyl-1H-indol-2-carbonsäureamid

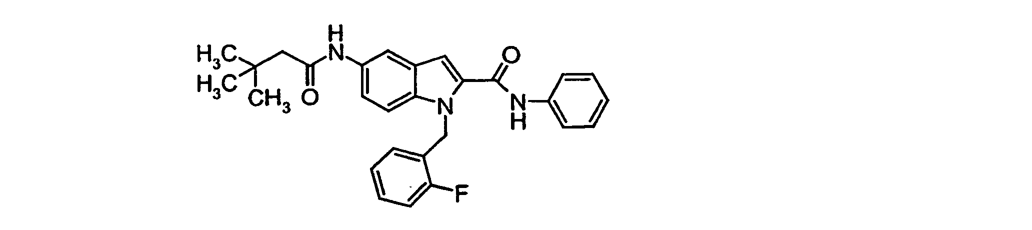

MS (ESIpos): m/z = 458 (M+H)⁺.
¹H-NMR (300 MHz, DMSO-d₆): δ = 10.31 (s, 1H), 9.71 (s, 1H), 8.08 (s, 1H), 7.72 (d, 2H), 7.47-7.14 (m, 7H), 7.09 (t, 1H), 7.00 (t, 1H), 6.61 (t, 1H), 5.90 (s, 2H), 2.19 (s, 2H), 1.04 (s, 9H).

### Beispiel 8

### 5-[(Bicyclo[2.2.1]hept-2-ylacetyl)amino]-N-phenyl-1-propyl-1H-indol-2-carbonsäureamid

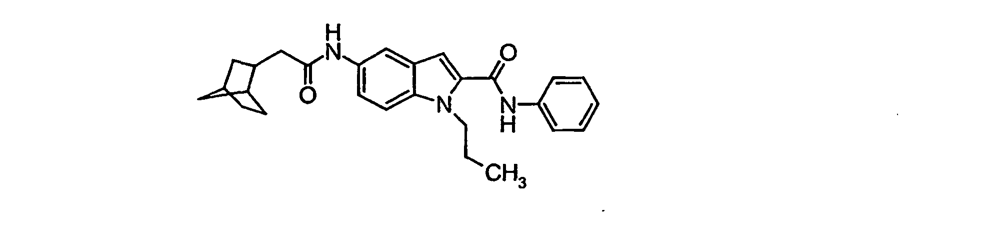

MS (ESIpos): m/z = 430 (M+H)⁺.
¹H-NMR (300 MHz, DMSO-d₆): δ = 10.25 (s, 1H), 9.75 (s, 1H), 8.03 (s, 1H), 7.77 (d, 2H), 7.52 (d, 1H),7.42-7.30 (m, 3H), 7.23 (s, 1H), 7.10 (dd, 1H), 4.50 (t, 2H), 2.40-2.09 (m, 5H), 1.84.-1.06 (m, 11H), 0.81 (t, 3H), 0.73 (m, 1H).

### Beispiel 9

### 5-[(Cyclohexylcarbonyl)amino]-1-(2-fluorobenzyl)-N-phenyl-1H-indol-2-carbonsäureamid

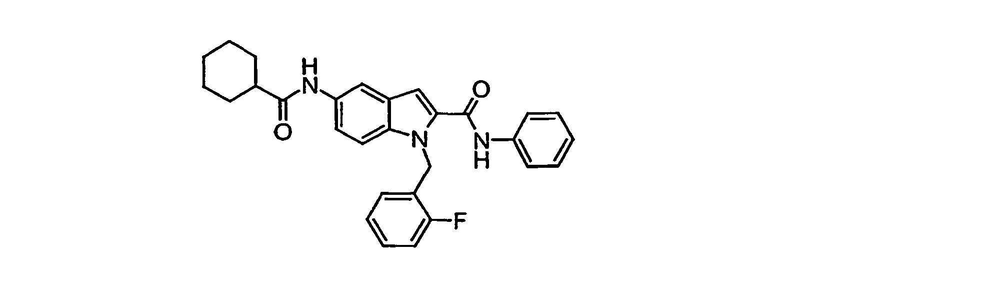

MS (ESIpos): m/z = 470 (M+H)⁺.
¹H-NMR (300 MHz, DMSO-d₆): δ = 10.32 (s, 1H), 9.71 (s, 1H), 8.11 (d, 1H), 7.72 (d, 2H),
7.47-7.13 (m, 7H), 7.08 (t, 1H), 6.98 (dt, 1H), 6.59 (dt, 1H), 5.90 (s, 2H), 2.33 (m, 1H), 1.88-1.60 (m, 5H), 1.52-1.15 (m, 5H).

Die in der folgenden Tabelle aufgeführten Beispiele können analog den oben beschriebenen Vorschriften aus den entsprechenden Ausgangsverbindungen hergestellt werden.

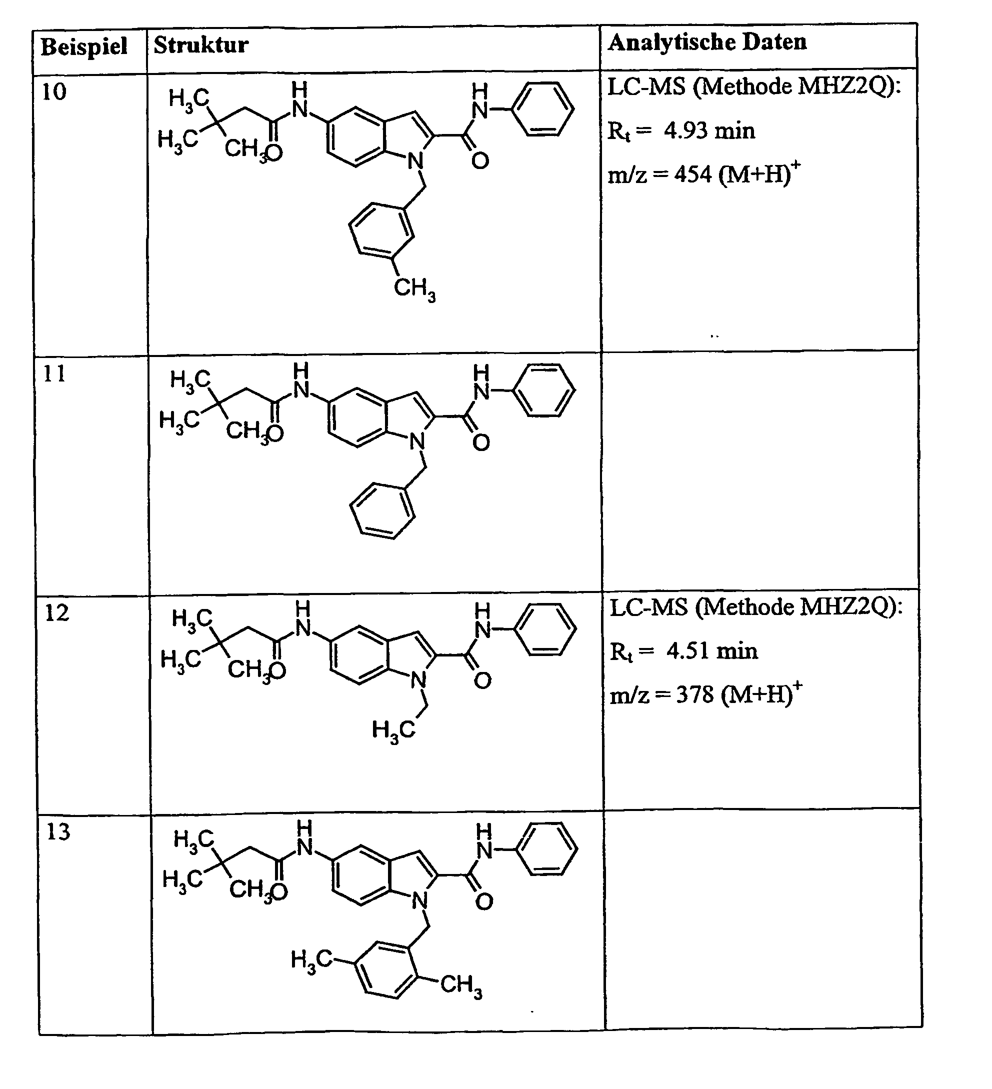

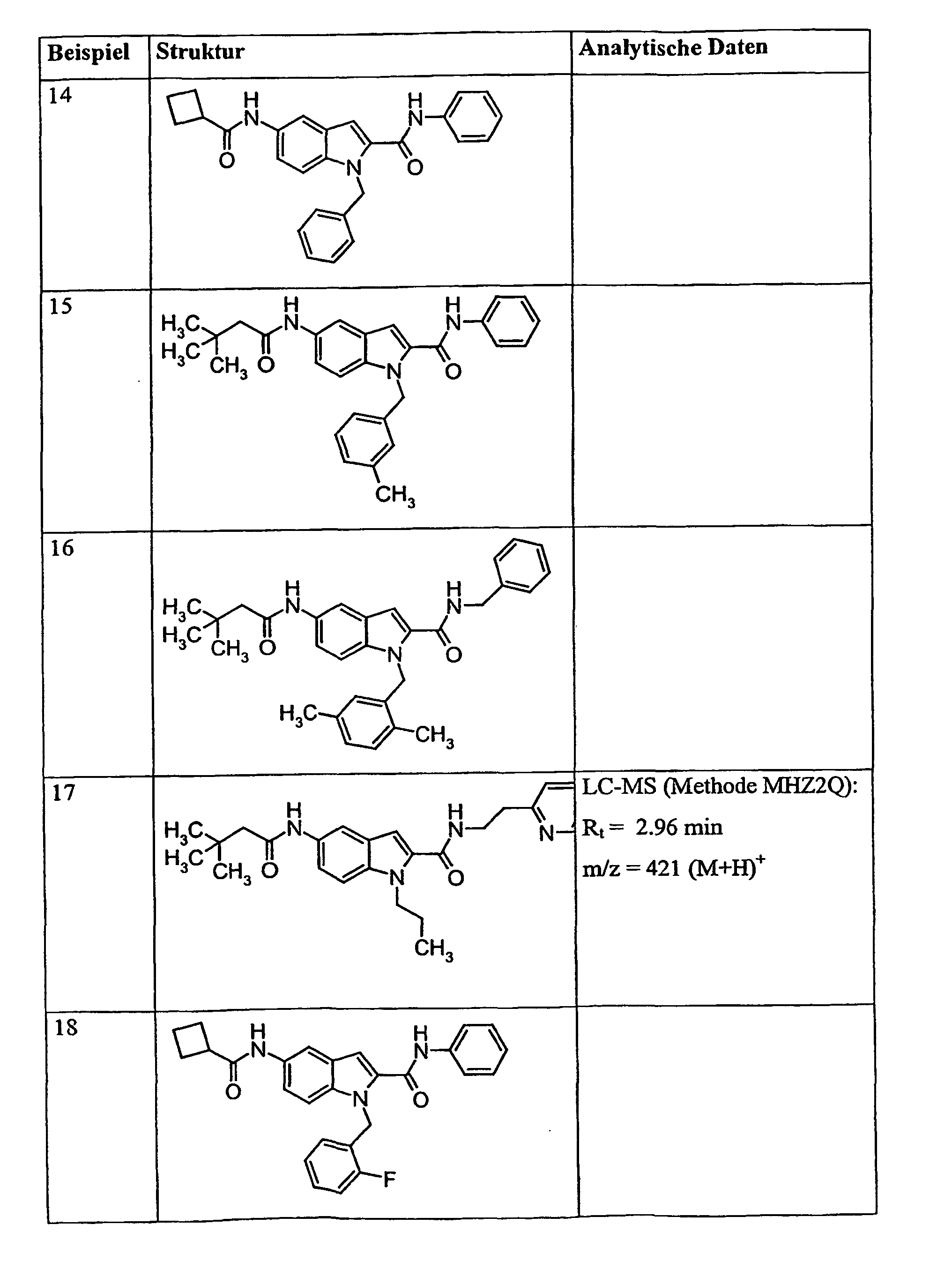

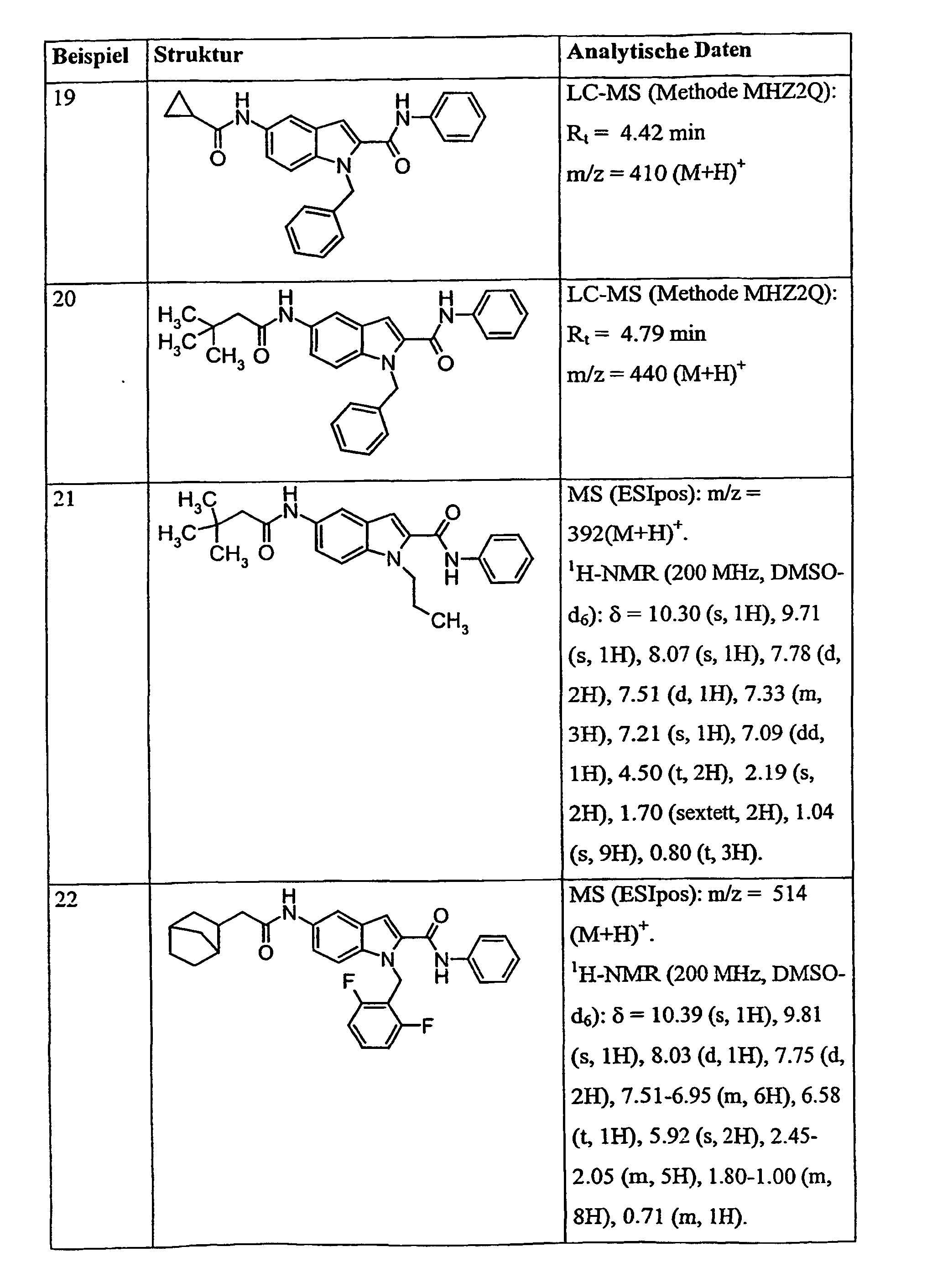

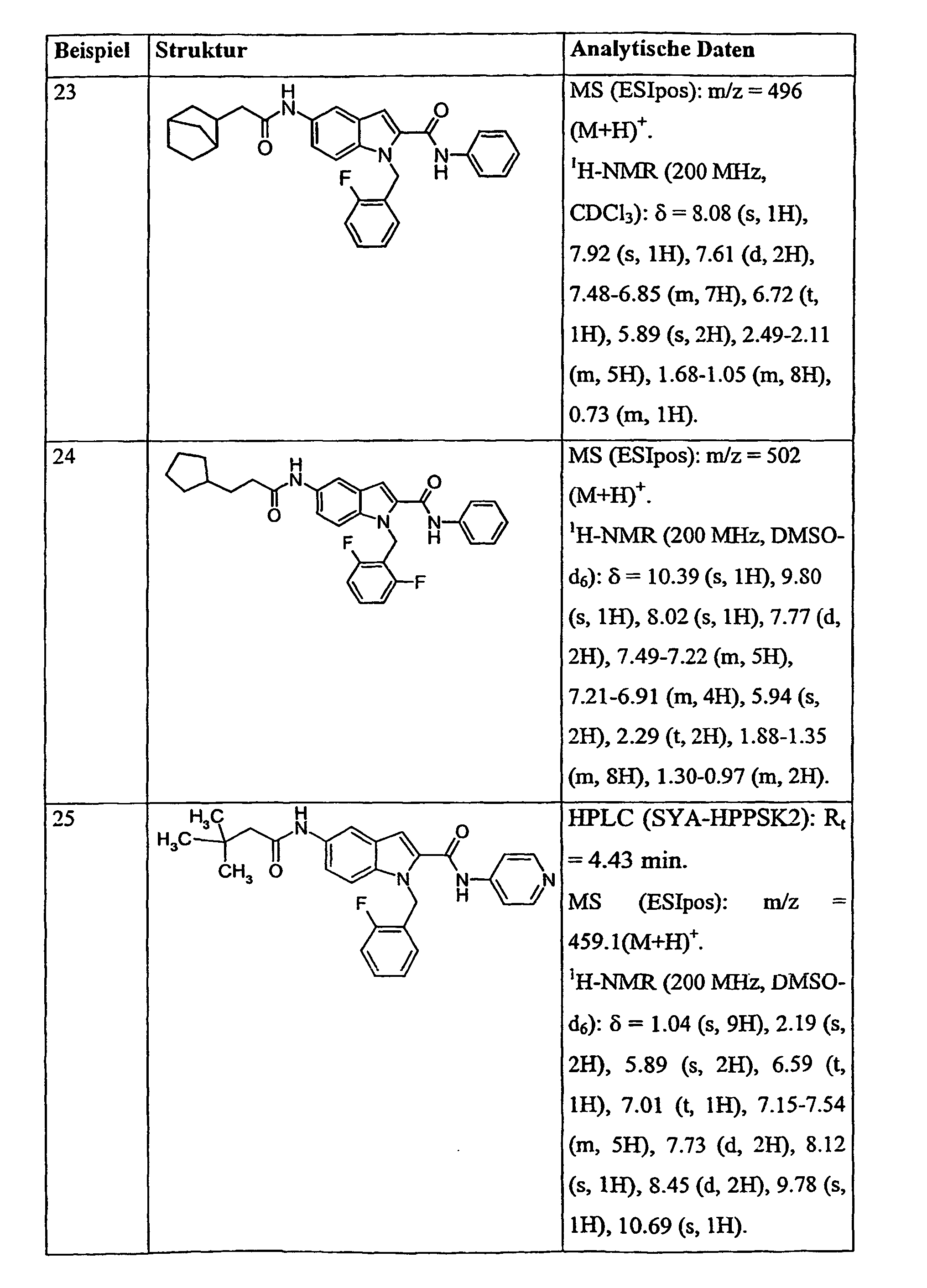

### Beispiel 26

### 1-(2-Fluorbenzyl)-5- {[(1-methylcyclopentyl)acetyl]amino}-N-phenyl-1H-indol-2-carboxamid

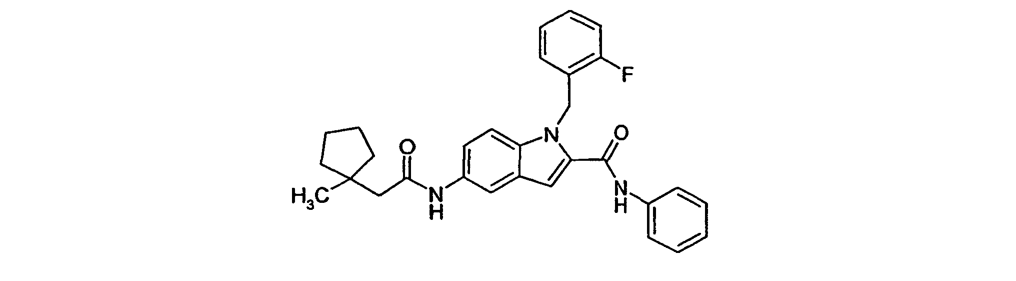

Man legt 62 mg (0.20 mmol) der Verbindung aus Beispiel XXVI, 58 mg (0.30 mmol) N'-(3-Dimethylaminopropyl)-N-ethylearbodiimid x HCl und 12 mg (0.1 mmol) 4-Dimethylaminopyridin in DMF vor. Dazu wird 34 mg (0.24 mmol) (1-Methylcyclopentyl)essigsäure (synthetisiert nach K. Bott, *Chem. Ber.* **1967**, *100,* 978-983) zugegeben und 5 h bei RT nachgerührt. Das Gemisch wird zur Aufarbeitung mit wässriger Salzsäure und Dichlormethan verdünnt und extrahiert. Die organische Phase wird mit ges. Natriumchloridlösung gewaschen, mit Natriumsulfat getrocknet, filtriert und im Vakuum getrocknet. Reinigung erfolgt durch Flashchromatographie an Kieselgel.
Ausbeute: 56 mg (57 % d. Th.)
LC/MS (SMKL-ZQ-2A): Rₜ = 4.15 min.
MS (ESIpos): m/z = 484.1 (M+H)⁺.
¹H-NMR (200 MHz, DMSO-d₆): δ = 1.05 (s, 3H), 1.63 (s, 8H), 2.29 (s, 2H), 5.90 (s, 2H), 6.60 (t, 1H), 6.91-7.39 (m, 9H), 7.44 (d, 1H), 7.72 (d, 2H), 8.10 (s, 1H), 9.79 (s, 1H), 10.37 (s, 1H).

Die Herstellung der folgenden Verbindungen erfolgt analog zu der in Beispiel 26 beschriebenen Weise aus den entsprechenden Ausgangsmaterialien:

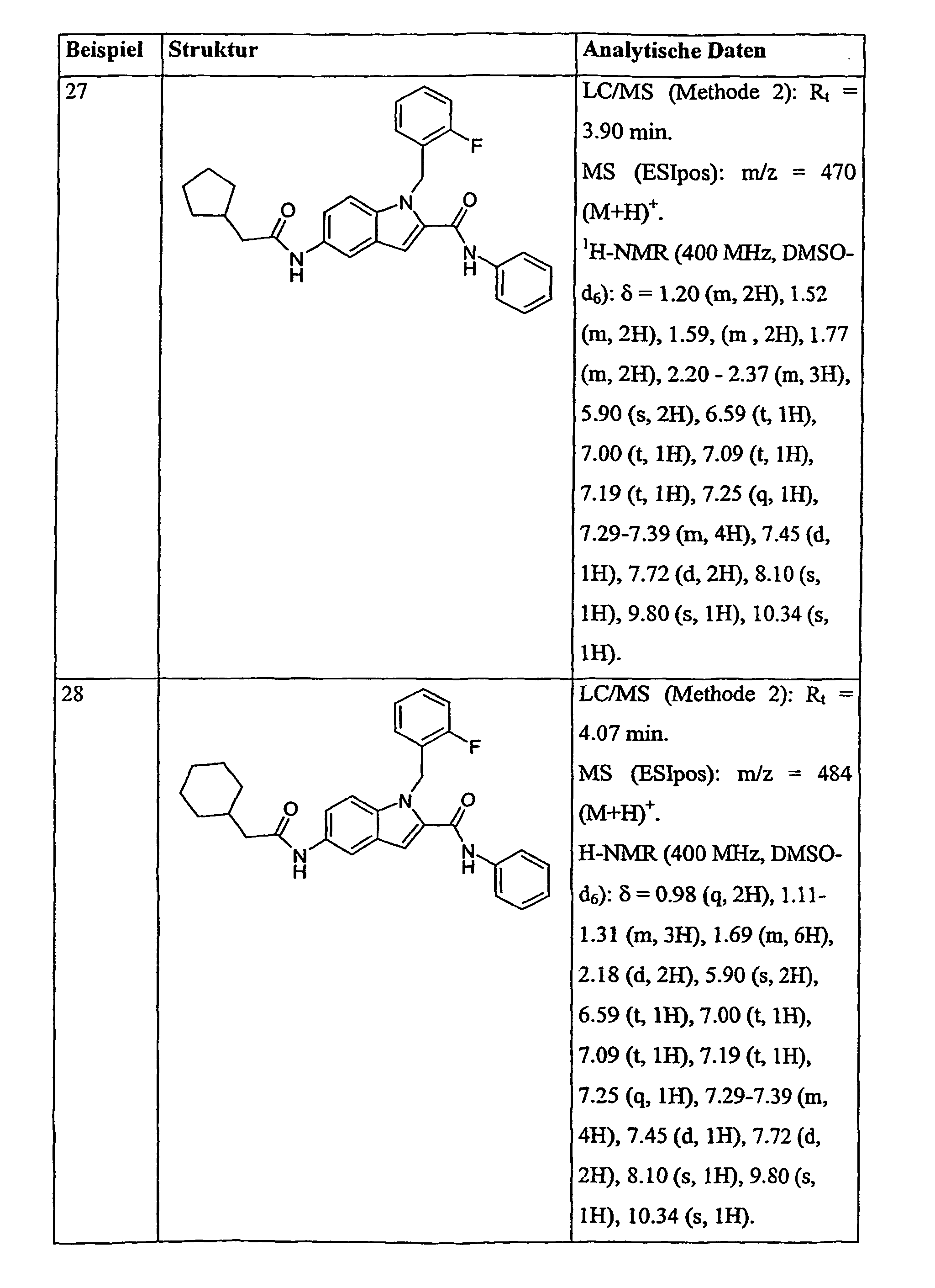

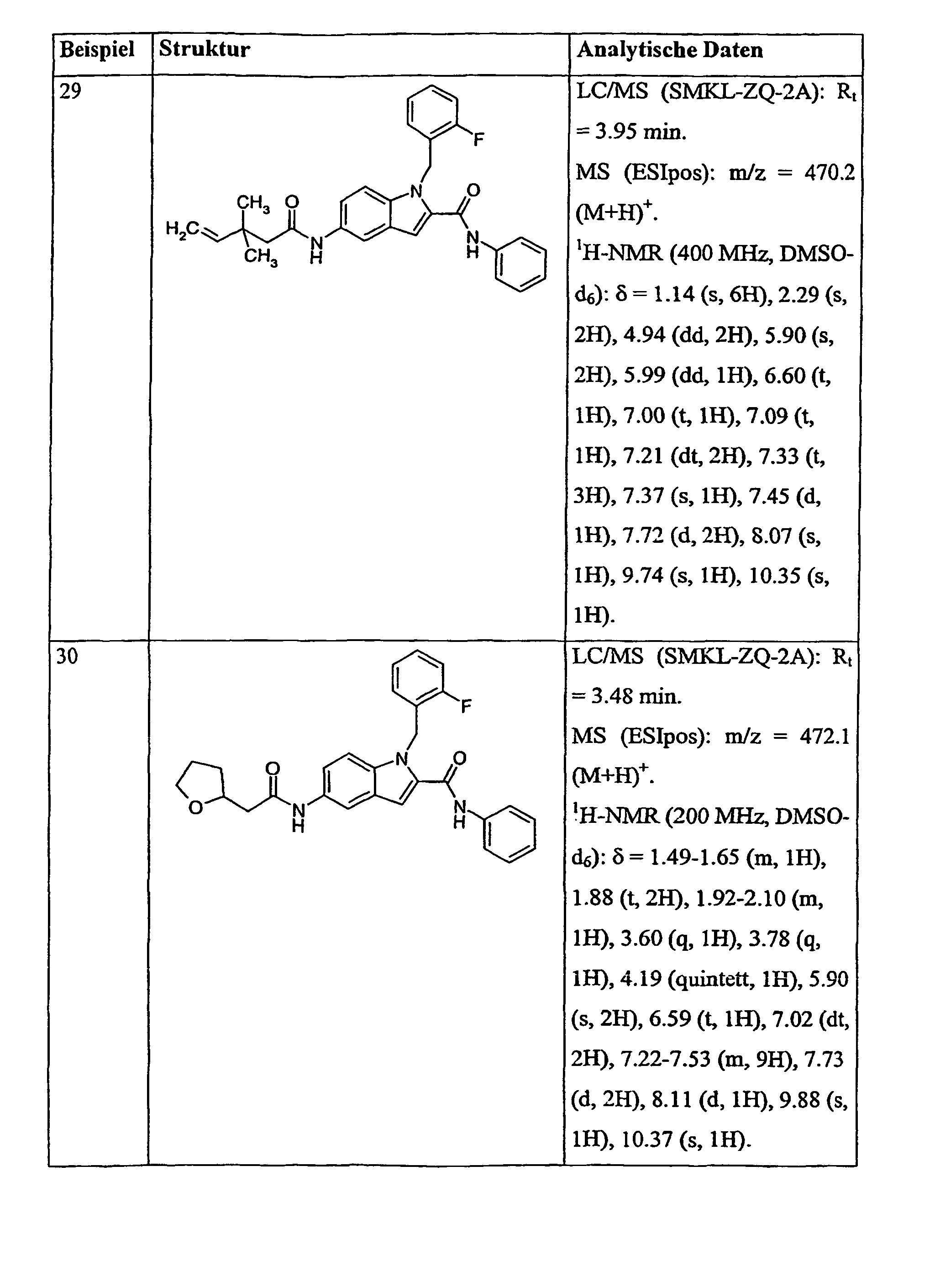

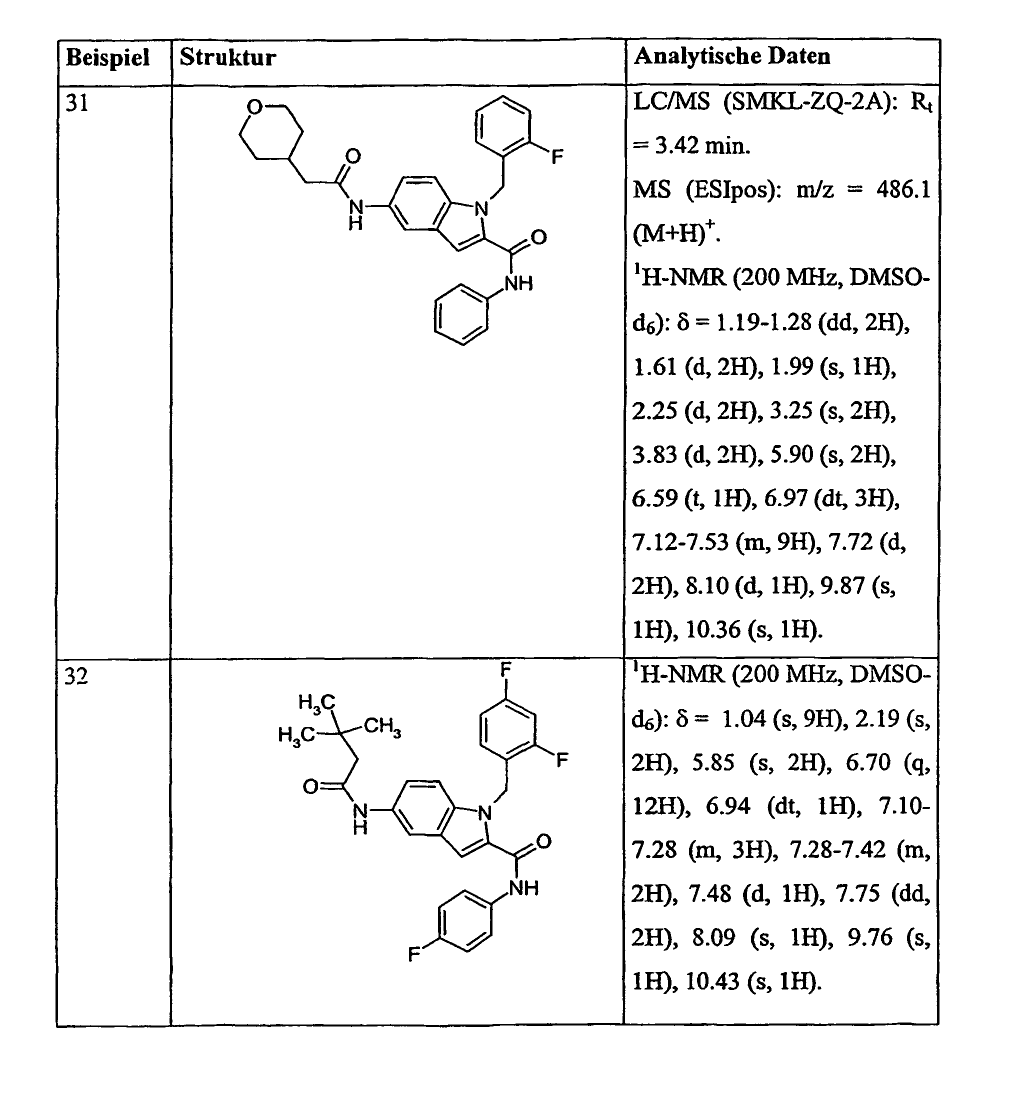

### Beispiel 33

### 1-(2-Fluorbenzyl)-5-[(5-hydroxy-3,3-dimethylpentyl)amino]-N-phenyl-1H-indol-2-carboxamid

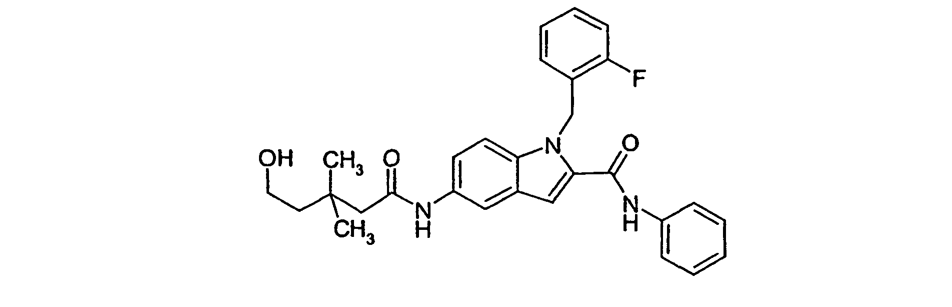

60 mg (0.13 mmol) der Verbindung aus Beispiel 29 werden in 2 ml THF gelöst und auf 0°C gekühlt. Zu dieser Lösung gibt man portionsweise innerhalb von 3.5 h Stunden verteilt insgesamt 0.93 ml (0.46 mmol) einer 0.5 molaren 9-Borabicyclo(3.3.1)nonan-Lösung in THF und lässt die Temperatur dabei auf RT ansteigen. Man rührt das Reaktionsgemisch noch eine weitere Stunde bei RT und versetzt es bei 0°C langsam mit jeweils 0.5 ml Natriumcarbonat-Lösung und Wasserstoffperoxid-Lösung. Nachdem die exotherme Reaktion beendet ist, rührt man für 30 min. bei RT nach. Anschließend verdünnt man das Reaktionsgemisch mit Ethylacetat und extrahiert mit dest. Wasser und gesättigter Natriumchlorid-Lösung. Die organische Phase wird über Natriumsulfat getrocknet, filtriert und das Lösungsmittel im Vakuum entfernt. Der Rückstand wird chromatographisch über Kieselgel aufgereinigt (Eluent: Cyclohexan / Ethylacetat 5:1 bis 1:1). Man erhält 53 mg (85 % d. Th.) des Produkts.
LC/MS (MHZ2P01): Rₜ = 4.63 min.
MS (ESIpos): m/z = 488.2 (M+H)⁺.

Die Herstellung der folgenden Verbindungen erfolgt analog zu der in Beispiel XXXV beschriebenen Weise:

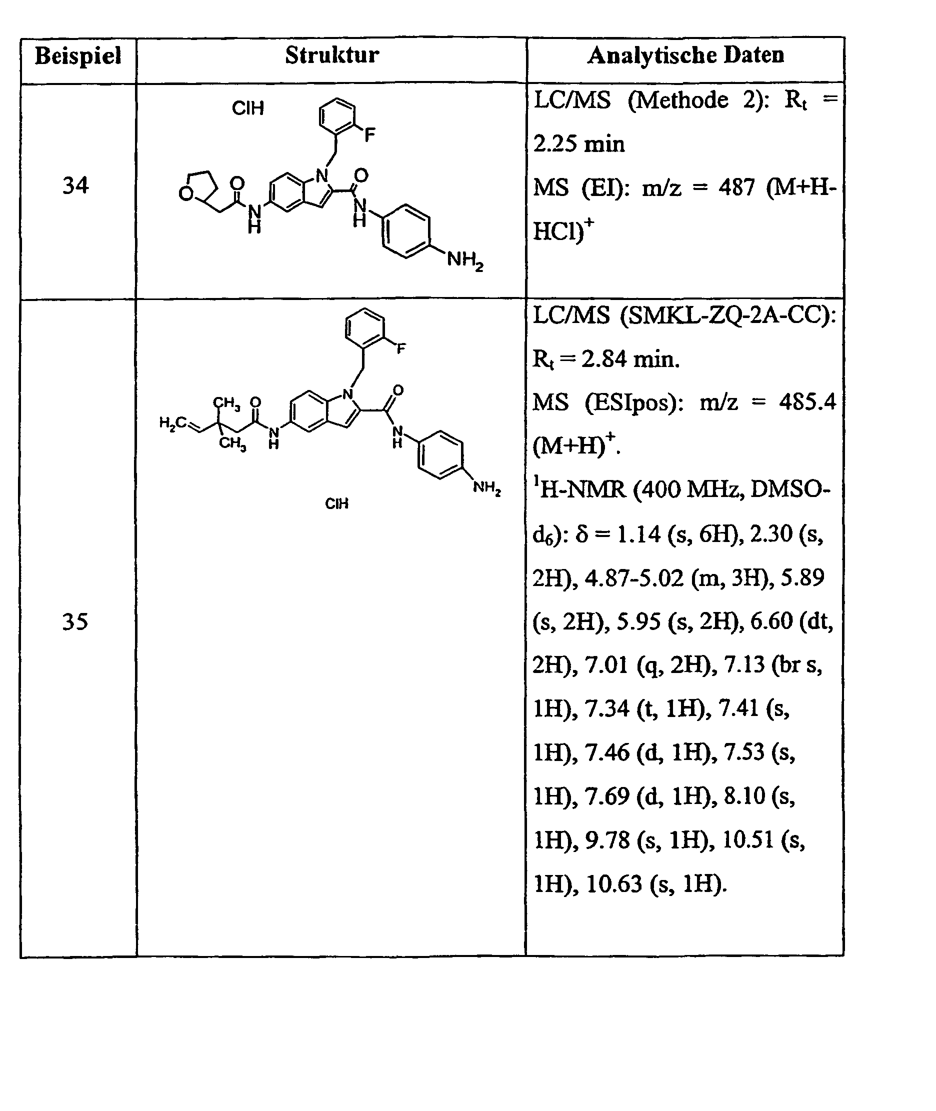

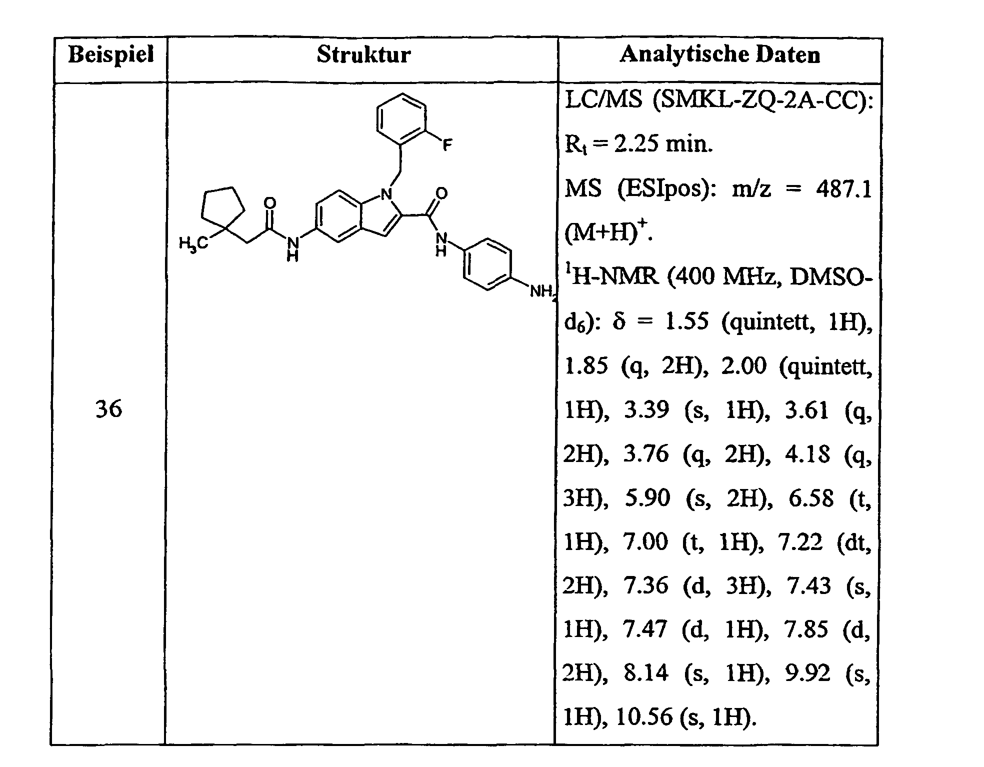

### Beispiel 37

### N-[4-(Acetylamino)phenyl]-1-(2-fluorbenzyl)-5-{[(1-ethylcyclopentyl)acetyl]amino}-1H-indol-2-carboxamid

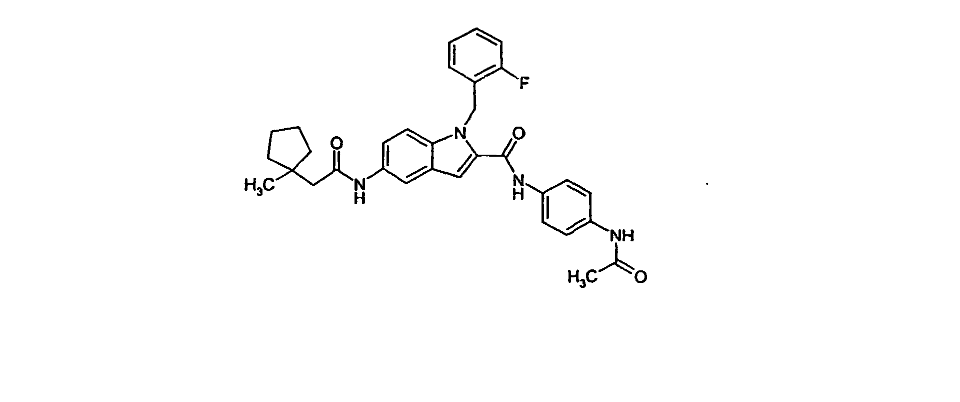

Man legt 79 mg (0.15 mol) der Verbindung aus Beispiel 36 und 30 mg (0.29 mmol) Triethylamin in 3 ml Dichlormethan vor und kühlt auf 0°C ab. Anschließend gibt man 11.5 mg (0.15 mmol) Acetylchlorid zu und rührt über Nacht bei RT. Man verdünnt mit 10 ml Dichlormethan und wäscht nacheinander mit 1 N Salzsäure, wässriger Natriumhydrogencarbonat-Lösung und Wasser, trocknet über Natriumsulfat und entfernt das Lösungsmittel unter vermindertem Druck. Der Rückstand wird mit Diisopropylether verrührt und durch Filtration isoliert und getrocknet.
Ausbeute: 68 mg (63 % d. Th.)
LC/MS (MHZ2P01): Rₜ = 4.82 min.
MS (ESIpos): m/z = 541.3 (M+H)⁺.
¹H-NMR (200 MHz, DMSO-d₆): δ = 1.07 (s, 3H), 1.63 (s, 8H), 2.02 (s, 3H), 2.29 (s, 2H), 5.90 (s, 2H), 6.57 (t, 1H), 7.00 (t, 1H), 7.13-7.72 (m, 7H), 8.09 (s, 1H), 9.77 (s, 1H), 9.91 (s, 1H), 10.31 (s, 1H)

Die Herstellung der folgenden Verbindungen erfolgt analog zu der in Beispiel 37 beschriebenen Weise:

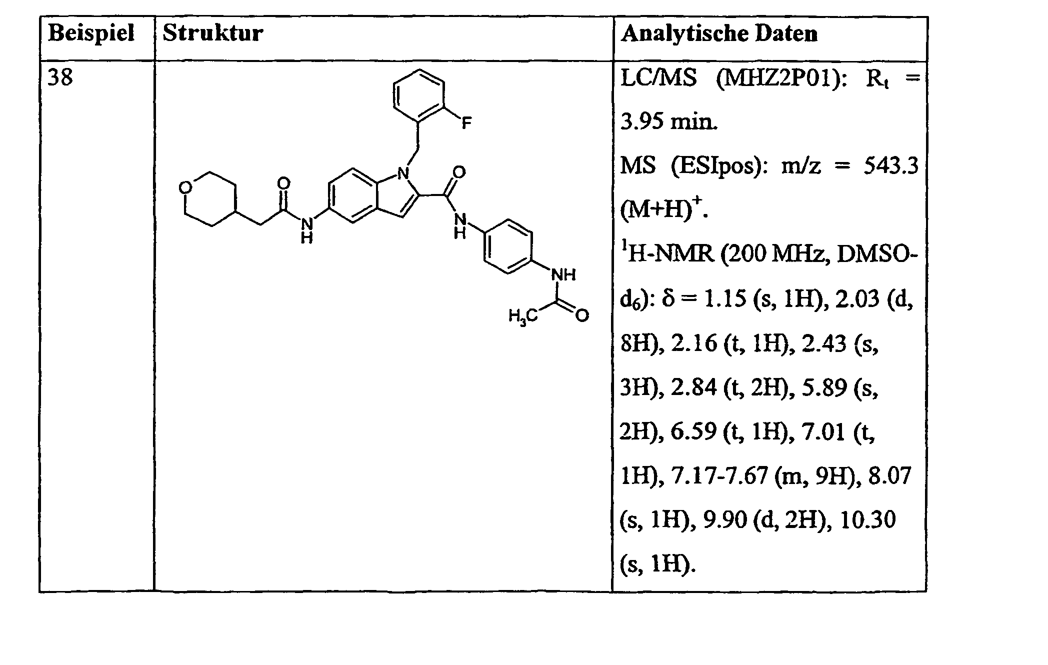

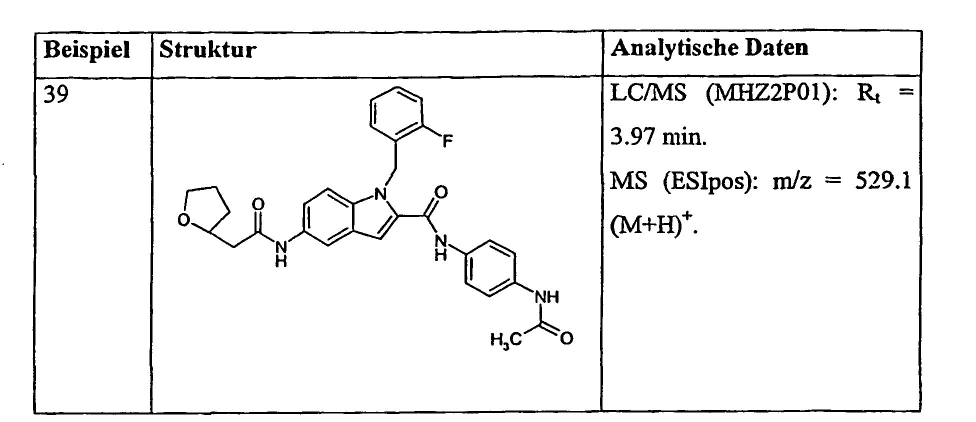

### Beispiel 40

### 5-[(4,4-Dimethylpentanoyl)amino]-1-(2-fluorbenzyl)-N-phenyl-1H-indol-2-carboxamid

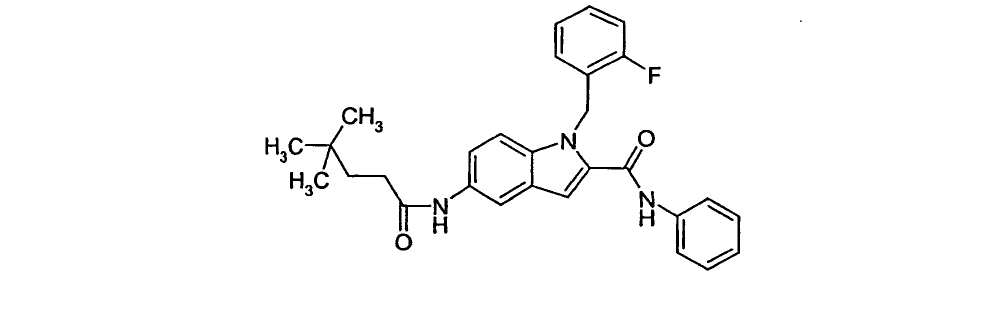

Eine Lösung aus 300 mg (0.83 mmol) der Verbindung aus Beispiel XVI, 51 mg (0.42 mmol) 4-Dimethylaminopyridin und 240 mg (1.25 mmol) N'-(3-Dimethylaminopropyl)-N-ethylcarbodiimid x HCl in 10 ml DMF wird vorgelegt. Dazu gibt man 126 mg (1.0 mmol) 4,4-Dimethyl-2-pentinsäure (hergestellt nach *J. Chem*. *Soc. Perkin II* **1990**, 1997ff.) und rührt über Nacht bei RT. Zur Aufarbeitung wird mit Dichlormethan und wässriger Salzsäure verdünnt und extrahiert. Die organische Phase wird mit ges. Natriumhydrogencarbonatlösung gewaschen, mit Natriumsulfat getrocknet, filtriert und im Vakuum einrotiert. Der Rückstand wird über eine präparative HPLC gereinigt. Man erhält 258 mg eines weißen Feststoffs (53 % d. Th.) von dem 100 mg (0.21 mmol) in 5 ml Ethanol gelöst und in Gegenwart von 50 mg 10 % Pd/Aktivkohle 3 h bei Normaldruck hydriert werden. Anschließend filtriert man die Lösung über Celite und wäscht den Filterkuchen gut mit Ethylacetat/Ethanol nach. Das Lösungsmittel wird im Vakuum entfernt.
Ausbeute: 101 mg (99 % d. Th.)
¹H-NMR (200 MHz,DMSO-d₆): δ = 0.91 (s, 9H), 1.53 (m, 2H), 2.29 (m, 2H), 5.90 (s, 2H), 6.59 (t, 1H), 7.06 (dt, 2H), 7.13-7.40 (m, 6H), 7.46 (d, 1H), 7.73 (d, 2H), 8.10 (s, 1H), 9.86 (s, 1H), 10.35 (s, 1H).

### Beispiel 41

### N-{4-[(Dimethylamino)carbonyl]phenyl}-5-[(3,3-dimethylbutanoyl)amino]-1-(2-fluorbenzyl)-1H-indol-2-carboxamid

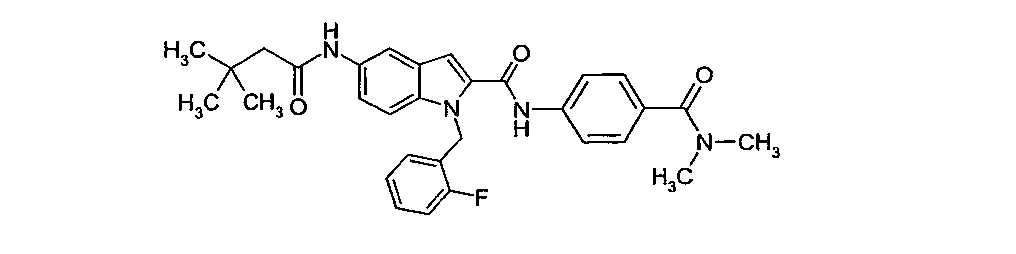

200 mg (0.43 mmol) 5-[(3,3-Dimethylbutanoyl)amino]-1-(2-fluorbenzyl)-1H-indol-2-carbonsäure (Beispiel XXXIX) werden unter Argon in 2 ml DMF gelöst und mit 4 ml Pyridin versetzt. Zu dieser Lösung gibt man 489.2 mg (1.29 mmol) HATU zu und tropft anschließend langsam 140.8 mg (0.86 mmol) 4-Amino-N,N-dimethylbenzamid zu und rührt das Reaktionsgemisch bei RT über Nacht. Zur Aufarbeitung versetzt man mit Wasser und extrahiert mehrmals mit Ethylacetat. Die vereinigten organischen Phasen werden über Natriumsulfat getrocknet, filtriert und das Lösungsmittel im Vakuum entfernt. Der Rückstand wird mittels präparativer HPLC aufgereinigt. Man erhält 47.4 mg (15 % d. Th.) des Produkts.
HPLC (SYA-HPPSK2): Rₜ = 4.69 min.
MS (ESIpos): m/z = 529 (M+H)⁺.
¹H-NMR (400 MHz, DMSO-d₆): δ = 1.04 (s, 9H), 2.19 (s, 2H), 2.96 (s, 6H), 5.90 (s, 2H), 6.60 (t, 1H), 7.19 (t, 1H), 7.25 (q, 1H), 7.27-7.50 (m, 3H), 7.79 (d, 2H), 7.81 (s, 1H), 8.10 (s, 1H), 9.75 (s, 1H), 10.51 (d, 1H).

Die Herstellung der folgenden Verbindungen erfolgt analog zu der in Beispiel 26 und 41 beschriebenen Weise aus den entsprechenden Ausgangsmaterialien:

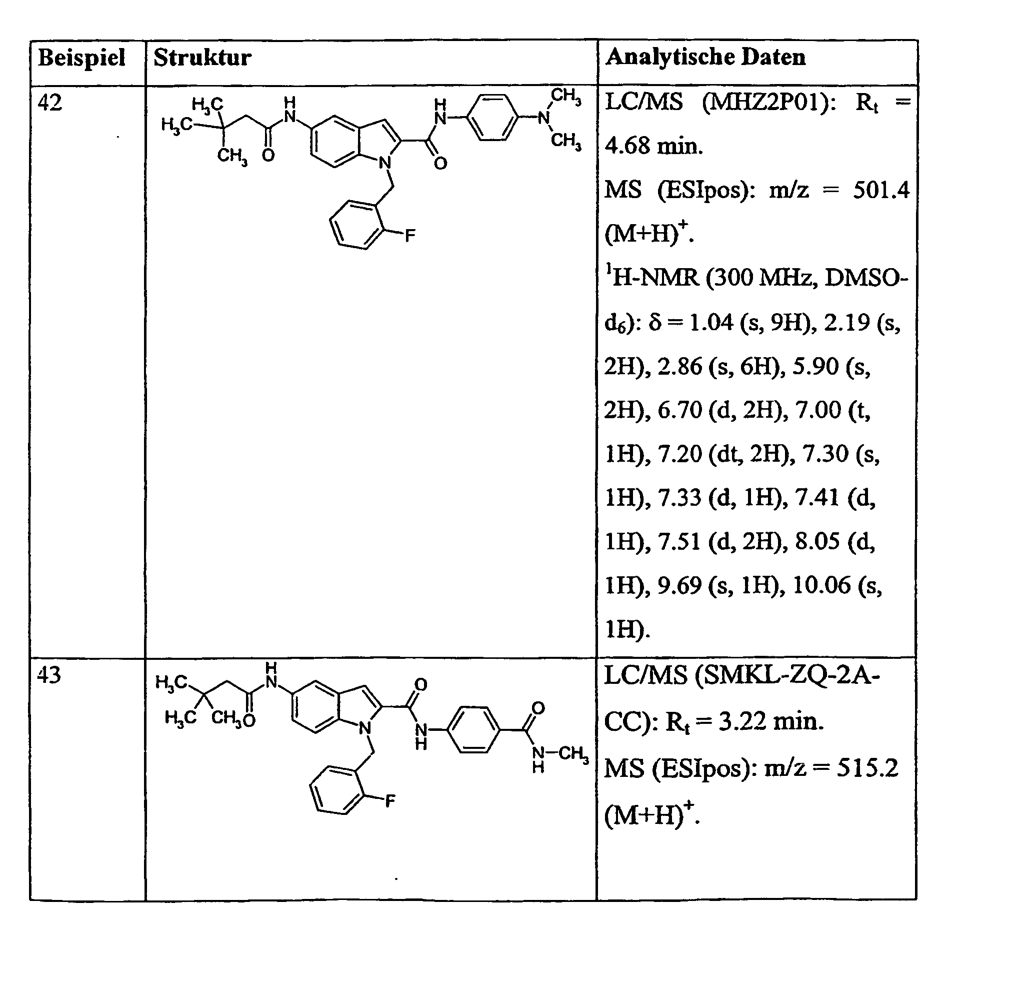

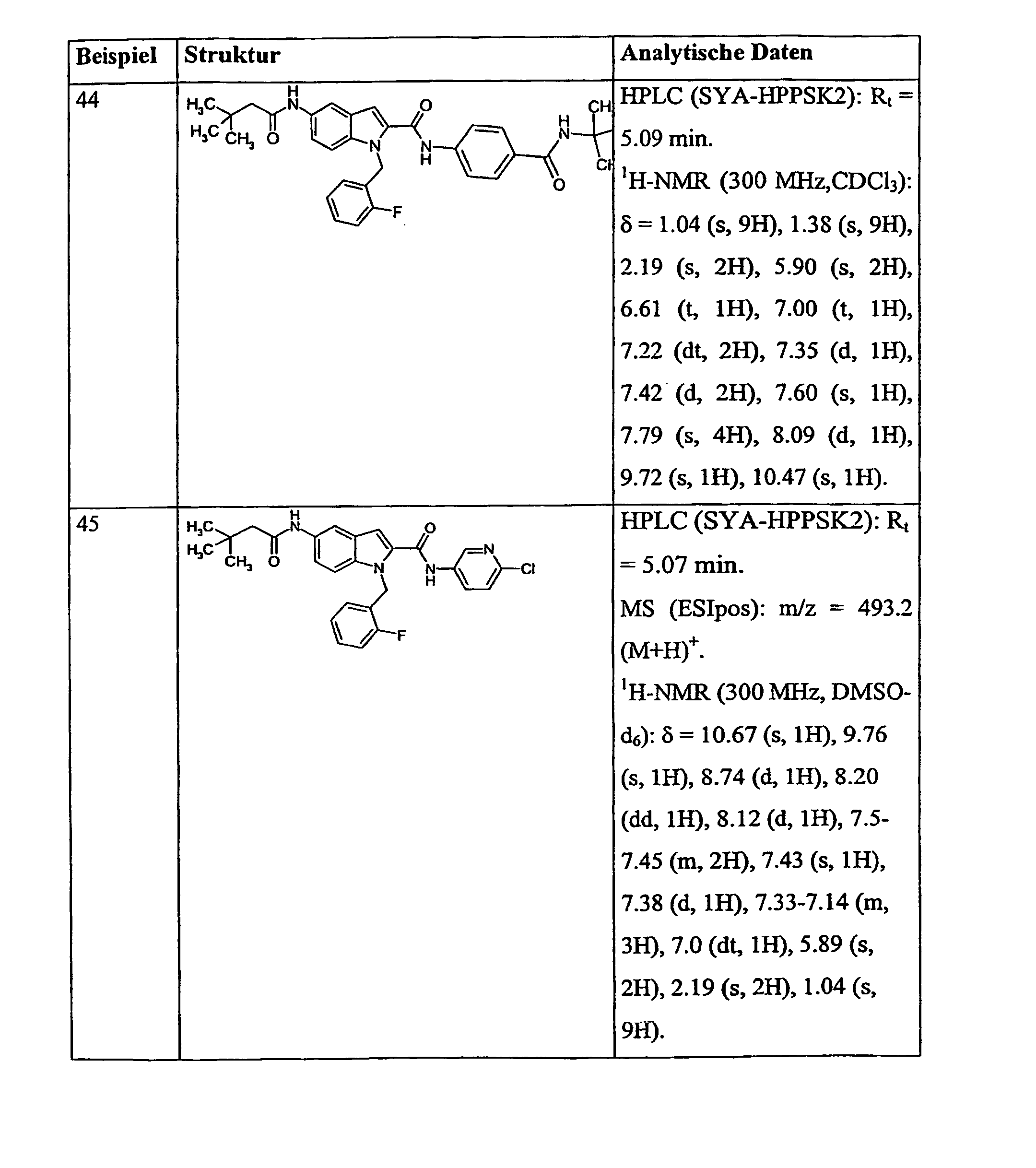

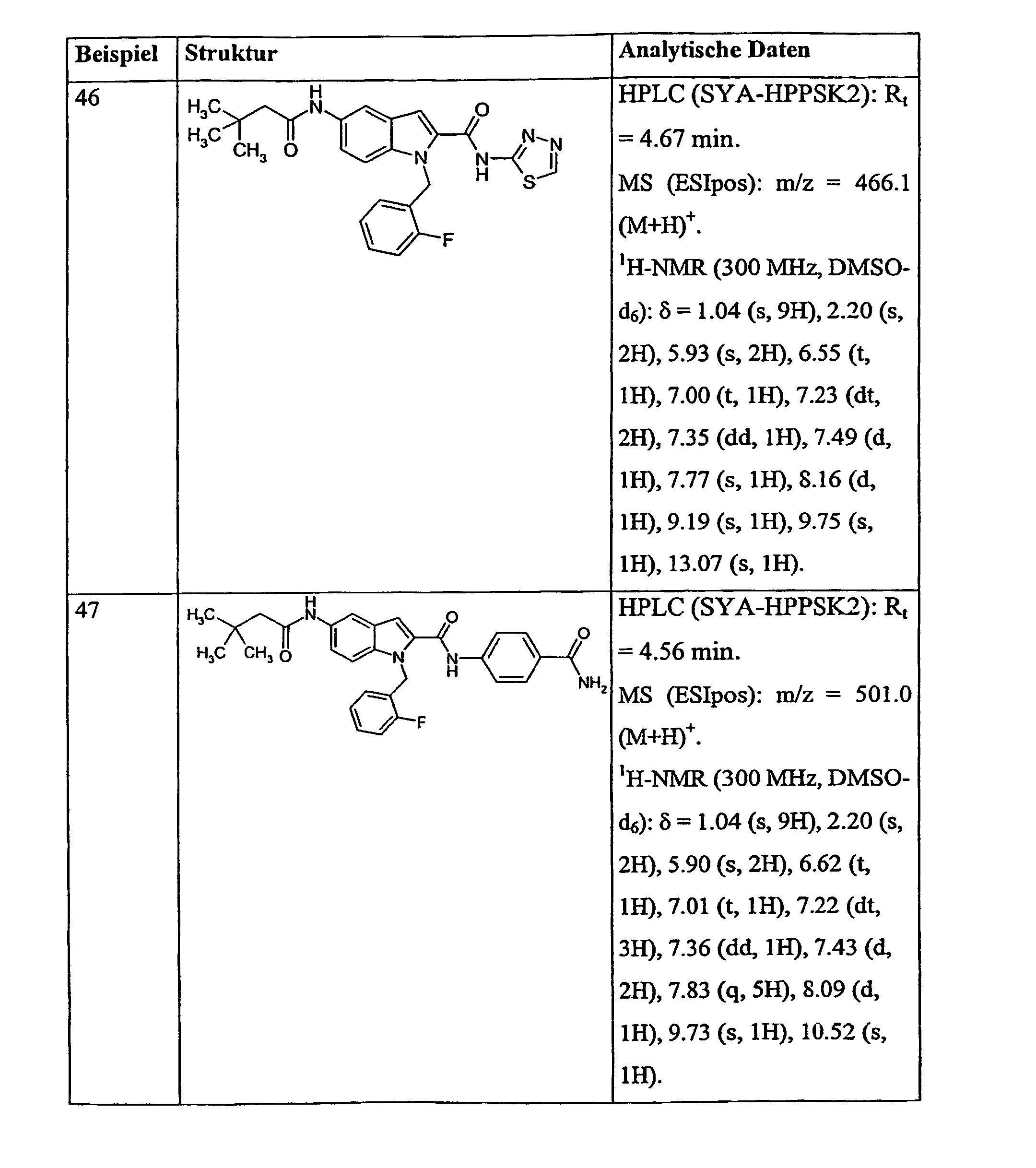

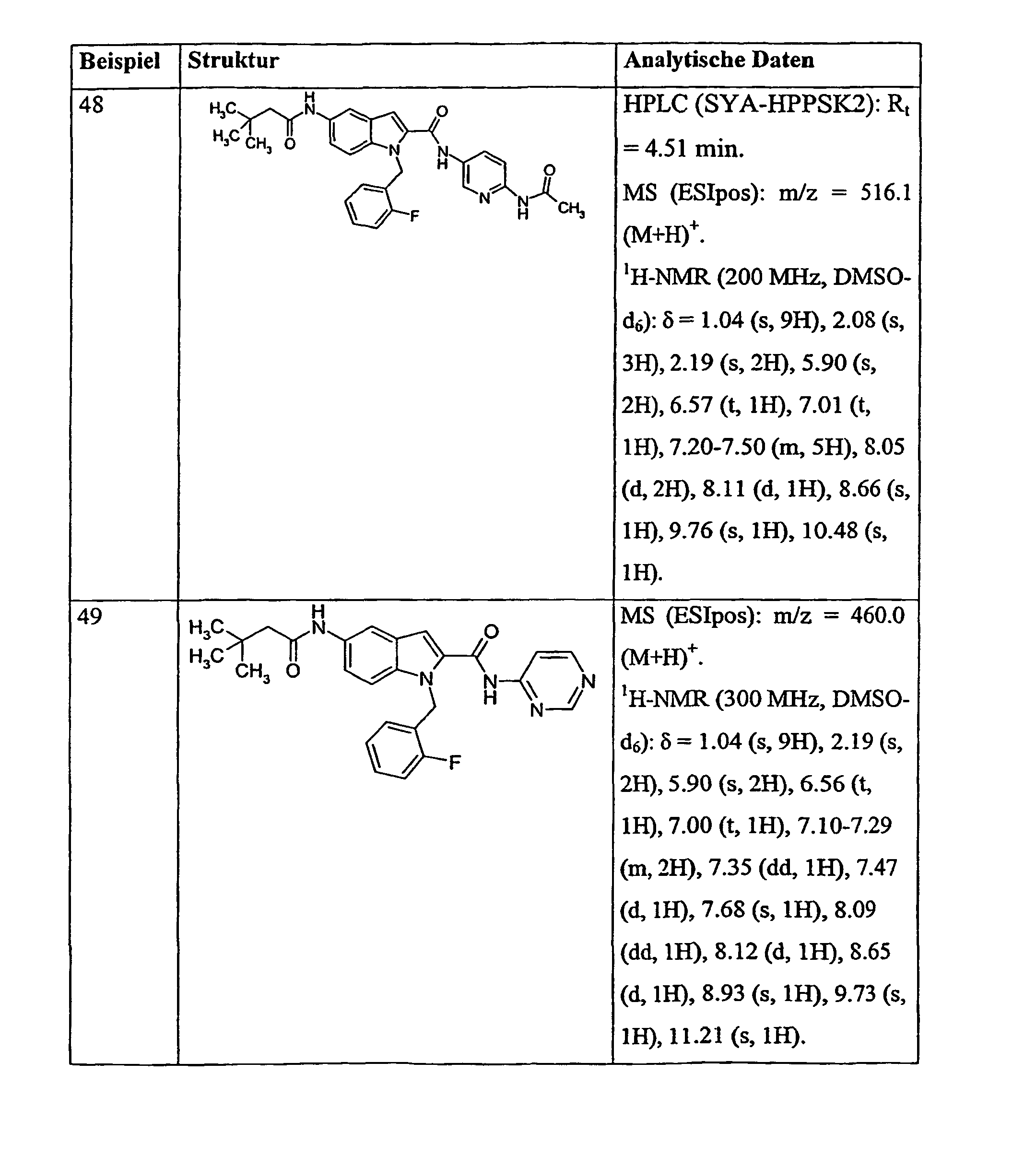

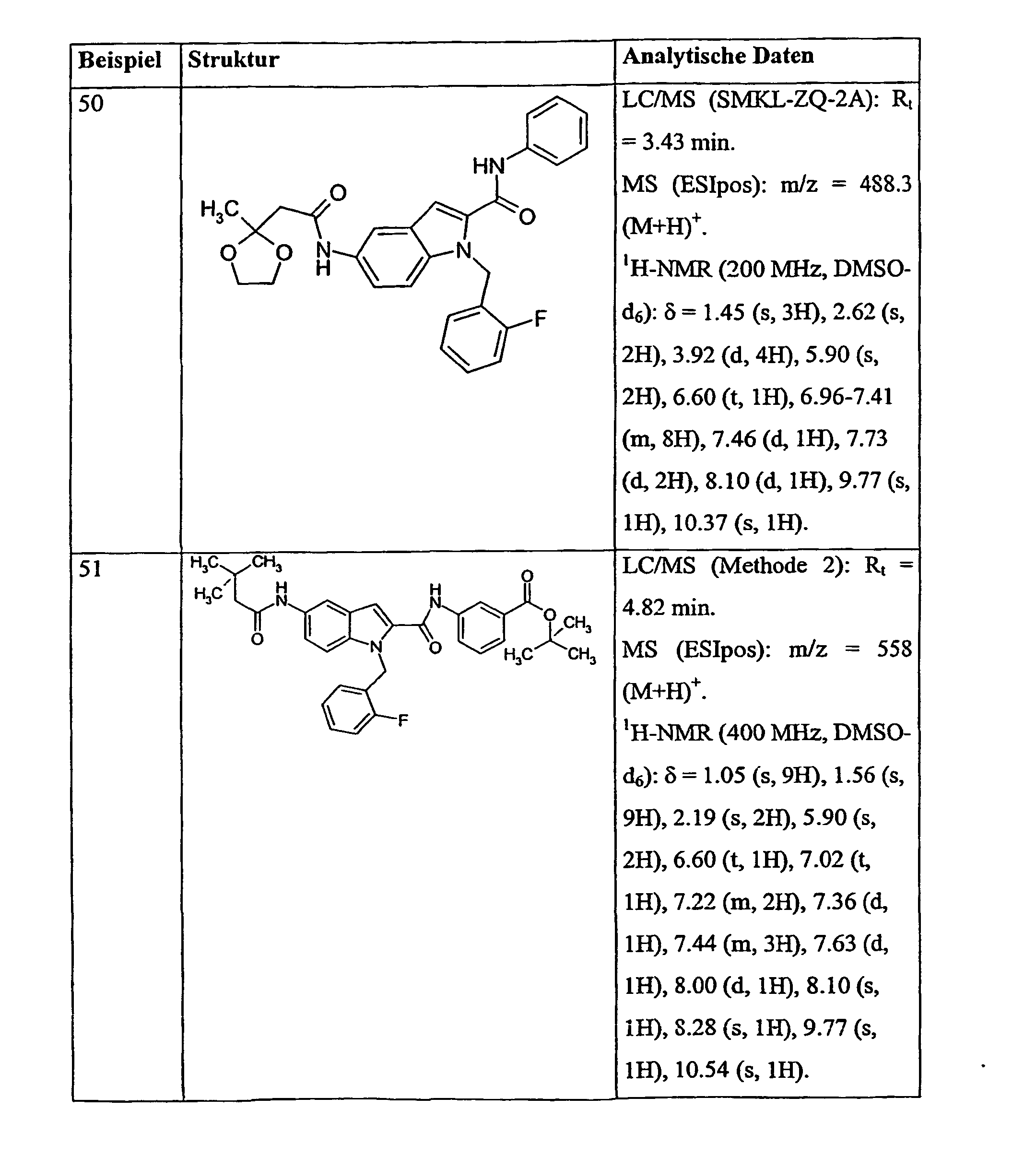

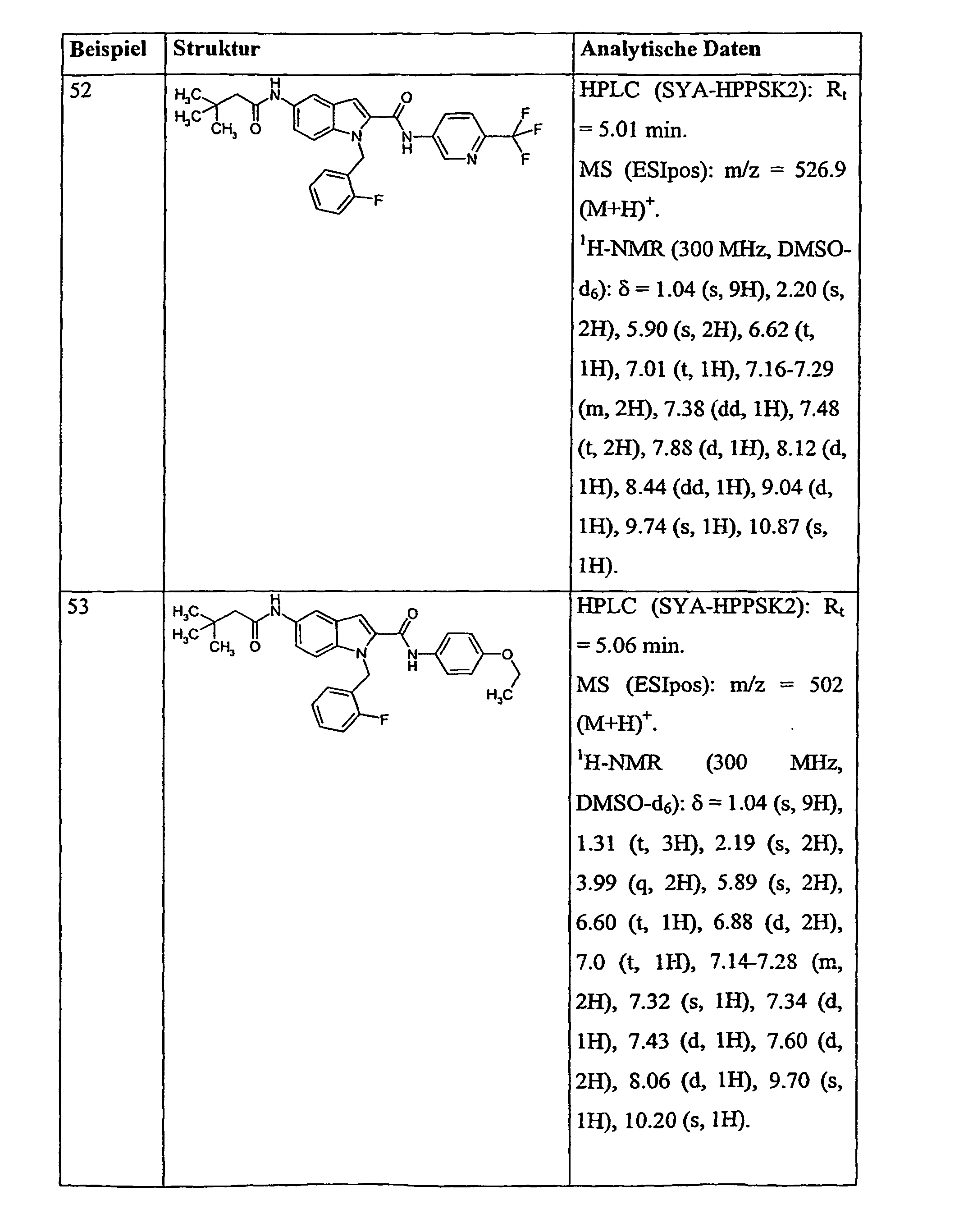

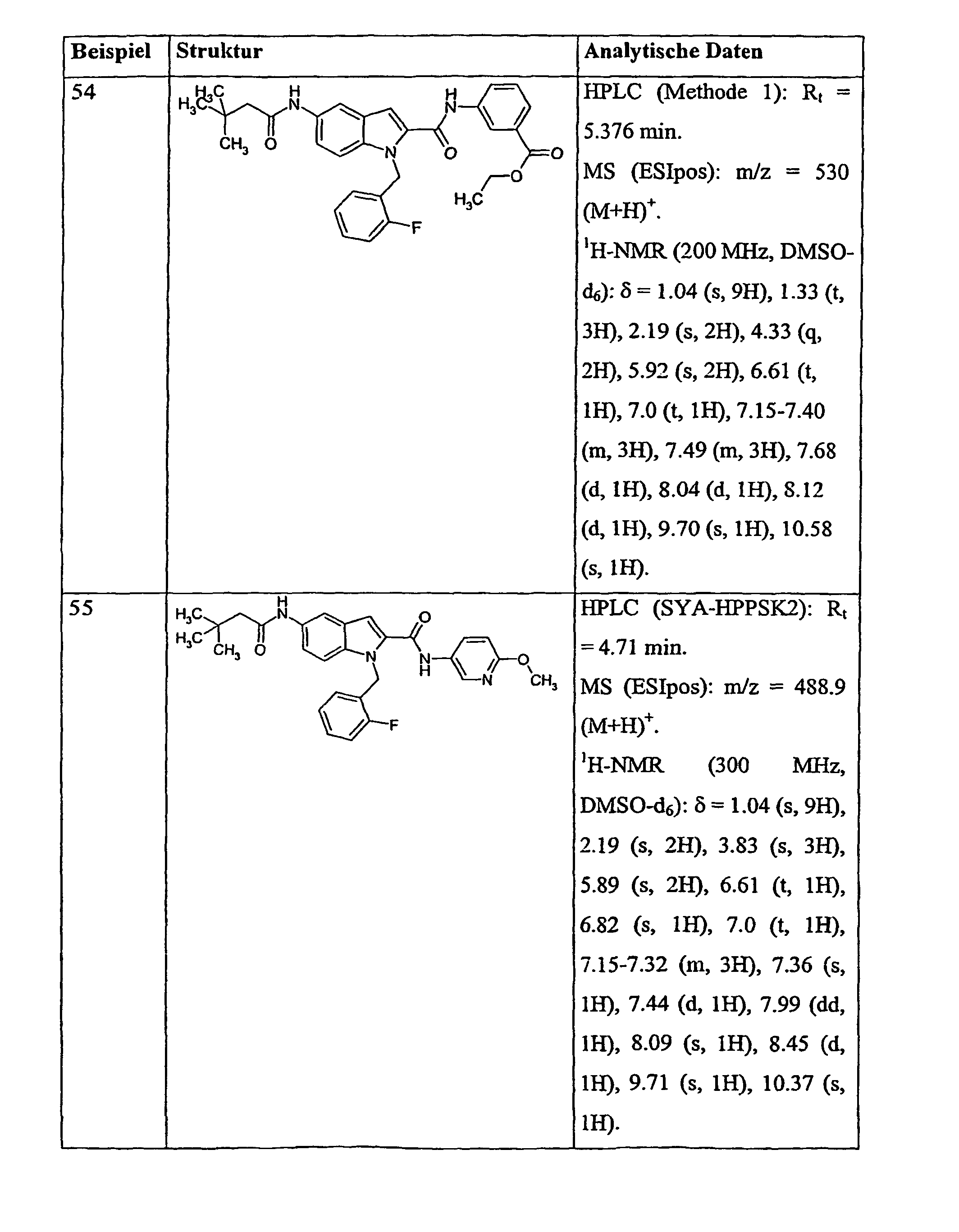

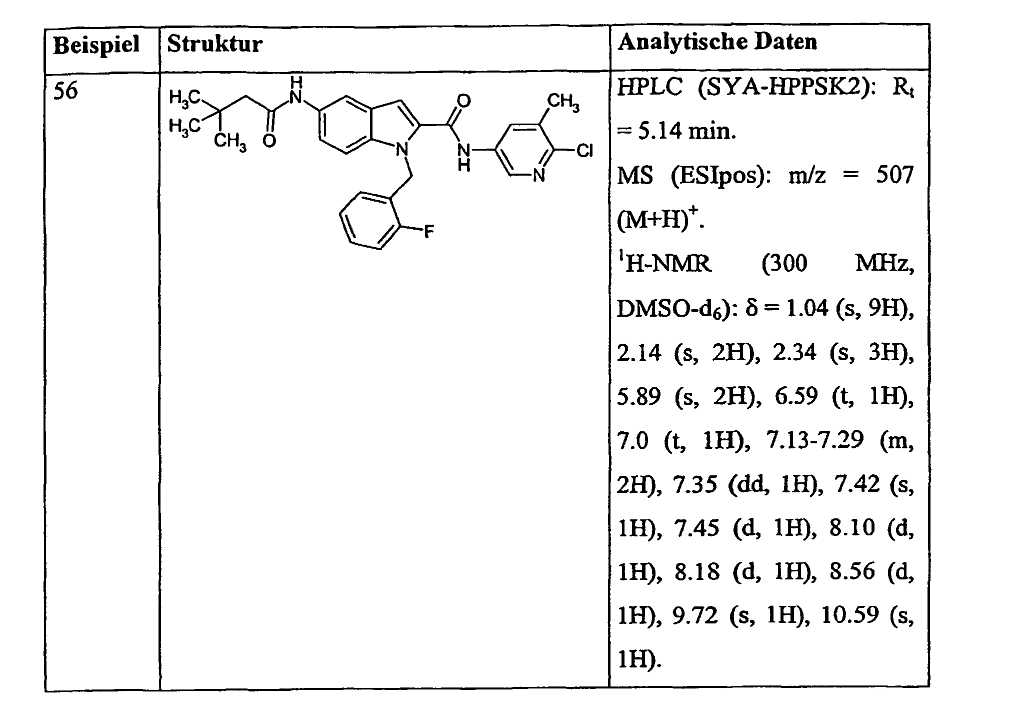

### Beispiel 57

### N-(4-Aminophenyl)-5-[(3,3-dimethylbutanoyl)amino]-1-(2-fluorbenzyl)-1H-indol-2-carboxamid-Hydrochlorid

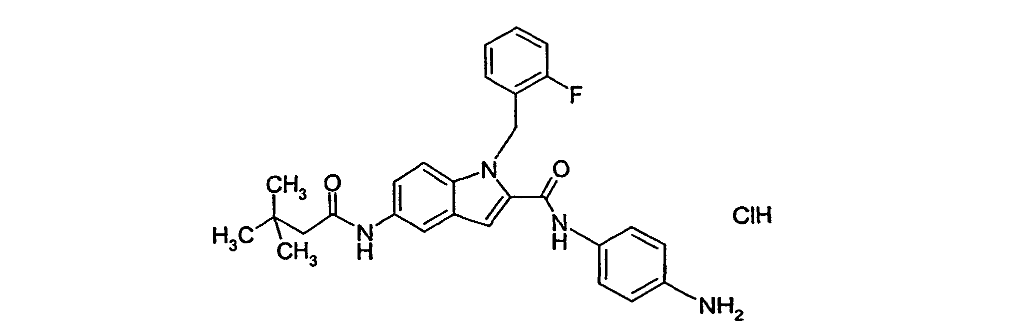

104 mg (0.18 mmol) tert-Butyl-4-({[5-[(3,3-dimethylbutanoyl)amino]-1-(2-fluorbenzyl)-1H-indol-2-yl]carbonyl}amino)phenylcarbamat (Beispiel XLV) werden in jeweils 1 ml Dioxan und 1 ml konzentrierter Salzsäure aufgenommen und 1 h bei RT gerührt. Das Lösungsmittel wird im Vakuum entfernt und die verbleibenden Kristalle abfiltriert und getrocknet. Man erhält 92.5 mg (75 %) des Produkts.
LC/MS (SMKL-ZQ-2): Rₜ = 3.09 min.
MS (ESIpos): m/z = 473 (M+H)⁺.
¹H-NMR (200 MHz, DMSO-d₆): δ = 1.04 (s, 9H), 1.47 (s, 9H), 2.19 (s, 2H), 5.90 (s, 2H), 6.56 (t, 1H), 7.00 (dt, 1H), 7.16-7.49 (m, 7H), 7.59 (d, 2H), 8.08 (s, 1H), 9.31 (s, 1H), 9.75 (s, 1H), 10.26 (s, 1H).

### Beispiel 58

### 5-[(3,3-Dimethylbutyl)amino]-1-(2-fluorbenzyl)-N-{4-[(methylsulfonyl)amino]-phenyl}-1H-indol-2-carboxamid

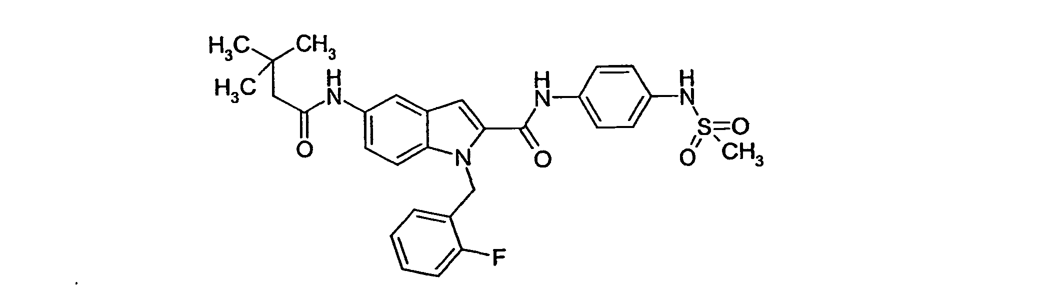

22.5 mg (0.20 mmol) Methansulfonylchlorid werden in 1 ml Dichlormethan gelöst und mit 38.9 mg (0.49 mmol) Pyridin versetzt. Zu dieser Mischung tropft man eine Lösung aus 100 mg (0.20 mmol) N-(4-Aminophenyl)-5-[(3,3-dimethylbutyl)amino]-1-(2-fluorbenzyl)-1H-indol-2-carboxamid-Hydrochlorid (Beispiel 57) in 1 ml Dichlormethan und rührt das Reaktionsgemisch über Nacht bei RT. Zur Aufarbeitung gibt man 22 ml ein molare Salzsäure zu und extrahiert mehrmals mit Dichlormethan. Die vereinigten organischen Phasen werden jeweils einmal mit gesättigter Kupfersulfat-Lösung, gesättigter Natriumhydrogencarbonat-Lösung und Wasser gewaschen. Anschließend werden sie über Natriumsulfat getrocknet, filtriert und das Lösungsmittel im Vakuum entfernt. Man erhält 96.2 mg (73 %) des Produkts.
LC/MS (MHZ2P01): Rₜ=4.81 min.
MS (ESIpos): m/z = 551 (M+H)⁺.
¹H-NMR (400 MHz, DMSO-d₆): δ = 1.04 (s, 9H), 2.19 (s, 2H), 2.94 (s, 3H), 5.89 (s, 2H), 6.58 (t, 1H), 7.00 (t, 1H), 7.14-7.29 (m, 4H), 7.34 (d, 2H), 7.45 (d, 1H), 7.68 (d, 2H), 8.09 (s, 1H), 9.58 (s, 1H), 9.73 (s, 1H), 10.35 (s, 1H).

Die Herstellung der folgenden Verbindung erfolgt analog zu der in Beispiel 58 beschriebenen Weise:

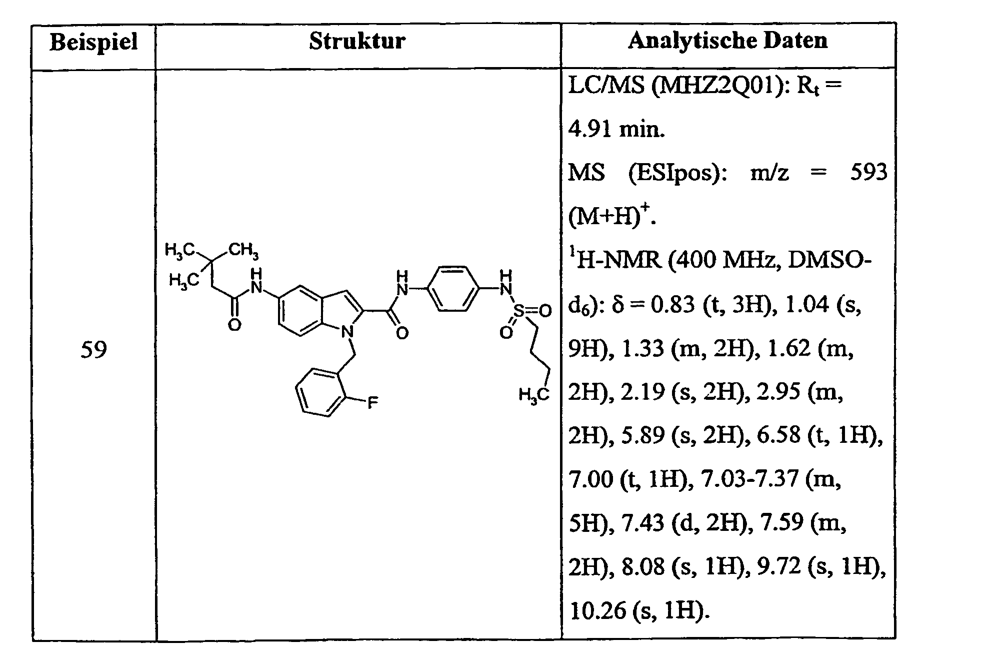

Die Herstellung der folgenden Verbindung erfolgt ausgehend von Beispiel 57 analog zu der in Beispiel 37 beschriebenen Weise:

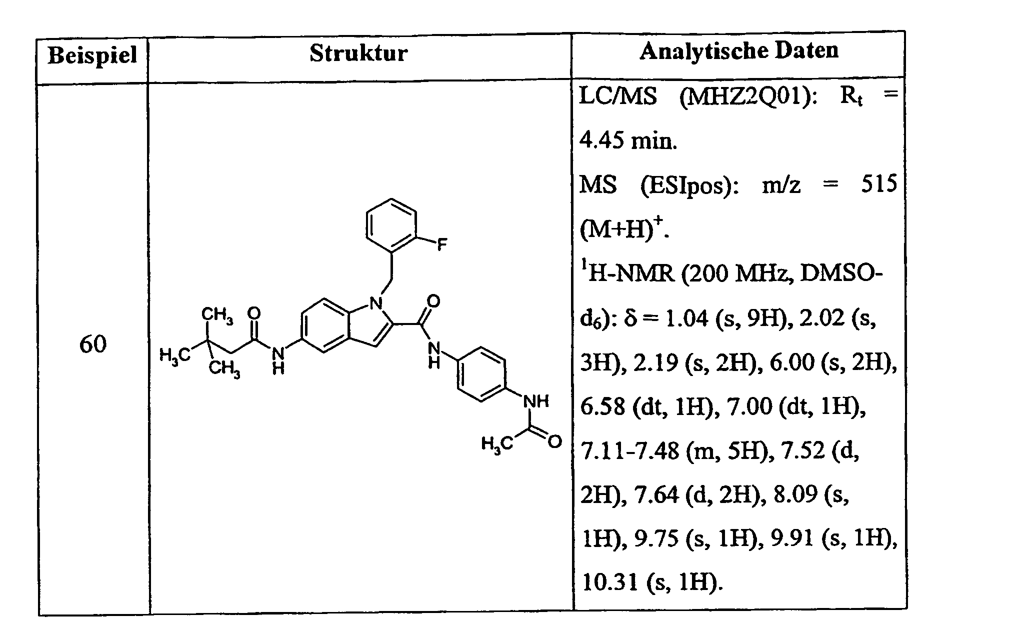

### Beispiel 61

### N-[4-(Butyrylamino)phenyl]-5-[(3,3-dimethylbutyl)amino]-1-(2-fluorbenzyl)-1H-indol-2-carboxamid

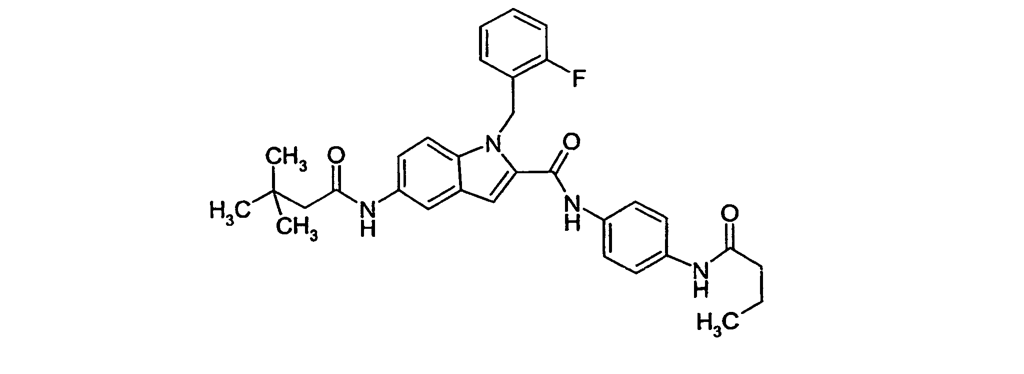

84 mg (0.14 mmol) Ethyl-2-({[4-({[5-[(3,3-dimethylbutanoyl)amino]-1-(2-fluorbenzyl)-1H-indol-2-yl]carbonyl}amino)phenyl]amino}carbonyl)butanoat (Beispiel XLVI) und 6.5 mg (0.27 mmol) Lithiumhydroxid werden in 0.5 ml Methanol und 0.5 ml THF aufgenommen und 30 min. auf 90°C erwärmt. Zur Aufarbeitung verdünnt man das erkaltete Reaktionsgemisch mit Ethylacetat und extrahiert jeweils einmal mit ein molarer Salzsäure und gesättigter Natriumchlorid-Lösung. Die organische Phase wird über Natriumsulfat getrocknet, filtriert und das Lösungsmittel im Vakuum entfernt. Der Rückstand wird mit Diethylether/Dichlormethan ausgerührt und der erhaltene Feststoff abfiltriert. Das Rohprodukt wird mittels präparativer HPLC aufgereinigt. Man erhält 4.9 mg (7 % d. Th.) des Produkts.
LC/MS (SMKL-ZQ-2A-CC): Rₜ = 3.54 min.
MS (ESIpos): m/z = 543.2 (M+H)⁺.

### Beispiel 62

### N-(6-Amino-3-pyridinyl)-5-[(3,3-dimethylbutanoyl)amino]-1-(2-fluorbenzyl)-1H-indol-2-carboxamid

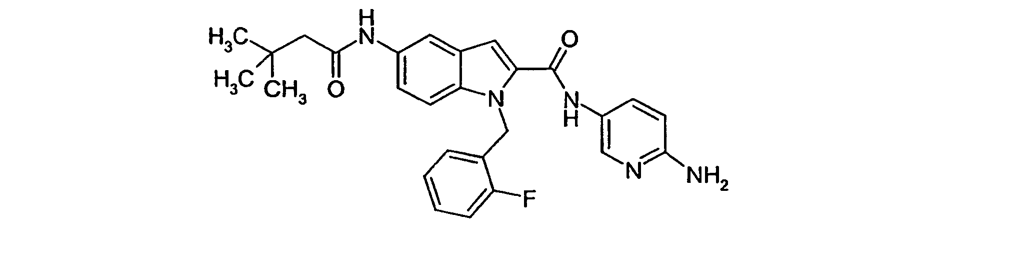

107 mg (0.12 mmol) Di-(tert-butyl)-5-({[5-[(3,3-dimethylbutanoyl)amino]-1-(2-fluorbenzyl)-1H-indol-2-yl]carbonyl}amino)-2-pyridinylimiddicarbonat (Beispiel XLII) werden in 2 ml Dichlormethan/Trifluoressigsäure (1:1) suspendiert und über Nacht bei RT gerührt. Das Lösungsmittel wird im Vakuum entfernt, der Rückstand in Wasser aufgenommen und mittels 1 molarer Natriumhydroxid-Lösung ein pH-Wert von 7-8 eingestellt. Man extrahiert mehrmals mit Ethylacetat, trocknet die vereinigten organischen Phasen über Natriumsulfat, filtriert und entfernt das Lösungsmittel im Vakuum. Das Rohprodukt wird mittels präparativer HPLC aufgereinigt. Man erhält 48 mg (83 %) des Produkts.
HPLC (SYA-HPPSK2): Rₜ = 4.47 min.
MS (ESIpos): m/z = 474.0 (M+H)⁺.
¹H-NMR (200 MHz, DMSC)-d₆): δ = 1.04 (s, 9H), 2.19 (s, 2H), 5.81 (s, 2H), 5.89 (s, 2H), 6.43 (d, 1H), 6.56 (t, 1H), 7.00 (t, 1H), 7.12-7.48 (m, 5H), 7.65 (d, 1H), 8.12 (d, 2H), 9.74 (s, 1H), 10.10 (s, 1H).

### Beispiel 63

### N-(5-Amino-2-pyridinyl)-5-[(3,3-dimethylbutanoyl)amino]-1-(2-fluorbenzyl)-1H-indol-2-carboxamid

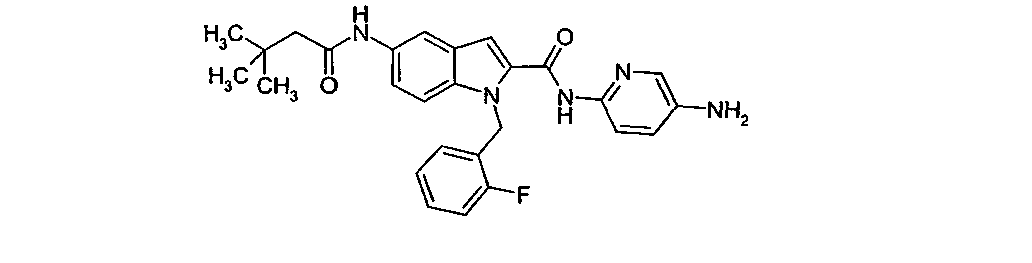

29 mg (0.05 mmol) Benzyl-6-({[5-[(3,3-dimethylbutanoyl)amino]-1-(2-fluorbenzyl)-1H-indol-2-yl]carbonyl}amino)-3-pyridinylcarbamat (Beispiel XLIII) werden mit 46.8 mg (0.19 mmol) Bromwasserstoff (33 %ige Lösung in Essigsäure) versetzt und über Nacht bei RT gerührt. Das Lösungsmittel wird im Vakuum entfernt und der Rückstand mittels präparativer HPLC aufgereinigt. Man erhält 6 mg (27 %) des Produkts.
LC/MS (MHZ2P01): Rₜ = 3.98 min.
MS (ESIpos): m/z=474.3 (M+H)⁺.
¹H-NMR (300 MHz, DMSO-d₆): δ = 1.04 (s, 9H), 2.19 (s, 2H), 5.14 (s, 2H), 5.91 (s, 2H), 6.52 (t, 1H), 6.99 (t, 1H), 7.14-7.29 (m, 3H), 7.30 (d, 1H), 7.49 (s, 1H), 7.69 (d, 1H), 7.73 (d, 1H), 8.08 (d, 1H), 9.69 (s, 1H), 10.32 (s, 1H).

### Beispiel 64

### 3-({[5-[(3,3-Dimethylbutanoyl)amino]-1-(2-fluorbenzyl)-1H-indol-2-yl]carbonyl}amino)benzoesäure

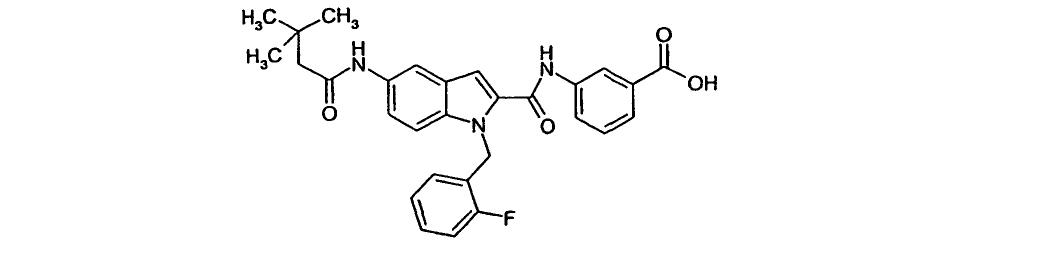

Eine Lösung von 182 mg (0.32 mmol) der Verbindung aus Beispiel 51 in 1 ml Dichlormethan wird mit 0.3 ml Trifluoressigsäure versetzt. Man rührt 1 h bei RT und kondensiert unter vermindertem Druck ein.
Ausbeute: 163 mg (100 % d. Th.)
LC/MS (MHZ2P01): Rₜ = 4.60 min.
MS (ESIpos): m/z = 502 (M+H)⁺.
¹H-NMR (300 MHz, DMSO-d₆): δ = 1.04 (s, 9H), 2.19 (s, 2H), 5.90 (s, 2H), 6.62 (t, 1H), 6.99 (t, 1H), 7.14-7.30 (m, 2H), 7.34 (dd, 1H), 7.44 (m, 3H), 7.65 (d, 1H), 7.97 (d, 1H), 8.09 (d, 1H), 8.38 (s, 1H), 9.71 (s, 1H), 10.48 (s, 1H).

### Beispiel 65

### N-{3-[(tert-Butylamino)carbonyl]phenyl}-5-[(3,3-dimethylbutanoyl)amino]-1-(2-fluorbenzyl)-1H-indol-2-carboxamid

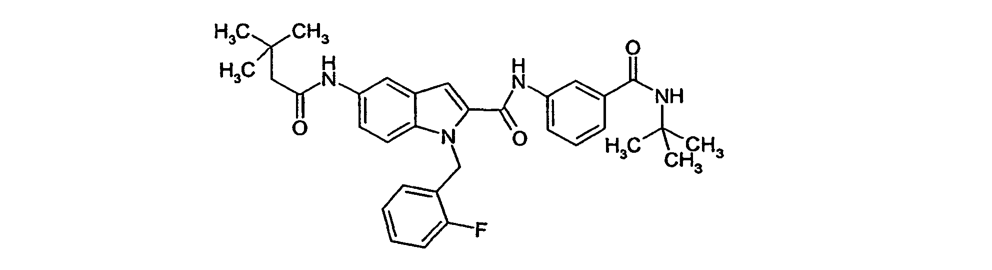

Man legt 20 mg (0.04 mmol) der Verbindung aus Beispiel 64, 11.5 mg (0.06 mmol) N'-(3-Dimethylaminopropyl)-N-ethylcarbodiimid x HCl und 2.5 mg (0.02 mmol) 4-Dimethylaminopyridin in 1 ml Dichlormethan vor. Dazu wird 3.5 mg (0.05 mmol) tert-Butylamin zugegeben und über Nacht bei RT nachgerührt. Das Gemisch wird zur Aufarbeitung mit wässriger Salzsäure und Dichlormethan verdünnt und extrahiert. Die organische Phase wird mit ges. Natriumchloridlösung gewaschen, mit Natriumsulfat getrocknet, filtriert und im Vakuum getrocknet. Reinigung erfolgt durch Chromatographie an Kieselgel.
Ausbeute: 10 mg (45 % d. Th.)
LC/MS (Methode 2): Rₜ = 3.82 min.
MS (ESIpos): m/z = 557 (M+H)⁺.
¹H-NMR (300 MHz, DMSO-d₆): δ = 1.04 (s, 9H), 1.41 (s, 9H), 2.20 (s, 2H), 5.91 (s, 2H), 6.60 (t, 1H), 6.99 (t, 1H), 7.25 (m, 2H), 7.40 (m, 4H), 7.62 (s, 1H), 7.86 (d, 1H), 8.09 (d, 2H), 9.71 (s, 1H), 10.41 (s, 1H).

### Beispiel 66

### 4-({[5-[(3,3-Dimethylbutanoyl)amino]-1-(2-fluorbenzyl)-1H-indol-2-yl]carbonyl}amino)benzoesäure

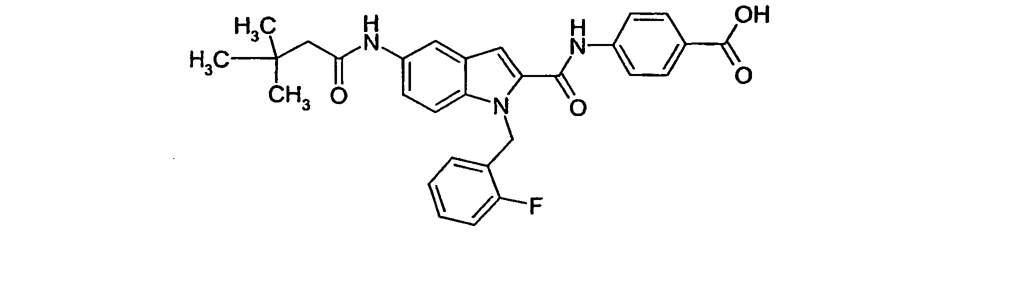

Zu einer Suspension von 1.0 g (0.8 mmol) der Substanz aus Beispiel XLI in 21 ml Pyridin/DMF (2:1) gibt man 912.6 mg (2.4 mmol) HATU und 1.24 g (6.4 mmol) tert-Butyl-4-aminobenzoat zu und schüttelt das Reaktionsgemisch über Nacht bei RT. Das Harz wird abgesaugt und mit DMF, Ethanol (30 %), Wasser, DMF, Methanol und Dichlormethan. Zur Abspaltung des Polymers suspendiert man das gebundene Produkt in Dichlormethan/Trifluoressigsäure (1:1) und schüttelt 30 min. bei RT. Das freie Polymer wird abgesaugt, mit Dichlormethan gewaschen und das Filtrat im Vakuum von Lösungsmittel befreit. Der Rückstand wird chromatographisch über Kieselgel aufgereinigt (Eluent: Dichlormethan/Methanol 5:1). Man erhält 127 mg (32 % d. Th.) des Produkts.
LC/MS (MHZ2P): Rₜ = 4.39 min.
MS (ESIpos): m/z = 502.3 (M+H)⁺.

Die Herstellung der folgenden Verbindung erfolgt analog zu der in Beispiel 66 beschriebenen Weise:

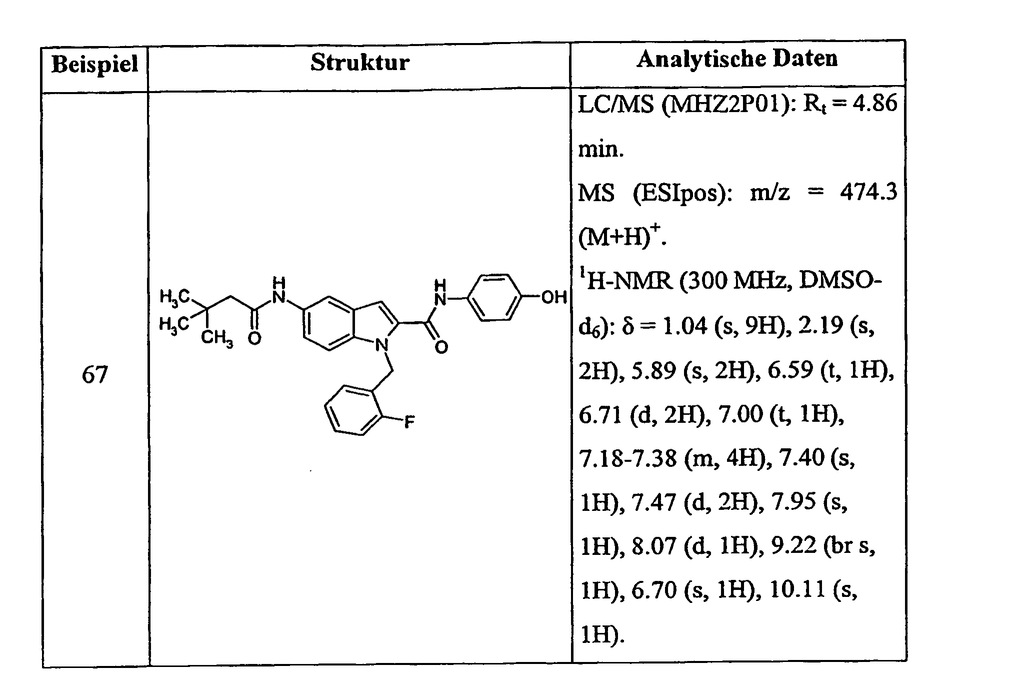

### Beispiel 68

### 5-[(Bicyclo[2.2.1]hept-2-ylacetyl)amino]-N-phenyl-1-(2-phenylethyl)-1H-indol-2-carboxamid

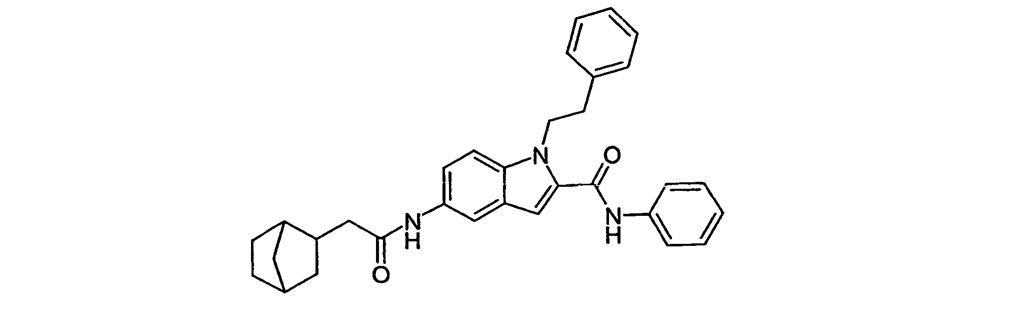

78 mg (0.22 mmol) 5-Amino-N-phenyl-1-(2-phenylethyl)-1H-indol-2-carboxamid (Beispiel LI) werden in 2 ml THF unter Argon gelöst und mit 24.4 mg (0.24 mmol) Triethylamin versetzt. Die Lösung wird auf 0°C gekühlt und tropfenweise mit einer Lösung aus 37.9 mg (0.22 mmol) Bicyclo[2.2.1]hept-2-ylacetylchlorid in 0.2 ml THF versetzt. Nachdem das Reaktionsgemisch 2 h bei RT nachgerührt wurde verdünnt man es mit 1 molarer Salzsäure und extrahiert mehrmals mit Ethylacetat. Die vereinigten organischen Phasen werden zweimal mit gesättigter Natriumchlorid-Lösung und einmal mit Natriumhydrogencarbonat-Lösung gewaschen, über Natriumsulfat getrocknet und filtriert. Der nach dem Entfernen des Lösungsmittels im Vakuum erhaltene Rückstand wird chromatographisch über Kieselgel aufgereinigt (Eluent: Dichlormethan/Ethylacetat). Man gewinnt 31.5 mg (29 % d. Th.) des Produkts.
LC/MS (MHZ2P): Rₜ = 5.17 min.
MS (ESIpos): m/z = 492 (M+H)⁺.

Die Herstellung der folgenden Verbindung erfolgt analog zu der in Beispiel 68 beschriebenen Weise aus dem Ausgangsmaterial aus Beispiel LI:

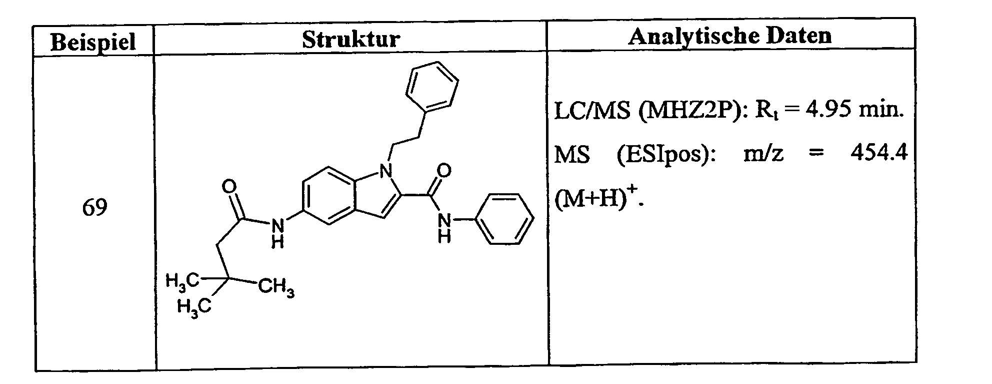

### Beispiel 70

### 5-[(3,3-Dimethylbutanoyl)amino]-1-(2-hydroxyethyl)-N-phenyl-1H-indol-2-carboxamid

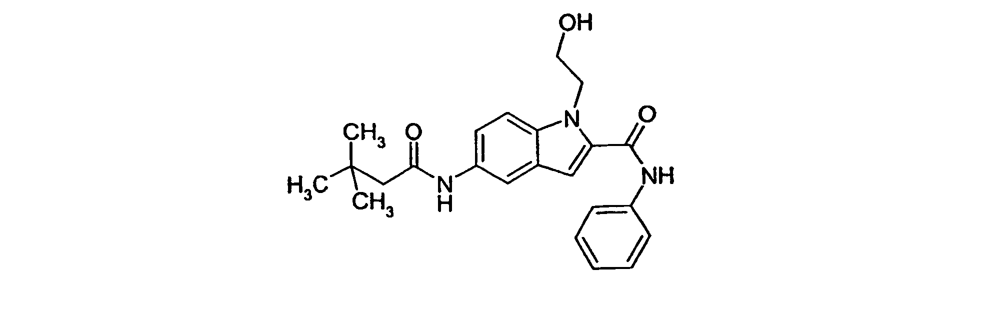

50 mg (0.12 mmol) der Verbindung aus Beispiel LV werden in 5 ml Methanol gelöst. Dazu gibt man portionsweise und über mehrere Stunden verteilt insgesamt 77.4 mg (2.05 mmol) Natriumborhydrid bei RT zu. Man rührt bei dieser Temperatur über Nacht und verdünnt das Reaktionsgemisch dann mit ein molarer Salzsäure und extrahiert mehrmals mit Ethylacetat. Die vereinigten organischen Phasen werden über Natriumsulfat getrocknet, filtriert und das Lösungsmittel im Vakuum entfernt. Man erhält 38 mg (84 % d. Th.) des Produkts. LC/MS (MHZ2P01): Rt = 4.14 min.
MS (ESIpos): m/z = 394.3 (M+H)⁺.
¹H-NMR (200 MHz, DMSO-d₆): δ = 1.04 (s, 9H), 2.19 (s, 2H), 3.69 (q, 2H), 4.57 (t, 2H), 4.88 (t, 1H), 7.10 (t, 1H), 7.22 (s, 1H), 7.25-7.63 (m, 4H), 7.77 (d, 2H), 8.06 (d, 1H), 9.72 (s, 1H), 10.32 (s, 1H).

### Beispiel 71

### 5-[(3,3-Dimethylbutanoyl)amino]-N,1-diphenyl-1H-indol-2-carboxamid

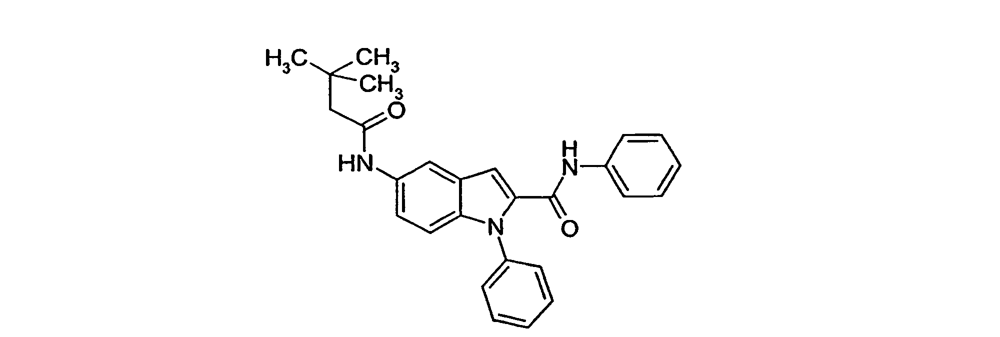

Zu einer Lösung aus 27 mg (0.08 mmol) 5-[(3,3-Dimethylbutanoyl)amino]-1-phenyl-1H-indol-2-carbonsäure (Beispiel LIX), 4.7 mg (0.09 mmol) DMAP und 22.2 mg (0.12 mmol) EDC in 2 ml Dichlormethan gibt man 7.9 mg (0.09 mmol) Anilin zu und rührt für 3h bei RT. Zur Aufarbeitung gibt man ein molare Salzsäure zu und extrahiert mehrmals mit Dichlormethan. Die vereinigten organischen Phasen werden mit gesättigter Natriumhydrogencarbonat-Lösung und Wasser gewaschen, über Natriumsulfat getrocknet und filtriert. Nachdem man das Lösungsmittel im Vakuum entfernt hat, erhält man 35 mg (99 % d. Th.) des Produkts.
LC/MS (MHZ2Q01): Rₜ = 4.85 min.
MS (ESIpos): m/z = 426.4 (M+H)⁺.
¹H-NMR (200 MHz, DMSO-d₆): δ =1.04 (s, 9H), 2.20 (s, 2H), 7.05-7.15 (m, 2H), 7.29-7.41 (m, 6H), 7.44-7.60 (m, 3H), 7.65 (d, 2H), 8.15 (d, 1H), 9.79 (s, 1H), 10.43 (s, 1H).

Die Herstellung der folgenden Verbindung erfolgt analog zu der in Herstellungsbeispiel 57 beschriebenen Weise:

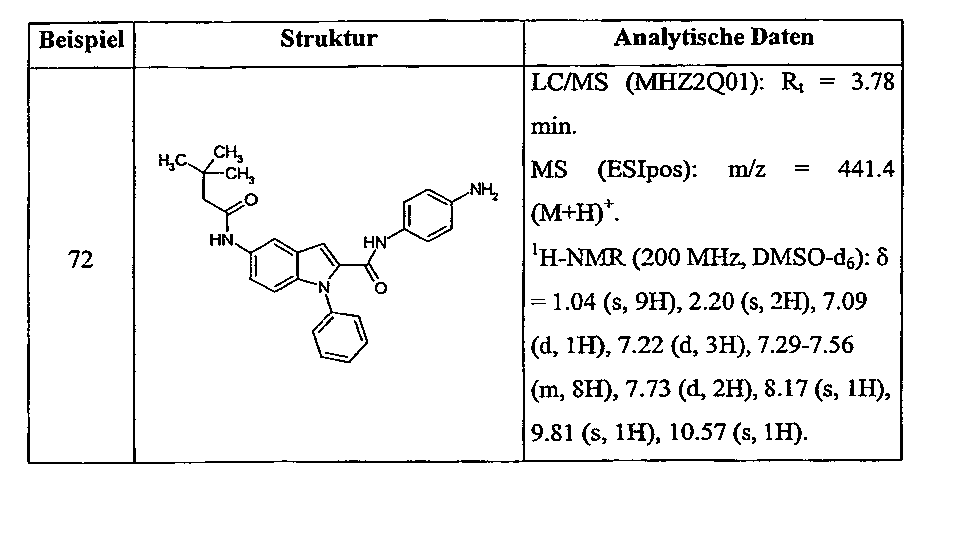

Die Herstellung der folgenden Verbindungen erfolgt analog zu der in Beispiel 26 und 41 beschriebenen Weise aus den entsprechenden Ausgangsmaterialien:

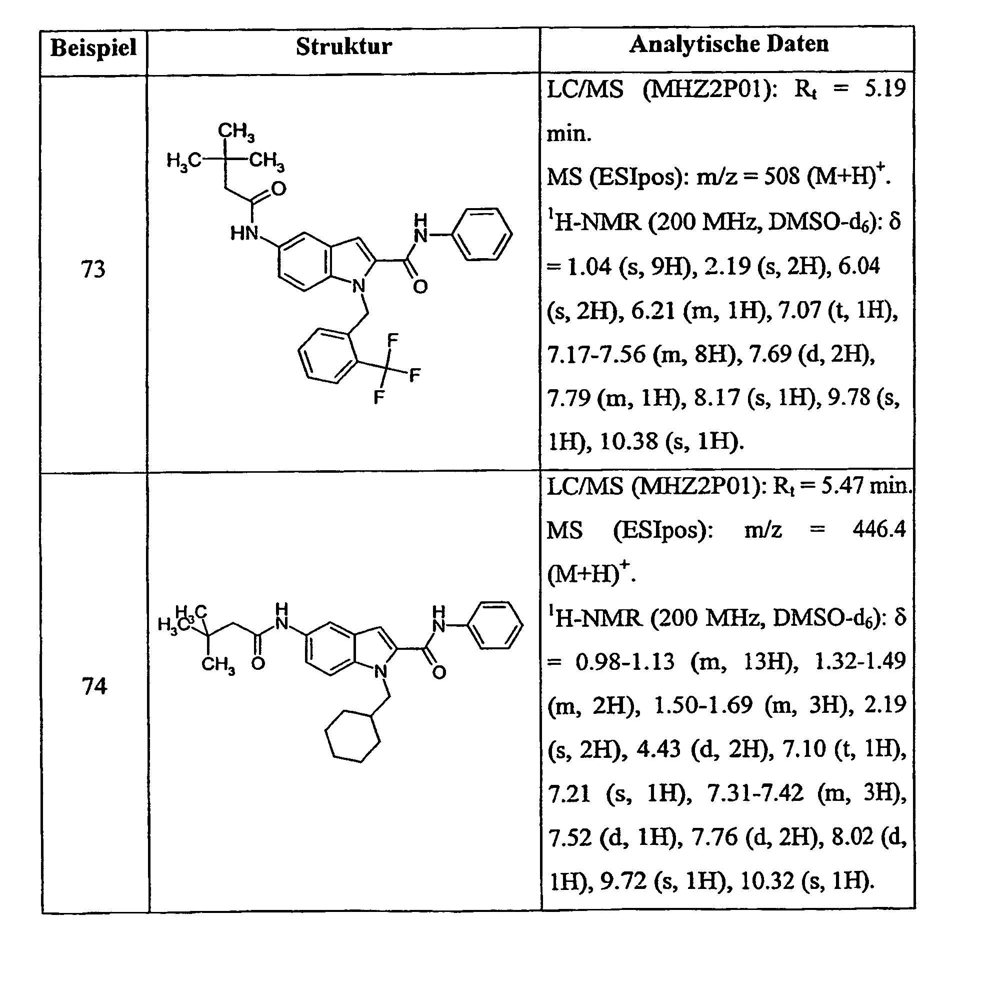

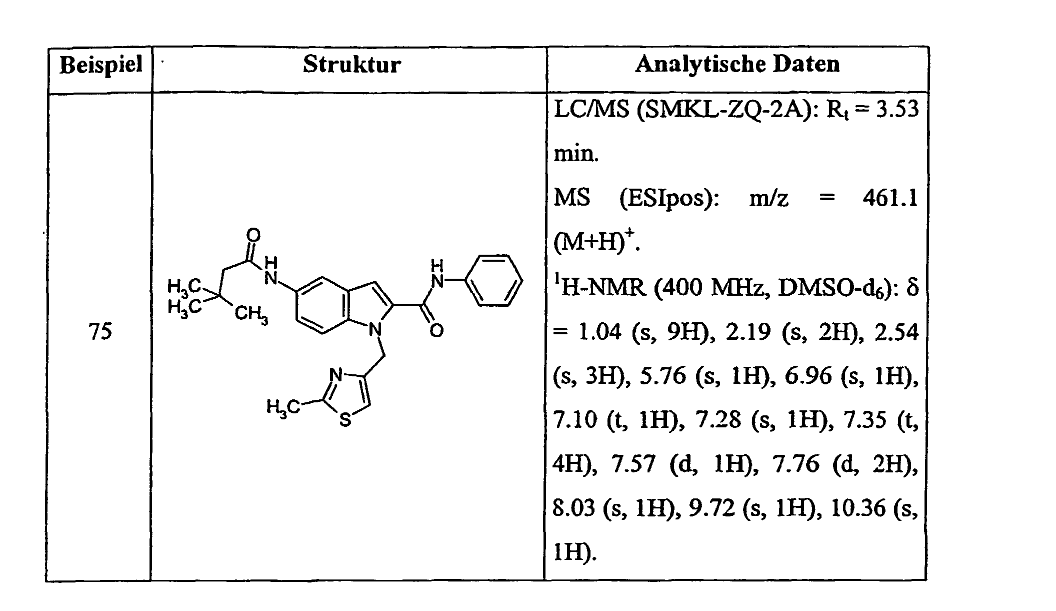

Die Herstellung der folgenden Verbindungen erfolgt analog zu der in Beispiel XXXV beschriebenen Weise:

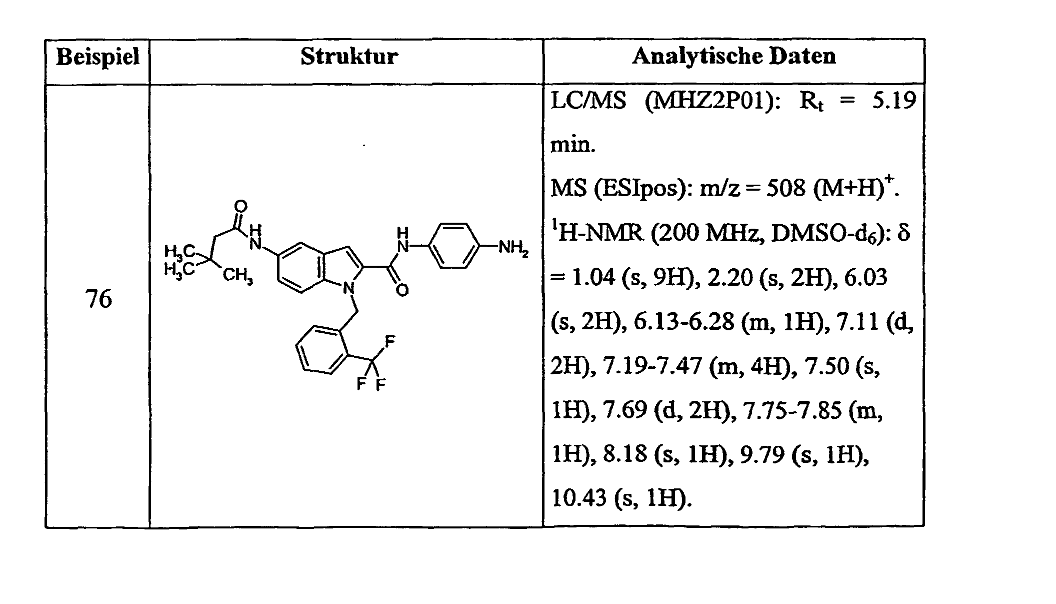

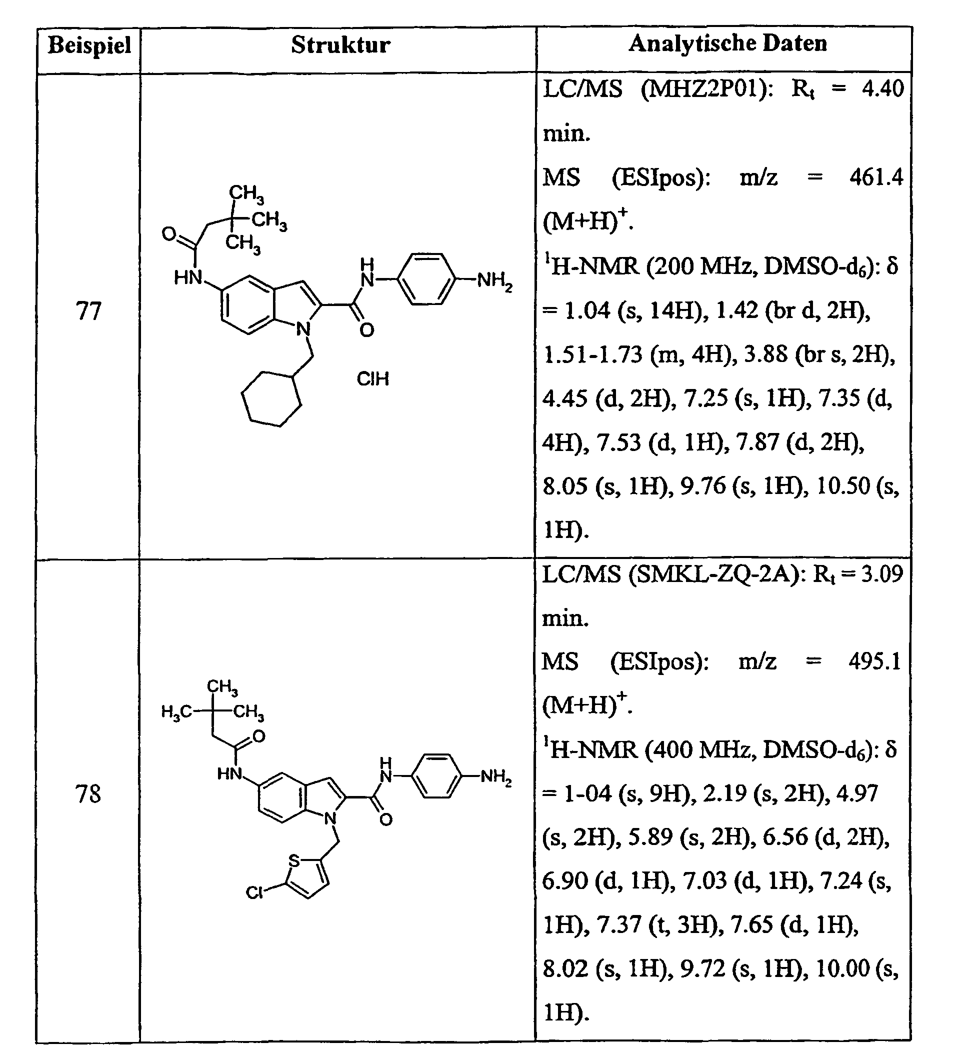

Die Herstellung der folgenden Verbindung erfolgt analog zu der in Beispiel 37 beschriebenen Weise:

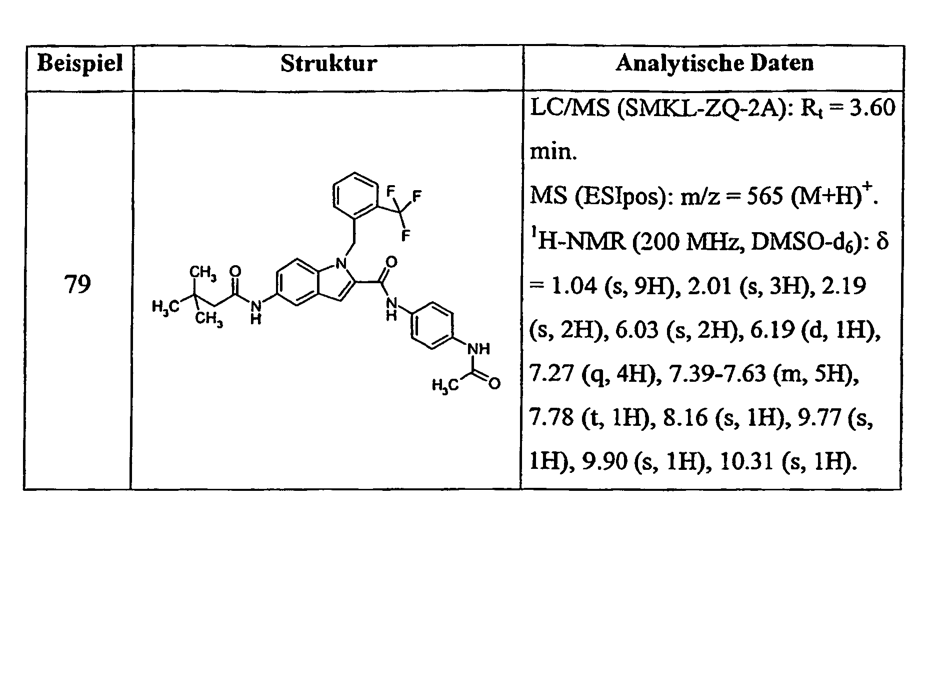

## Patentansprüche

1. Verbindungen der Formel (I) 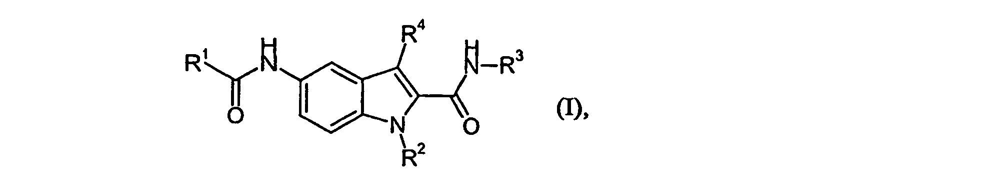 in welcher
R¹ für (C₅-C₁₅)-Alkyl, (C₅-C₁₅)-Alkenyl oder (CH₂)ₙG steht,
worin
G für Cycloalkyl oder für einen 5- oder 6-gliedrigen Heterocyclus mit einem oder zwei Sauerstoffatomen steht,
n für 0 bis 4 steht und
Alkyl, Alkenyl und G gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Carboxyl, Alkoxycarbonyl, Amino, Alkylamino, Alkylcarbonylamino und Alkylaminocarbonyl, substituiert sind,
R² für (C₁-C₈)-Alkyl, (CH₂)ₘCycloalkyl, (CH₂)ₘHeterocyclyl, (CH₃)ₘAryl oder (CH₂)ₘHeteroaryl steht,
worin
m für 0 bis 4 steht und
Alkyl, Cycloalkyl, Heterocyclyl, Aryl und Heteroaryl gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Alkoxycarbonyl, Amino, Alkylamino, Alkylcarbonylamino, Alkylaminocarbonyl, Alkylaminosulfonyl und Alkylsulfonylamino, substituiert sind,
R³ für (CH₂)ₒCycloalkyl, (CH₂)ₒHeterocyclyl, (CH₂)ₒAryl oder (CH₂)ₒHeteroaryl steht,
worin
o für 0 bis 4 steht und Cycloalkyl, Heterocyclyl, Aryl und Heteroaryl gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Hydroxycarbonyl, Alkoxycarbonyl, Amino, Alkylamino, Alkylcarbonylamino, Alkylaminocarbonyl, Alkylaminosulfonyl und Alkylsulfonylamino, substituiert sind,
R⁴ für Wasserstoff, (C₁-C₄)-Alkyl, (CH₂)ₚCycloalkyl, (CH₂)ₚHeterocyclyl, (CH₂)ₚAryl oder (CH₂)ₚHeteroaryl steht,
worin
p für 0 bis 4 steht und
Alkyl, Cycloalkyl, Heterocyclyl, Aryl und Heteroaryl gegebenenfalls durch 1 bis 3 Substituenten, unabhängig voneinander ausgewählt aus der Gruppe von Halogen, Hydroxy, Trifluomiethyl, Trifluormethoxy, Cyano, Nitro, Alkyl, Alkoxy, Alkylthio, Hydroxycarbonyl, Alkoxycarbonyl, Amino, Alkylamino, Alkylcarbonylamino, Alkylaminocarbonyl Alkylaminosulfonyl und Alkylsulfonylamino, substituiert sind,
und ihre Salze, Hydrate, Hydrate der Salze und Solvate.

2. Verbindungen der Formel (I) nach Anspruch 1
in welcher
R¹ für (C₅-C₁₅)-Alkyl oder (CH₂)ₙCycloalkyl steht,
worin
n für 0 bis 4 steht und
Alkyl und Cycloalkyl gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Carboxyl, Alkoxycarbonyl, Alkylcarbonylamino und Alkylaminocarbonyl, substituiert sind,
R² für (C₁-C₈)-Alkyl, (CH₂)ₘCycloalkyl, (CH₂)ₘHeterocyclyl, (CH₂)ₘAryl oder (CH₂)ₘHeteroaryl steht,
worin
m für 0 bis 4 steht und
Alkyl, Cycloalkyl, Heterocyclyl, Aryl und Heteroaryl gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Alkoxycarbonyl, Amino, Alkylamino, Alkylcarbonylamino, Alkylaminocarbonyl, Alkylaminosulfonyl und Alkylsulfonylamino, substituiert sind,
R³ für (CH₂)ₒCycloalkyl, (CH₂)ₒHeterocyclyl, (CH₂)ₒAryl oder (CH₂)ₒHeteroaryl steht,
worin
o für 0 bis 4 steht und Cycloalkyl, Heterocyclyl, Aryl und Heteroaryl gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Hydroxycarbonyl, Alkoxycarbonyl, Amino, Alkylamino, Alkylcarbonylamino, Alkylaminocarbonyl Alkylaminosulfonyl und Alkylsulfonylamino, substituiert sind,
R⁴ für Wasserstoff, (C₁-C₄)-Alkyl, (CH₂)ₚCycloalkyl, (CH₂)ₚHeterocyclyl, (CH₂)ₚAryl oder (CH₂)ₚHeteroaryl steht,
worin
p für 0 bis 4 steht und
Alkyl, Cycloalkyl, Heterocyclyl, Aryl und Heteroaryl gegebenenfalls durch 1 bis 3 Substituenten, unabhängig voneinander ausgewählt aus der Gruppe von Halogen, Hydroxy, Trifluormethyl, Trifluormethoxy, Cyano, Nitro, Alkyl, Alkoxy, Alkylthio, Hydroxycarbonyl, Alkoxycarbonyl, Amino, Alkylamino, Alkylcarbonylamino, Alkylaminocarbonyl Alkylaminosulfonyl und Alkylsulfonylamino, substituiert sind,
und ihre Salze, Hydrate, Hydrate der Salze und Solvate.

3. Verbindungen der Formel (I) nach Anspruch 1,
in welcher
R¹ für Neopentyl, (Bicyclo[2.2.1]heptyl)methyl, Cyclohexylmethyl, Cyclobutylmethyl, Cyclopentylmethyl, 2,2-Dimethyl-1-butyl, 2-Ethyl-2-metyl-1-butyl, (1-Methylcyclopentyl)methyl, 1-Methylcyclohexyl, 4-Hydroxy-2,2-dimethyl-1-butyl oder 2,2-Dimethyl-1-but-3-enyl steht,
R² für (C₁-C₄)-Alkyl, das durch Hydroxy oder Fluor substituiert sein kann oder für Benzyl, das gegebenenfalls durch 1 oder 2 Substituenten, unabhängig voneinander ausgewählt aus der Gruppe von Fluor, Chlor, Brom, Methyl und Trifluormethyl substituiert ist, steht,
R³ für Phenyl, Pyridyl oder Pyrimidyl steht, die ihrerseits gegebenenfalls durch einen Substituenten ausgewählt aus der Gruppe von Fluor, Chlor, Trifluormethyl, Methyl, Ethyl, Methoxy, Ethoxy, n-Propoxy, Isopropoxy, Amino, Hydroxy, Hydroxycarbonyl, (C₁-C₃)-Alkylcarbonylamino und Mono-(C₁-C₄)-Alkylaminocarbonyl substituiert sind,
R⁴ für Wasserstoff steht
und ihre Salze, Hydrate, Hydrate der Salze und Solvate.

4. Verfahren zur Herstellung der Verbindungen der Formel (I), wie in Anspruch 1 definiert, **dadurch gekennzeichnet, dass** man entweder
[**A**] Verbindungen der Formel (II) 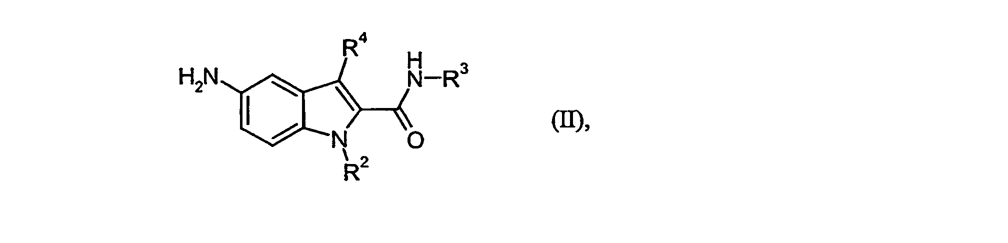 in welcher
R², R³ und R⁴ die in Anspruch 1 angegebene Bedeutung aufweisen,
mit Verbindungen der Formel (III) 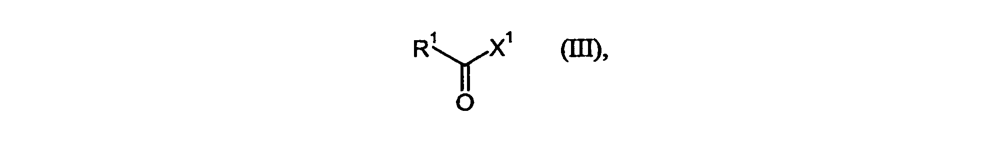 in welcher
R¹ die in Anspruch 1 angegebene Bedeutung aufweist und
X¹ für Halogen oder Hydroxy steht,
oder
[**B**] Verbindungen der Formel (XI) 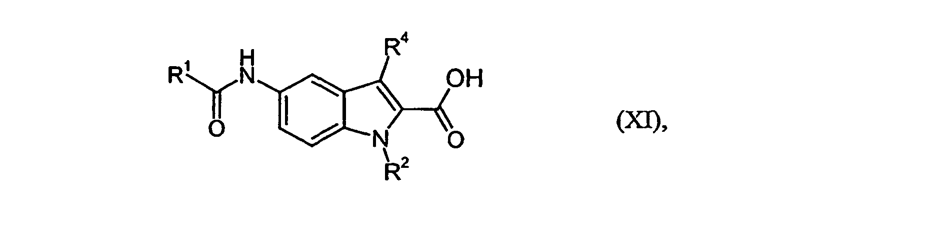
in welcher
R¹, R² und R⁴ die in Anspruch 1 angegebene Bedeutung aufweisen,
mit Verbindungen der Formel (VI) 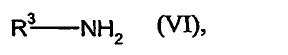 in welcher
R³ die in Anspruch 1 angegebene Bedeutung aufweist,
umsetzt.

5. Verbindungen der Formel (I), wie in Anspruch 1 definiert, zur Prophylaxe und/oder Behandlung von Erkrankungen.

6. Arzneimittel, enthaltend mindestens eine Verbindung der Formel (I), wie in Anspruch 1 definiert, und mindestens einen weiteren Hilfsstoff.

7. Arzneimittel, enthaltend mindestens eine Verbindung der Formel (I), wie in Anspruch 1 definiert, und mindestens einen weiteren Wirkstoff.

8. Verwendung von Verbindungen der Formel (I), wie in Anspruch 1 definiert, zur Herstellung von Arzneimitteln zur Prophylaxe und/oder Behandlung von kardiovaskulären Erkrankungen.

9. Verwendung von Verbindungen der Formel (I), wie in Anspruch 1 definiert, zur Herstellung von Arzneimitteln zur Prophylaxe und/oder Behandlung von Herzinfarkt, Angina pectoris und Herzinsuffizienz.

## Claims

1. Compounds of the formula (I) 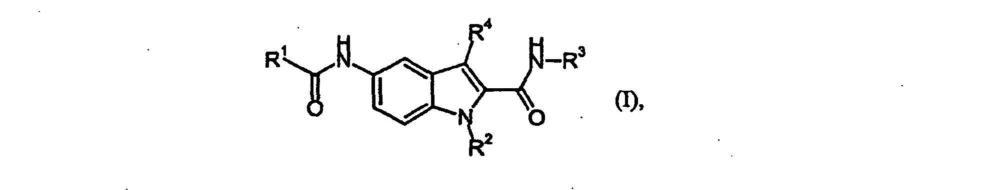 in which
R¹ represents (C₅-C₁₅)-alkyl, (C₅-C₁₅)-alkenyl or (CH₂)ₙG,
in which
G represents cycloalkyl or represents a 5-or 6-membered heterocycle having one or two oxygen atoms,
n represents 0 to 4 and
alkyl, alkenyl and G are optionally substituted by 1 to 3 substituents, independently of one another selected from the group consisting of halogen, hydroxyl, trifluoromethyl, trifluoromethoxy, cyano, nitro, alkyl, alkoxy, alkylthio, carboxyl, alkoxycarbonyl, amino, alkylamino, alkylcarbonylamino and alkylaminocarbonyl,
R² represents (C₁-C₈)-alkyl, (CH₂)ₘcycloalkyl, (CH₂)ₘheterocyclyl, (CH₂)ₘaryl or (CH₂)ₘheteroaryl,
in which
m represents 0 to 4 and
alkyl, cycloalkyl, heterocyclyl, aryl and heteroaryl are optionally substituted by 1 to 3 substituents, independently of one another selected from the group consisting of halogen, hydroxyl, trifluoromethyl, trifluoromethoxy, cyano, nitro, alkyl, alkoxy, alkylthio, alkoxycarbonyl, amino, alkylamino, alkylcarbonylamino, alkylaminocarbonyl, alkylaminosulphonyl and alkylsulphonylamino,
R³ represents (CH₂)ₒcycloalkyl, (CH₂) ₒheterocyclyl, (CH₂) ₒaryl or (CH₂) ₒheteroaryl,
in which
o represents 0 to 4 and
cycloalkyl, heterocyclyl, aryl and heteroaryl are optionally substituted by 1 to 3 substituents, independently of one another selected from the group consisting of halogen, hydroxyl,
R⁴ trifluoromethyl, trifluoromethoxy, cyano, nitro, alkyl, alkoxy, alkylthio, hydroxycarbonyl, alkoxycarbonyl, amino, alkylamino, alkylcarbonylamino, alkylaminocarbonyl, alkylaminosulphonyl and alkylsulphonylamino, represents hydrogen, (C₁-C₄) -alkyl, (CH₂)ₚcycloalkyl, (CH₂)pheterocyclyl, (CH₂)ₚaryl or (CH₂)ₚheteroaryl,
in which
p represents 0 to 4 and
alkyl, cycloalkyl, heterocyclyl, aryl and heteroaryl are optionally substituted by 1 to 3 substituents, independently of one another selected from the group consisting of halogen, hydroxyl, trifluoromethyl, trifluoromethoxy, cyano, nitro, alkyl, alkoxy, alkylthio, hydroxycarbonyl, alkoxycarbonyl, amino, alkylamino, alkylcarbonylamino, alkylaminocarbonyl, alkylaminosulphonyl and alkylsulphonylamino,
and their salts, hydrates, hydrates of the salts and solvates.

2. Compounds of the formula (I) according to Claim 1
in which
R¹ represents (C₅-C₁₅)-alkyl or (CH₂)ₙcycloalkyl,
in which
n represents 0 to 4 and
alkyl and cycloalkyl are optionally substituted by 1 to 3 substituents, independently of one another selected from the group consisting of halogen, hydroxyl, trifluoromethyl, trifluoromethoxy, cyano, nitro, alkyl, alkoxy, alkylthio, carboxyl, alkoxycarbonyl, alkylcarbonylamino and alkylaminocarbonyl,
R² represents (C₁-C₈)-alkyl, (CH₂)ₘcycloalkyl, (CH₂)ₘ heterocyclyl, (CH₂)ₘaryl or (CH₂)ₘheteroaryl ,
in which
m represents 0 to 4 and
alkyl, cycloalkyl, heterocyclyl, aryl and heteroaryl are optionally substituted by 1 to 3 substituents, independently of one another selected from the group consisting of halogen, hydroxyl, trifluoromethyl, trifluoromethoxy, cyano, nitro, alkyl, alkoxy, alkylthio, alkoxycarbonyl, amino, alkylamino, alkylcarbonylamino, alkylaminocarbonyl, alkylaminosulphonyl and alkylsulphonylamino,
R³ represents (CH₂)ₒcycloalkyl, (CH₂)ₒheterocyclyl, (CH₂)ₒaryl or (CH₂) ₒheteroaryl,
in which
o represents 0 to 4 and
cycloalkyl, heterocyclyl, aryl and heteroaryl are optionally substituted by 1 to 3 substituents, independently of one another selected from the group consisting of halogen, hydroxyl, trifluoromethyl, trifluoromethoxy, cyano, nitro, alkyl, alkoxy, alkylthio, hydroxycarbonyl, alkoxycarbonyl, amino, alkylamino, alkylcarbonylamino, alkylaminocarbonyl, alkylaminosulphonyl and alkylsulphonylamino,
R⁴ represents hydrogen, (C₁-C₄) -alkyl, (CH₂)ₚcycloalkyl, (CH₂)ₚheterocyclyl, (CH₂)ₚaryl or (CH₂)ₚeheteroaryl ,
in which
p represents 0 to 4 and
alkyl, cycloalkyl, heterocyclyl, aryl and heteroaryl are optionally substituted by 1 to 3 substituents, independently of one another selected from the group consisting of halogen, hydroxyl, trifluoromethyl, trifluoromethoxy, cyano, nitro, alkyl, alkoxy, alkylthio, hydroxycarbonyl, alkoxycarbonyl, amino, alkylamino, alkylcarbonylamino, alkylaminocarbonyl, alkylaminosulphonyl and alkylsulphonylamino,
and their salts, hydrates, hydrates of the salts and solvates.

3. Compounds of the formula (I) according to Claim 1,
in which
R¹ represents neopentyl, (bicyclo[2.2.1]heptyl)methyl, cyclohexylmethyl, cyclobutylmethyl, cyclopentylmethyl, 2,2-dimethyl-1-butyl, 2-ethyl-2-methyl-1-butyl, (1-methylcyclopentyl)methyl), 1-methylcyclohexyl, 4-hydroxy-2,2-dimethyl-1-butyl or 2,2-dimethyl-1-but-3-enyl,
R² represents (C₁-C₄)-alkyl which may be substituted by hydroxyl or fluorine or represents benzyl which is optionally substituted by 1 or 2 substituents, independently of one another selected from the group consisting of fluorine, chlorine, bromine, methyl and trifluoromethyl,
R³ represents phenyl, pyridyl or pyrimidyl which for their part are optionally substituted by a substituent selected from the group consisting of fluorine, chlorine, trifluoromethyl, methyl, ethyl, methoxy, ethoxy, n-propoxy, isopropoxy, amino, hydroxyl, hydroxycarbonyl, (C₁-C₃)-alkylcarbonylamino and mono-(C₁-C₄)-alkylaminocarbonyl,
R⁴ represents hydrogen
and their salts, hydrates, hydrates of the salts and solvates.

4. Process for preparing compounds of the formula (I) as defined in Claim 1, **characterized in that** either
**[A]** compounds of the formula (II) 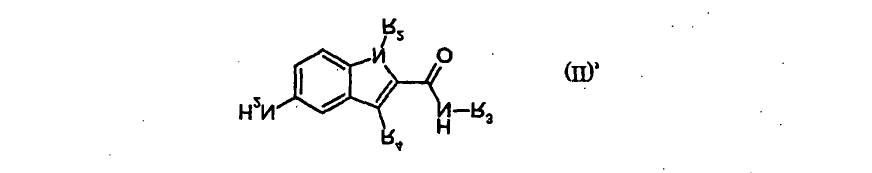 in which
R², R³ and R⁴ are as defined in Claim 1,
are reacted with compounds of the formula (III) 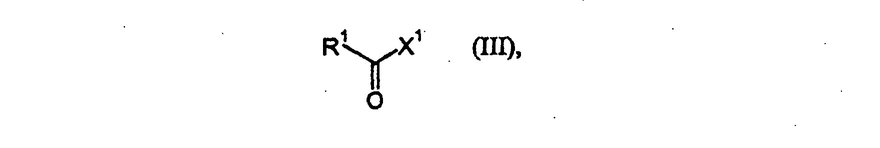 in which
R¹ is as defined in Claim 1 and
X¹ represents halogen or hydroxyl,
or
**[B]** compounds of the formula (XI) 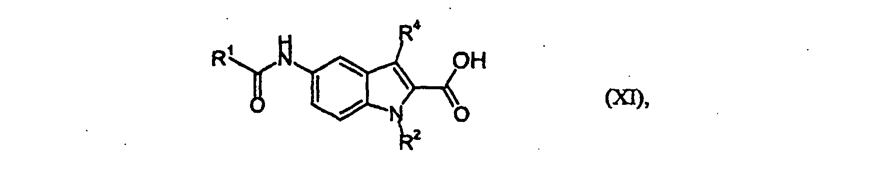 in which
R¹, R² and R⁴ are as defined in Claim 1,
are reacted with compounds of the formula (VI)
R³ - NH₂ (VI)
in which
R³ is as defined in Claim 1.

5. Compounds of the formula (I) as defined in Claim 1 for the prophylaxis and/or treatment of disorders.

6. Medicaments, comprising at least one compound of the formula (I) as defined in Claim 1 and at least one further auxiliary.

7. Medicaments, comprising at least one compound of the formula (I) as defined in Claim 1 and at least one further active compound.

8. Use of compounds of the formula (I) as defined in Claim 1 for preparing medicaments for the prophylaxis and/or treatment of cardiovascular disorders.

9. Use of compounds of the formula (I) as defined in Claim 1 for preparing medicaments for the prophylaxis and/or treatment of myocardial infarction, angina pectoris and cardiac insufficiency.

## Revendications

1. Composés de formule (I) 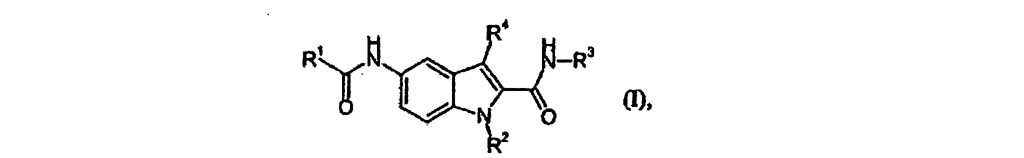 dans laquelle
R¹ est un reste alkyle en C₅ à C₁₅, alcényle en C₅ à C₁₅ ou (CH₂)ₙG,
où
G est un reste cycloalkyle ou un hétérocycle pentagonal ou hexagonal ayant un ou deux atomes d'oxygène,
n a une valeur de 0 à 4 et les restes alkyle, alcényle et G portent éventuellement 1 à 3 substituants choisis, indépendamment les uns des autres, dans le groupe des substituants halogéno, hydroxy, trifluorométhyle, trifluorométhoxy, cyano, nitro, alkyle, alkoxy, alkylthio, carboxyle, alkoxycarbonyle, amino, alkylamino, alkylcarbonylamino et alkylaminocarbonyle,
R² est un reste alkyle en C₁ à C₈, (CH₂)ₘcycloalkyle, (CH₂)ₘhetérocyclyle, (CH₂)ₘaryle ou (CH₂)ₘhétéroaryle,
où
m a une valeur de 0 à 4 et
les restes alkyle, cycloalkyle, hétérocyclyle, aryle et hétéroaryle portent éventuellement 1 à 3 substituants choisis, indépendamment les uns des autres, dans le groupe des substituants halogéno, hydroxy, trifluorométhyle, trifluorométhoxy, cyano, nitiro, alkyle, alkoxy, alkylthio, alkoxycarbonyle, amino, alkylamino, alkylcarbonylamino, alkylaminocarbonyle, alkylaminosulfonyle et alkylsulfonylamino,
R³ est un reste (CH₂) ₒcycloalkyle, (CH₂)ₒhétérocyclyle, (CH₂)ₒaryle ou (CH₂)ₒhétéroaryle,
où
o a une valeur de 0 à 4 et les restes cycloalkyle, hétérocyclyle, aryle et hétéroaryle portent éventuellement 1 à 3 substituants choisis, indépendamment les uns des autres, dans le groupe des substituants halogéno, hydroxy, trifluorométhyle, trifluorométhoxy, cyano, nitro, alkyle, alkoxy, alkylthio, hydroxycarbonyle, alkoxycarbonyle, amino, alkylamino, alkylcarbonylamino, alkylaminocarbonyle, alkylaminosulfonyle et alkylsulfonylamino,
R⁴ représente l'hydrogène, un reste alkyle en C₁ à C₄, (CH₂)ₚcycloalkyle, (CH₂)ₚhétérocyclyle, (CH₂)ₚaryle ou (CH₂)ₚhétéroaryle,
où
p a une valeur de 0 à 4 et
les restes alkyle, cycloalkyle, hétérocyclyle, aryle et hétéroaryle portent éventuellement 1 à 3 substituants choisis, indépendamment les uns des autres, dans le groupe des substituants halogéno, hydroxy, trifluorométhyle, trifluorométhoxy, cyano, nitro, alkyle, alkoxy, alkylthio, hydroxycarbonyle, alkoxycarbonyle, amino, alkylamino, alkylcarbonylamino, alkylaminocarbonyle, alkylaminosulfonyle et alkylsulfonylamino,
et leurs sels, leurs hydrates, les hydrates de leurs sels et leurs produits de solvatation.

2. Composés de formule (I) suivant la revendication 1, formule dans laquelle
R¹ est un reste alkyle en C₅ à C₁₅ ou un reste (CH₂) ₙcycloalkyle,
où
n a une valeur de 0 à 4 et
les restes alkyle et cycloalkyle portent éventuellement 1 à 3 substituants choisis, indépendamment les uns des autres, dans le groupe des substituants halogéno, hydroxy, trifluorométhyle, trifluorométhoxy, cyano, nitro, alkyle, alkoxy, alkylthio, carboxyle, alkoxycarbonyle, alkylcarbonylamino et alkylaminocarbonyle,
R² est un reste alkyle en C₁ à C₈, (CH₂)ₘcycloalkyle, (CH₂)ₘhétérocyclyle, (CH₂)ₘaryle ou (CH₂)ₘhétéroaryle,
où
m a une valeur de 0 à 4 et
les restes alkyle, cycloalkyle, hétérocyclyle, aryle et hétéroaryle portent éventuellement 1 à 3 substituants choisis, indépendamment les uns des autres, dans le groupe des substituants halogéno, hydroxy, trifluorométhyle, trifluorométhoxy, cyano, nitro, alkyle, alkoxy, alkylthio, alkoxycarbonyle, amino, alkylamino, alkylcarbonylamino, alkylaminocarbonyle, alkylaminosulfonyle et alkylsulfonylamino,
R³ est un reste (CH₂)ₒcycloalkyle, (CH₂)ₒhétérocyclyle, (CH₂)ₒaryle ou (CH₂)ₒhétéroaryle,
où
o a une valeur de 0 à 4 et
les restes cycloalkyle, hétérocyclyle, aryle et hétéroaryle portent éventuellement 1 à 3 substituants choisis, indépendamment les uns des autres, dans le groupe des substituants halogéno, hydroxy, trifluorométhyle, trifluorométhoxy, cyano, nitro, alkyle, alkoxy, alkylthio, hydroxycarbonyle, alkoxycarbonyle, amino, alkylamino, alkylcarbonylamino, alkylaminocarbonyle, alkylaminosulfonyle et alkylsulfonylamino,
R⁴ représente l'hydrogène, un reste alkyle en C₁ à C₄, (CH₂)ₚcycloalkyle, (CH₂)ₚhétérocyclyle, (CH₂)ₚaryle ou (CH₂)ₚhétéroaryle,
où
p a une valeur de 0 à 4 et
les restes alkyle, cycloalkyle, hétérocyclyle, aryle et hétéroaryle portent éventuellement 1 à 3 substituants choisis, indépendamment les uns des autres, dans le groupe des substituants halogéno, hydroxy, trifluorométhyle, trifluorométhoxy, cyano, nitro, alkyle, alkoxy, alkylthio, hydroxycarbonyle, alkoxycarbonyle, amino, alkylamino, alkylcarbonylamino, alkylaminocarbonyle, alkylaminosulfonyle et alkylsulfonylamino,
et leurs sels, leurs hydrates, les hydrates de leurs sels et leurs produits de solvatation.

3. Composés de formule (I) suivant la revendication 1, formule dans laquelle
R¹ est un reste néopentyle, (bicyclo[2.2.1]heptyl)méthyle, cyclohexylméthyle, cyclobutylméthyle, cyclopentylméthyle, 2,2-diméthyl-1-butyle, 2-éthyl-2-méthyl-1-butyle, (1-méthylcyclopentyl)méthyle, 1-méthylcyclohexyle, 4-hydroxy-2,2-diméthyl-1-butyle ou 2,2-diméthyl-1-but-3-ényle,
R² est un reste alkyle en C₁ à C₄ qui peut être substitué par un radical hydroxy ou fluoro, ou un groupe benzyle qui porte éventuellement 1 ou 2 substituants choisis, indépendamment l'un de l'autre, dans le groupe des substituants fluoro, chloro, bromo, méthyle et trifluorométhyle,
R³ est un reste phényle, pyridyle ou pyrimidyle, portant chacun le cas échéant un substituant choisi dans le groupe des substituants fluoro, chloro, trifluorométhyle, méthyle, éthyle, méthoxy, éthoxy, n-propoxy, isopropoxy, amino, hydroxy, hydroxycarbonyle, (alkyle en C₁ à C₃)carbonylamino et mono (alkyle en C₁ à C₄) aminocarbonyle,
R⁴ représente l'hydrogène,
et leurs sels, leurs hydrates, les hydrates de leurs sels et leurs produits de solvatation.

4. Procédé de production des composés de formule (I), tels que définis dans la revendication 1, **caractérisé en ce que**
[A] on fait réagir des composés de formule (II) 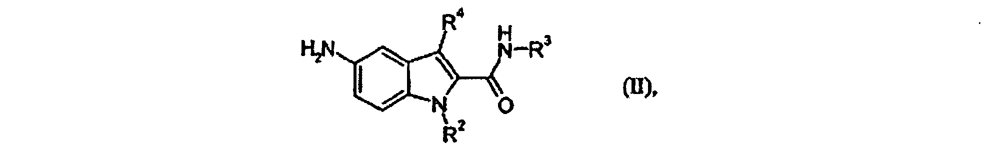 dans laquelle
R², R³ et R⁴ ont la définition indiquée dans la revendication 1,
avec des composés de formule (III) 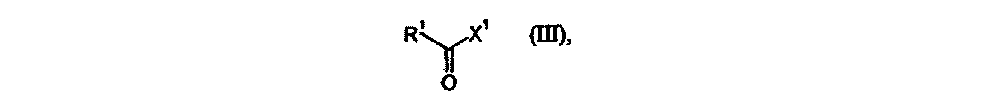 dans laquelle
R¹ a la définition indiquée dans la revendication 1 et
X¹ représente un halogène ou un groupe hydroxy,
ou bien
[B] on fait réagir des composés de formule (XI) 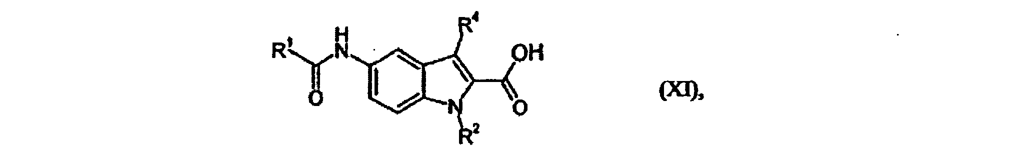
dans laquelle
R¹, R² et R⁴ avec des ont la définition indiquée dans la revendication 1, composés de formule (VI) 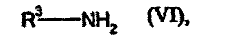 dans laquelle
R³ a la définition indiquée dans la revendication 1.

5. Composés de formule (I) tels que définis dans la revendication 1, destinés à la prophylaxie et/ou au traitement de maladies.

6. Médicament, contenant au moins un composé de formule (I) tel que défini dans la revendication 1, et au moins une autre substance auxiliaire.

7. Médicament, contenant au moins un composé de formule (I) tel que défini dans la revendication 1, et au moins une autre substance active.

8. Utilisation de composés de formule (I), tels que définis dans la revendication 1, pour la préparation de médicaments destinés à la prophylaxie et/ou au traitement de maladies cardio-vasculaires.

9. Utilisation de composés de formule (I), tels que définis dans la revendication 1, pour la préparation de médicaments destinés à la prophylaxie et/ou au traitement de l'infarctus du myocarde, de l'angine de poitrine et de l'insuffisance cardiaque.
